(19) Europäisches Patentamt
European Patent Office
Office européen des brevets

(11) **EP 3 095 861 B1**

(12) **EUROPEAN PATENT SPECIFICATION**

(45) Date of publication and mention of the grant of the patent:
**26.06.2019 Bulletin 2019/26**

(21) Application number: **16177383.3**

(22) Date of filing: **05.05.2011**

(51) Int Cl.:
*C12N 9/54* (2006.01) *C11D 3/386* (2006.01)
*C11D 3/39* (2006.01)

(54) **CONSUMER PRODUCTS WITH PROTEASE VARIANTS**

KONSUMGÜTER MIT PROTEASEVARIANTEN

PRODUITS DE CONSOMMATION COMPRENANT DES VARIANTS DE PROTÉASES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**06.05.2010 US 332151 P**
**06.05.2010 US 332006 P**
**12.10.2010 US 392364 P**
**12.10.2010 US 392188 P**

(43) Date of publication of application:
**23.11.2016 Bulletin 2016/47**

(62) Document number(s) of the earlier application(s) in accordance with Art. 76 EPC:
**11720664.9 / 2 566 960**

(73) Proprietors:
- **The Procter & Gamble Company**
  **Cincinnati, OH 45202 (US)**
- **Danisco US Inc.**
  **Palo Alto, CA 94304 (US)**

(72) Inventors:
- **SOUTER, Philip Frank**
  **Newcastle upon Tyne NE12 9TS (GB)**
- **WARD, Glenn Steven**
  **Newcastle upon Tyne NE12 9TS (GB)**
- **POULOSE, Ayrookaran Joseph**
  **Belmont, CA 94002 (US)**
- **ESTELL, David A.**
  **San Francisco, CA 94114 (US)**
- **KELLIS Jr., James T.**
  **Woodside, CA 94062 (US)**
- **COLLIER, Katherine D.**
  **Los Altos, CA 94024 (US)**
- **CASCAO-PEREIRA, Luis Gustavo**
  **Redwood City, CA 94065 (US)**
- **ALEKSEYEV, Viktor Yuryevich**
  **Palo Alto, CA 94303 (US)**
- **AMIN, Neelam S.**
  **Palo Alto, CA 94306 (US)**
- **YAO, Jian**
  **Sunnyvale, CA 94087 (US)**
- **AUGUSTYN, Katherine**
  **Menlo Park, CA 94025 (US)**

(74) Representative: **Peet, Jillian Wendy**
**Procter & Gamble Technical Centres Limited**
**Whitley Road**
**Longbenton**
**Newcastle upon Tyne**
**NE12 9TS (GB)**

(56) References cited:
**WO-A1-91/00345** **WO-A1-99/20727**
**WO-A2-99/20771** **WO-A2-2009/149200**

EP 3 095 861 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> Procter & Gamble

<120> CONSUMER PRODUCTS

<130> 11710M&D

<160> 77

<170> PatentIn version 3.5

<210> 1
<211> 269
<212> PRT

<213> Bacillus lentus

<400> 1

```
Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Ser Gly Ser Gly Ser Val Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175
```

```
Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265
```

<210> 2
<211> 275
<212> PRT
<213> BAcillus Amyloliquefaciens

<400> 2

```
Ala Gln Ser Val Pro Tyr Gly Val Ser Gln Ile Lys Ala Pro Ala Leu
1               5                   10                  15

His Ser Gln Gly Tyr Thr Gly Ser Asn Val Lys Val Ala Val Ile Asp
            20                  25                  30

Ser Gly Ile Asp Ser Ser His Pro Asp Leu Lys Val Ala Gly Gly Ala
        35                  40                  45

Ser Met Val Pro Ser Glu Thr Asn Pro Phe Gln Asp Asn Asn Ser His
    50                  55                  60

Gly Thr His Val Ala Gly Thr Val Ala Ala Leu Asn Asn Ser Ile Gly
65                  70                  75                  80

Val Leu Gly Val Ala Pro Ser Ala Ser Leu Tyr Ala Val Lys Val Leu
                85                  90                  95

Gly Ala Asp Gly Ser Gly Gln Tyr Ser Trp Ile Ile Asn Gly Ile Glu
            100                 105                 110

Trp Ala Ile Ala Asn Asn Met Asp Val Ile Asn Met Ser Leu Gly Gly
        115                 120                 125
```

Pro Ser Gly Ser Ala Ala Leu Lys Ala Ala Val Asp Lys Ala Val Ala
    130                 135                 140

Ser Gly Val Val Val Val Ala Ala Ala Gly Asn Glu Gly Thr Ser Gly
145                 150                 155                 160

Ser Ser Ser Thr Val Gly Tyr Pro Gly Lys Tyr Pro Ser Val Ile Ala
                165                 170                 175

Val Gly Ala Val Asp Ser Ser Asn Gln Arg Ala Ser Phe Ser Ser Val
            180                 185                 190

Gly Pro Glu Leu Asp Val Met Ala Pro Gly Val Ser Ile Gln Ser Thr
        195                 200                 205

Leu Pro Gly Asn Lys Tyr Gly Ala Tyr Asn Gly Thr Ser Met Ala Ser
    210                 215                 220

Pro His Val Ala Gly Ala Ala Ala Leu Ile Leu Ser Lys His Pro Asn
225                 230                 235                 240

Trp Thr Asn Thr Gln Val Arg Ser Ser Leu Glu Asn Thr Thr Thr Lys
                245                 250                 255

Leu Gly Asp Ser Phe Tyr Tyr Gly Lys Gly Leu Ile Asn Val Gln Ala
            260                 265                 270

Ala Ala Gln
        275

<210> 3
<211> 380
<212> PRT
<213> Bacillus lentus

<400> 3

Val Arg Ser Lys Lys Leu Trp Ile Val Ala Ser Thr Ala Leu Leu Ile
1               5                   10                  15

Ser Val Ala Phe Ser Ser Ser Ile Ala Ser Ala Ala Glu Glu Ala Lys
            20                  25                  30

Glu Lys Tyr Leu Ile Gly Phe Asn Glu Gln Glu Ala Val Ser Glu Phe
        35                  40                  45

Val Glu Gln Val Glu Ala Asn Asp Glu Val Ala Ile Leu Ser Glu Glu
    50                  55                  60

```
Glu Glu Val Glu Ile Glu Leu Leu His Glu Phe Glu Thr Ile Pro Val
65                  70                  75                  80

Leu Ser Val Glu Leu Ser Pro Glu Asp Val Asp Ala Leu Glu Leu Asp
                85                  90                  95

Pro Ala Ile Ser Tyr Ile Glu Glu Asp Ala Glu Val Thr Thr Met Ala
            100                 105                 110

Gln Ser Val Pro Trp Gly Ile Ser Arg Val Gln Ala Pro Ala Ala His
        115                 120                 125

Asn Arg Gly Leu Thr Gly Ser Gly Val Lys Val Ala Val Leu Asp Thr
    130                 135                 140

Gly Ile Ser Thr His Pro Asp Leu Asn Ile Arg Gly Gly Ala Ser Phe
145                 150                 155                 160

Val Pro Gly Glu Pro Ser Thr Gln Asp Gly Asn Gly His Gly Thr His
                165                 170                 175

Val Ala Gly Thr Ile Ala Ala Leu Asn Asn Ser Ile Gly Val Leu Gly
            180                 185                 190

Val Ala Pro Ser Ala Glu Leu Tyr Ala Val Lys Val Leu Gly Ala Ser
        195                 200                 205

Gly Ser Gly Ser Val Ser Ser Ile Ala Gln Gly Leu Glu Trp Ala Gly
    210                 215                 220

Asn Asn Gly Met His Val Ala Asn Leu Ser Leu Gly Ser Pro Ser Pro
225                 230                 235                 240

Ser Ala Thr Leu Glu Gln Ala Val Asn Ser Ala Thr Ser Arg Gly Val
                245                 250                 255

Leu Val Val Ala Ala Ser Gly Asn Ser Gly Ala Gly Ser Ile Ser Tyr
            260                 265                 270

Pro Ala Arg Tyr Ala Asn Ala Met Ala Val Gly Ala Thr Asp Gln Asn
        275                 280                 285

Asn Asn Arg Ala Ser Phe Ser Gln Tyr Gly Ala Gly Leu Asp Ile Val
    290                 295                 300

Ala Pro Gly Val Asn Val Gln Ser Thr Tyr Pro Gly Ser Thr Tyr Ala
305                 310                 315                 320
```

```
Ser Leu Asn Gly Thr Ser Met Ala Thr Pro His Val Ala Gly Ala Ala
                325                 330                 335

Ala Leu Val Lys Gln Lys Asn Pro Ser Trp Ser Asn Val Gln Ile Arg
            340                 345                 350

Asn His Leu Lys Asn Thr Ala Thr Ser Leu Gly Ser Thr Asn Leu Tyr
        355                 360                 365

Gly Ser Gly Leu Val Asn Ala Glu Ala Ala Thr Arg
    370                 375                 380
```

<210> 4
<211> 1143
<212> DNA
<213> Bacillus lentus

<400> 4

```
gtgagaagca aaaaattgtg gatcgtcgcg tcgaccgcac tactcatttc tgttgctttc      60
agttcatcga tcgcatcggc tgctgaagaa gcaaaagaaa aatatttaat tggctttaat     120
gagcaggaag ctgtcagtga gtttgtagaa caagtagagg caaatgacga ggtcgccatt     180
ctctctgagg aagaggaagt cgaaattgaa ttgcttcatg aatttgaaac gattcctgtt     240
ttatccgttg agttaagccc agaagatgtg gacgcgcttg agctcgatcc agcgatttct     300
tatattgaag aggatgcaga agtaacgaca atggcgcaat cagtgccatg gggaattagc     360
cgtgtgcaag ccccagctgc ccataaccgt ggattgacag gttctggtgt aaaagttgct     420
gtcctcgata caggtatttc cactcatcca gacttaaata ttcgtggtgg cgctagcttt     480
gtaccagggg aaccatccac tcaagatggg aatgggcatg gcacgcatgt ggccgggacg     540
attgctgctt taaacaattc gattggcgtt cttggcgtag cgccgagcgc ggaactatac     600
gctgttaaag tattaggggc gagcggttca ggttcggtca gctcgattgc ccaaggattg     660
gaatgggcag ggaacaatgg catgcacgtt gctaatttga gtttaggaag cccttcgcca     720
agtgccacac ttgagcaagc tgttaatagc gcgacttcta gaggcgttct tgttgtagcg     780
gcatctggaa attcaggtgc aggctcaatc agctatccgg cccgttatgc gaacgcaatg     840
gcagtcggag ctactgacca aaacaacaac cgcgccagct tttcacagta tggcgcaggg     900
cttgacattg tcgcaccagg tgtaaacgtg cagagcacat acccaggttc aacgtatgcc     960
agcttaaacg gtacatcgat ggctactcct catgttgcag gtgcagcagc ccttgttaaa    1020
caaaagaacc catcttggtc caatgtacaa atccgcaatc atctaaagaa tacggcaacg    1080
agcttaggaa gcacgaactt gtatggaagc ggacttgtca atgcagaagc tgcaactcgt    1140
taa                                                                  1143
```

<210> 5
<211> 54
<212> DNA
<213> Artificial Sequence

<220>
<223> Primer

<400> 5
aaagtattag gggcgagcgg tgcaggtgga cttagctcga ttgcccaagg attg 54

<210> 6
<211> 46
<212> DNA
<213> Artificial Sequence

<220>
<223> Primer

<400> 6

catctggaaa ttcaggtgca gaatcaatca gctatccggc ccgtta 46

<210> 7
<211> 45
<212> DNA
<213> Artificial Sequence

<220>
<223> Primer

<400> 7
ctgcccataa ccgtggattg gcaggttctg gtgtaaaagt tgctg 45

<210> 8
<211> 45
<212> DNA
<213> Artificial Sequence

<220>
<223> Primer

<400> 8
ctgcccataa ccgtggattg gcaggttctg gtgtaaaagt tgctg 45

<210> 9
<211> 44
<212> DNA
<213> Artificial Sequence

<220>
<223> Primer

<400> 9
gaagcggact tgtcaatgca ttcgctgcaa ctcgttaaag cttg 44

<210> 10
<211> 48
<212> DNA
<213> Artificial Sequence

<220>
<223> Primer

<400> 10
aaagtattag gggcgagcgg tgcaggttcg gtcagctcga ttgcccaa 48

<210> 11
<211> 47
<212> DNA
<213> Artificial Sequence

<220>
<223> Primer

<400> 11
tattaggggc gagcggttca ggtggagtca gctcgattgc ccaagga 47

<210> 12

<211> 46
<212> DNA
<213> Artificial Sequence

<220>
<223> Primer

<400> 12
gtcagctcga ttgcccaagg agtagaatgg gcagggaaca atggca 46

<210> 13
<211> 48
<212> DNA
<213> Artificial Sequence

<220>
<223> Primer

<400> 13
cgttgctaat ttgagtttag gaaacccttc gccaagtgcc acacttga 48

<210> 14
<211> 42
<212> DNA
<213> Artificial Sequence

<220>
<223> Primer

<400> 14
gaaccatcca ctcaagatgg ggaagggcat ggcacgcatg tg 42

<210> 15
<211> 47
<212> DNA
<213> Artificial Sequence

<220>
<223> Primer

<400> 15
accaaaacaa caaccgcgcc gacttttcac agtatggcgc agggctt 47

<210> 16
<211> 23
<212> DNA
<213> Artificial Sequence

<220>
<223> Primer

<400> 16
catatgagtt atgcagtttg tag 23

<210> 17
<211> 23
<212> DNA
<213> Artificial Sequence

<220>
<223> Primer

<400> 17
tgtttttctt ggaattgtgc tgt 23

<210> 18
<211> 23
<212> DNA
<213> Artificial Sequence

<220>
<223> Primer

<400> 18
cagtttgtag aatgcaaaaa gtg 23

<210> 19
<211> 23
<212> DNA
<213> Artificial Sequence

<220>
<223> Primer

<400> 19
gacaaggtaa aggataaaac agc 23

<210> 20
<211> 40
<212> DNA
<213> Artificial Sequence

<220>
<223> Primer

<400> 20
cataaccgtg gattggcagg ttctggtgta aaagttgctg 40

<210> 21
<211> 40
<212> DNA
<213> Artificial Sequence

<220>
<223> Primer

<400> 21
acttttacac cagaacctgc caatccacgg ttatgggcag 40

<210> 22
<211> 35
<212> DNA
<213> Artificial Sequence

<220>
<223> Primer

<400> 22
cactcaagat ggggaagggc atggcacgca tgtgg 35

<210> 23
<211> 39
<212> DNA
<213> Artificial Sequence

<220>
<223> Primer

<400> 23
atgcgtgcca tgcccttccc catcttgagt ggatggttc 39

<210> 24
<211> 39
<212> DNA
<213> Artificial Sequence

<220>
<223> Primer

<400> 24
gcgagcggtt caggtggagt cagctcgatt gcccaagga 39

<210> 25
<211> 40
<212> DNA
<213> Artificial Sequence

<220>
<223> Primer

<400> 25
tgggcaatcg agctgactcc acctgaaccg ctcgccccta 40

<210> 26
<211> 52
<212> DNA
<213> Artificial Sequence

<220>
<223> Primer

<400> 26
gtggagtcag ctcgattgcc caaggagtag aatgggcagg gaacaatggc at 52

<210> 27
<211> 52
<212> DNA
<213> Artificial Sequence

<220>
<223> Primer

<400> 27
cattctactc cttgggcaat cgagctgact ccacctgaac cgctcgcccc ta 52

<210> 28
<211> 42
<212> DNA
<213> Artificial Sequence

<220>
<223> Primer

<400> 28
gcgagcggtg gaggtgcgat cagctcgatt gcccaaggat tg 42

<210> 29
<211> 50
<212> DNA
<213> Artificial Sequence

<220>
<223> Primer

<400> 29
cttgggcaat cgagctgatc gcacctccac cgctcgcccc taatacttta 50

<210> 30
<211> 42
<212> DNA
<213> Artificial Sequence

<220>
<223> Primer

<400> 30
gcgagcggtg caggtggact tagctcgatt gcccaaggat tg 42

<210> 31
<211> 50
<212> DNA
<213> Artificial Sequence

<220>
<223> Primer

<400> 31
cttgggcaat cgagctaagt ccacctgcac cgctcgcccc taatacttta 50

<210> 32
<211> 40
<212> DNA
<213> Artificial Sequence

<220>
<223> Primer

<400> 32
tattaggggc gagcggtgca ggttcggtca gctcgattgc 40

<210> 33
<211> 42
<212> DNA

<213> Artificial Sequence

<220>
<223> Primer

<400> 33
atcgagctga ccgaacctgc accgctcgcc cctaatactt ta 42

<210> 34
<211> 41
<212> DNA
<213> Artificial Sequence

<220>
<223> Primer

<400> 34
ctaatttgag tttaggaaac ccttcgccaa gtgccacact t 41

<210> 35
<211> 41
<212> DNA
<213> Artificial Sequence

<220>
<223> Primer

<400> 35
gcacttggcg aagggtttcc taaactcaaa ttagcaacgt g 41

<210> 36
<211> 39
<212> DNA
<213> Artificial Sequence

<220>
<223> Primer

<400> 36
gaaattcagg tgcagactca atcagctatc cggcccgtt 39

<210> 37
<211> 38
<212> DNA
<213> Artificial Sequence

<220>
<223> Primer

<400> 37
ggatagctga ttgagtctgc acctgaattt ccagatgc 38

<210> 38
<211> 39
<212> DNA
<213> Artificial Sequence

<220>

<223> Primer

<400> 38
gaaattcagg tgcagaatca atcagctatc cggcccgtt 39

<210> 39
<211> 38
<212> DNA
<213> Artificial Sequence

<220>
<223> Primer

<400> 39
ggatagctga ttgattctgc acctgaattt ccagatgc 38

<210> 40
<211> 41
<212> DNA
<213> Artificial Sequence

<220>
<223> Primer

<400> 40
aacgtgcaga gcacagaacc aggttcaacg tatgccagct t 41

<210> 41
<211> 40
<212> DNA
<213> Artificial Sequence

<220>
<223> Primer

<400> 41
catacgttga acctggttct gtgctctgca cgtttacacc 40

<210> 42
<211> 41
<212> DNA
<213> Artificial Sequence

<220>
<223> Primer

<400> 42
tcgattgccc aaggagtaga atgggcaggg aacaatggca t 41

<210> 43
<211> 43
<212> DNA
<213> Artificial Sequence

<220>
<223> Primer

<400> 43

cattgttccc tgcccattct actccttggg caatcgagct gac 43

<210> 44
<211> 810
<212> DNA
<213> Bacillus lentus

<400> 44

```
gcgcaatcag tgccatgggg aattagccgt gtgcaagccc cggctgccca taaccgtgga      60
ttgacaggtt ctggtgtaaa agttgctgtc ctcgatacag gtatttccac tcatccagac     120
ttaaatattc gtggtggcgc tagctttgta ccaggggaac catccactca agatgggaat     180
gggcatggca cgcatgtggc cgggacgatt gctgctctaa acaattcgat tggcgtactt     240
ggcgtagcgc cgagcgcgga actatacgct gttaaagtat taggggcgag cggtgggggc     300
gccatcagct cgattgccca aggattggaa tgggcaggga acaatggcat gcacgttgct     360
aatttgagtt taggaagccc ttcgccaagt gccacacttg agcaagctgt taatagcgcg     420
acttctaggg gcgttcttgt tgtagcggca tctggaaatt cgggtgcaga ctcaatcagc     480
tatccggccc gttatgcgaa cgcaatggca gtcggagcta ctgaccaaaa caacaaccgc     540
gccagctttt cacagtatgg cgcagggctt gacatcgtcg caccaggtgt aaacgtgcag     600
agcacatacc caggttcaac gtatgccagc ttaaacggta catcgatggc tactcctcat     660
gttgcaggtg cagcagtcct tgttaaacat aagaacccat cttggtccaa tgtacgaatc     720
cgcgatcatc taaagaaaac ggcaacgagc ttaggaagca cgaacttgta tggaagcgga     780
cttgtcaatg ccgaagctgc aactcgttaa                                      810
```

<210> 45
<211> 269
<212> PRT
<213> Bacillus lentus

<400> 45

```
Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Ser Gly Gly Gly Ala Ile Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Asp Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220
```

```
Ala Val Leu Val Lys His Lys Asn Pro Ser Trp Ser Asn Val Arg Ile
225                 230                 235                 240

Arg Asp His Leu Lys Lys Thr Ala Thr Ser Leu Gly Ser Thr Asn Leu
                245                 250                 255

Tyr Gly Ser Gly Leu Val Asn Ala Glu Ala Ala Thr Arg
            260                 265
```

<210> 46
<211> 41
<212> DNA
<213> Artificial Sequence

<220>
<223> Primer

<400> 46
gtattagggg cgagcggtgg cggcgccatc agctcgattg c 41

<210> 47
<211> 41
<212> DNA
<213> Artificial Sequence

<220>
<223> Primer

<400> 47
gcaatcgagc tgatggcgcc gccaccgctc gcccctaata c 41

<210> 48
<211> 40
<212> DNA
<213> Artificial Sequence

<220>
<223> Primer

<400> 48
ggggcgagcg gtgggggcgg catcagctcg attgcccaag 40

<210> 49
<211> 40
<212> DNA
<213> Artificial Sequence

<220>
<223> Primer

<400> 49
cttgggcaat cgagctgatg ccgcccccac cgctcgcccc 40

<210> 50
<211> 44
<212> DNA

<213> Artificial Sequence

<220>
<223> Primer

<400> 50
gggcgagcgg tgggggcgcc gttagctcga ttgcccaagg attg 44

<210> 51
<211> 44
<212> DNA
<213> Artificial Sequence

<220>
<223> Primer

<400> 51
caatccttgg gcaatcgagc taacggcgcc cccaccgctc gccc 44

<210> 52
<211> 45
<212> DNA
<213> Artificial Sequence

<220>
<223> Primer

<400> 52
gcatctggaa attcgggtgc aggctcaatc agctatccgg cccgt 45

<210> 53
<211> 45
<212> DNA
<213> Artificial Sequence

<220>
<223> Primer

<400> 53
acgggccgga tagctgattg agcctgcacc cgaatttcca gatgc 45

<210> 54
<211> 43
<212> DNA
<213> Artificial Sequence

<220>
<223> Primer

<400> 54
ctcatgttgc aggtgcagca gcacttgtta aacataagaa ccc 43

<210> 55
<211> 43
<212> DNA
<213> Artificial Sequence

<220>

<223> Primer

<400> 55
gggttcttat gtttaacaag tgctgctgca cctgcaacat gag 43

<210> 56
<211> 44
<212> DNA
<213> Artificial Sequence

<220>
<223> Primer

<400> 56
gtgcagcagt ccttgttaaa caaaagaacc catcttggtc caat 44

<210> 57
<211> 44
<212> DNA
<213> Artificial Sequence

<220>
<223> Primer

<400> 57
attggaccaa gatgggttct tttgtttaac aaggactgct gcac 44

<210> 58
<211> 44
<212> DNA
<213> Artificial Sequence

<220>
<223> Primer

<400> 58
ccatcttggt ccaatgtaca aatccgcgat catctaaaga aaac 44

<210> 59
<211> 44
<212> DNA
<213> Artificial Sequence

<220>
<223> Primer

<400> 59
gttttcttta gatgatcgcg gatttgtaca ttggaccaag atgg 44

<210> 60
<211> 43
<212> DNA
<213> Artificial Sequence

<220>
<223> Primer

<400> 60

ggtccaatgt acgaatccgc aatcatctaa agaaaacggc aac 43

<210> 61
<211> 43
<212> DNA
<213> Artificial Sequence

<220>
<223> Primer

<400> 61
gttgccgttt tctttagatg attgcggatt cgtacattgg acc 43

<210> 62
<211> 43
<212> DNA
<213> Artificial Sequence

<220>
<223> Primer

<400> 62
gaatccgcga tcatctaaag aatacggcaa cgagcttagg aag 43

<210> 63
<211> 43
<212> DNA
<213> Artificial Sequence

<220>
<223> Primer

<400> 63
cttcctaagc tcgttgccgt attctttaga tgatcgcgga ttc 43

<210> 64
<211> 807
<212> DNA
<213> Bacillus lentus

<400> 64

```
gcgcaatcag tgccatgggg aattagccgt gtgcaagccc cggctgccca taaccgtgga        60
ttgacaggtt ctggtgtaaa agttgctgtc ctcgatacag gtatttccac tcatccagac       120
ttaaatattc gtggtggcgc tagctttgta ccaggggaac catccactca agatgggaat       180
gggcatggca cgcatgtggc cgggacgatt gctgctctaa acaattcgat tggcgtactt       240
ggcgtagcgc cgagcgcgga actatacgct gttaaagtat taggggcgag cggtgggggc       300
gccatcagct cgattgccca aggattggaa tgggcaggga acaatggcat gcacgttgct       360
aatttgagtt taggaagccc ttcgccaagt gccacacttg agcaagctgt taatagcgcg       420
acttctaggg gcgttcttgt tgtagcggca tctggaaatt cgggtgcaga ctcaatcagc       480
tatccggccc gttatgcgaa cgcaatggca gtcggagcta ctgaccaaaa caacaaccgc       540

gccagctttt cacagtatgg cgcagggctt gacatcgtcg caccaggtgt aaacgtgcag       600
agcacatacc caggttcaac gtatgccagc ttaaacggta catcgatggc tactcctcat       660
gttgcaggtg cagcagtcct tgttaaacaa aagaacccat cttggtccaa tgtacgaatc       720
cgcgatcatc taaagaatac ggcaacgagc ttaggaagca cgaacttgta tggaagcgga       780
cttgtcaatg ccgaagctgc aactcgt                                           807
```

<210> 65
<211> 269
<212> PRT
<213> Bacillus lentus

<400> 65

```
Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Ser Gly Gly Gly Ala Ile Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Asp Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
```

180 185 190

Val Ala Pro Gly Val Asn Val Gln Ser Thr Tyr Pro Gly Ser Thr Tyr
195 200 205

Ala Ser Leu Asn Gly Thr Ser Met Ala Thr Pro His Val Ala Gly Ala
210 215 220

Ala Val Leu Val Lys Gln Lys Asn Pro Ser Trp Ser Asn Val Arg Ile
225 230 235 240

Arg Asp His Leu Lys Asn Thr Ala Thr Ser Leu Gly Ser Thr Asn Leu
245 250 255

Tyr Gly Ser Gly Leu Val Asn Ala Glu Ala Ala Thr Arg
260 265

<210> 66
<211> 38
<212> DNA
<213> Artificial Sequence

<220>
<223> Primer

<400> 66
gtacgaatcc gcgatagact aaagaatacg gcaacgag 38

<210> 67
<211> 44
<212> DNA
<213> Artificial Sequence

<220>
<223> Primer

<400> 67
gcggacttgt caatgccttt gctgcaactc gttaaagctt acat 44

<210> 68
<211> 41
<212> DNA
<213> Artificial Sequence

<220>
<223> Prinmer

<400> 68
ggaaattcgg gtgcagaatc aatcagctat ccggcccgtt a 41

<210> 69
<211> 38
<212> DNA
<213> Artificial Sequence

<220>
<223> Primer

<400> 69
ctggaaattc gggtgaagac tcaatcagct atccggcc 38

<210> 70
<211> 51
<212> DNA
<213> Artificial Sequence

<220>
<223> Primer

<400> 70
cggcatctgg aaattcgggt gaaggctcaa tcagctatcc ggcccgttat g 51

<210> 71
<211> 42
<212> DNA
<213> Artificial Sequence

<220>
<223> Primer

<400> 71
cataaccgtg gattggcagg ttctggtgta aaagttgctg tc 42

<210> 72
<211> 38
<212> DNA
<213> Artificial Sequence

<220>
<223> Primer

<400> 72
tccactcaag atggggaagg gcatggcacg catgtggc 38

<210> 73
<211> 42
<212> DNA
<213> Artificial Sequence

<220>
<223> Primer

<400> 73
cttgttaaac aaaagagacc atcttggtcc aatgtacgaa tc 42

<210> 74
<211> 42
<212> DNA
<213> Artificial Sequence

<220>
<223> Primer

<400> 74
aatgtacgaa tccgcagaca tctaaagaat acggcaacga gc 42

<210> 75
<211> 43
<212> DNA
<213> Artificial Sequence

<220>
<223> Primer

<400> 75
cgatcatcta aagaatagag caacgagctt aggaagcacg aac 43

<210> 76
<211> 42
<212> DNA
<213> Artificial Sequence

<220>
<223> Primer

<400> 76
gtggattgac aggtagaggt gtaaaagttg ctgtcctcga ta 42

<210> 77
<211> 43
<212> DNA
<213> Artificial Sequence

<220>
<223> Primer

<400> 77
acgattgctg ctctagataa ttcgattggc gtacttggcg tag 43

## Description

FIELD OF INVENTION

This invention relates to consumer products comprising proteases, as well as methods of treating and/or cleaning a surface using such consumer products, as defined in the appended claims.

BACKGROUND OF THE INVENTION

Detergent manufacturers incorporate proteases into their products to provide good cleaning of stains (such as blood). For example, WO99/20771 relates to multiply substituted protease variants with altered net charge for use in detergents. However, given the sustainability and consumer trends to lower wash temperatures it is proving increasingly difficult to deliver consumer acceptable benefits and there remains a need to improve the cleaning and freshness profile of these laundry detergent compositions. The Inventors have found that additionally incorporating certain proteases into consumer products, for example, a laundry detergent composition that may, in one aspect, comprise a hueing agent, a cold-water soluble brightener, a bleach catalyst, a first wash lipase, a bacterial cleaning cellulase, a Guerbet nonionic surfactant and/or a perfume capsule, improves one or more of the cleaning, whiteness, whiteness perception, and/or freshness profile of such consumer product.

SUMMARY OF THE INVENTION

This invention relates to a composition comprising an adjunct material and a cold water protease variant as defined in claim 1 and a method of treating/cleaning a surface as defined in claim 5.

BRIEF DESCRIPTION OF THE DRAWINGS

FIG. 1 provides an alignment of the mature reference proteases including: Bacillus amyloliquefaciens subtilisin BPN' (SEQ ID NO:2) and B. lentus subtilisin GG36 protease (SEQ ID NO: 1). Each amino acid position of each protease variant described herein, including each cold water protease variant, is numbered according to the numbering of the corresponding amino acid position in the amino acid sequence of *Bacillus amyloliquefaciens* subtilisin BPN' shown in Figure 1 as determined by alignment of the protease variant amino acid sequence with the *Bacillus amyloliquefaciens* subtilisin BPN' amino acid sequence.
Figure 2 shows the pHPLT-GG36 Expression Plasmid.
Figure 3 shows the pRA68 Expression Plasmid.
Figure 4 shows the pRA96 Expression Plasmid.

DETAILED DESCRIPTION OF THE INVENTION

Definitions

As used herein "consumer product" means fabric and home care product.

As used herein, the term "fabric and home care product" refers to fabric and home care products and/or generally intended to be used or consumed in the form in which they are sold and that are for treating fabrics, hard surfaces and any other surfaces, and cleaning systems all for the care and cleaning of inanimate surfaces, as well as fabric conditioner products and other products designed specifically for the care and maintenance of fabrics, and air care products, including: air care including air fresheners and scent delivery systems, car care, pet care, livestock care, personal care, jewelry care, dishwashing, fabric conditioning (including softening and/or freshening), laundry detergency, laundry and rinse additive and/or care, pre-treatment cleaning compositions, hard surface cleaning and/or treatment including floor and toilet bowl cleaners, glass cleaners and/or treatments, tile cleaners and /or treatments, ceramic cleaners and/or treatments, and other cleaning for consumer or institutional use. In some embodiments, the fabric and home care products are suitable for use on wounds and/or skin. "Fabric and home care product" includes consumer and institutional products. This definition does not include products (a) intended to be used to clean contact lenses, or ultrafiltration membranes, or (b) in healing wounds or for the medical treatment of skin conditions. Such fabric and home care products are generally intended to be used or consumed in the form in which they are sold.

As used herein, the term "cleaning and/or treatment composition" is a subset of fabric and home care products. Such products include, but are not limited to, products for treating fabrics, hard surfaces and any other surfaces in the

area of fabric and home care, including: air care including air fresheners and scent delivery systems, car care, dishwashing, fabric conditioning (including softening and/or freshening), laundry detergency, laundry and rinse additive and/or care, hard surface cleaning and/or treatment including floor and toilet bowl cleaners, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use: car or carpet shampoos, bathroom cleaners including toilet bowl cleaners; as well as cleaning auxiliaries such as bleach additives and "stain-stick" or pre-treat types, substrate-laden products such as dryer added sheets.

As used herein, the term "fabric and/or hard surface cleaning and/or treatment composition" is a subset of cleaning and treatment compositions that includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, car or carpet shampoos, bathroom cleaners including toilet bowl cleaners; fabric conditioning products including softening and/or freshening that may be in liquid, solid and/or dryer sheet form ; as well as cleaning auxiliaries such as bleach additives and "stain-stick" or pre-treat types, substrate-laden products such as dryer added sheets. All of such products which are applicable may be in standard, concentrated or even highly concentrated form even to the extent that such products may in certain aspect be non-aqueous.

As used herein, the term "cold water protease variant" means a variant of a parent protease, said parent protease’s sequence being at least 95%, at least 96%, at least 97%, at least 98%, at least 99%, at least 99.5% or 100% identical to the amino acid sequence of SEQ ID NO:1, said variant having one or more of the following characteristics:

a) a Test Method 2 performance index of at least 1.1, at least 1.2, at least 1.3, at least 1.4, at least 1.5, at least 1.6, at least 1.7, at least 1.8, at least 1.9, at least 2; from 1.1 to 10, from 1.1 to 8 or even from 1.1 to 5;
b) a Test Method 3 performance index of at least 1.1, at least 1.2, at least 1.3, at least 1.4, at least 1.5, at least 1.6, at least 1.7, at least 1.8, at least 1.9, at least 2; from 1.1 to 10, from 1.1 to 8 or even from 1.1 to 5;
c) a Test Method 4 performance index of at least 1.0, at least 1.1, at least 1.2, at least 1.3, at least 1.4, at least 1.5, at least 1.6, at least 1.7, at least 1.8, at least 1.9, at least 2, from 1.0 to 10, from 1.0 to 8 or even from 1.0 to 5;
d) a Test Method 6 performance index of at least 1.1, at least 1.2, at least 1.3, at least 1.4, at least 1.5, at least 1.6, at least 1.7, at least 1.8, at least 1.9, at least 2; from 1.1 to 10, from 1.1 to 8 or even from 1.1 to 5.

Test Method 2, Test Method 3, Test Method 4 and Test Method 6 are explicitly described *infra* in the section entitled "TEST METHODS".

Herein the cold water protease is mutated such that the mutations consist of one of the following sets of mutations: T022A-N062E, T022A-P129E, T022A-L111V, T022A-S188D, A016S-T022A, T022A-E089P, T022A-G159E, T022A-A158E, T22A-L111V-G159E, T22A-S103G-G159E, T22A-S128N-E271F-Y209E, T22A-Y209E-E271F, T22A-S101A-Y209E, T22A-L111V-S128N, T22A-S101A-G159F, T22A-S101A-S103G-V104L, T22A-S101G-S103A-V104I-G159D-A232V-Q245R-N248D-H249R, T22A-S101G-S103A-V104I-G159D-A232V-Q245R-N248D-E271F, T022A-S101G-S103A-V104I-P129E-A158E-S188D-A232V-Q245R-N248D-H249R, T022A-S024R-S101G-S103A-V104I-P129E-A158E-S188D-A232V-Q245R-N248D-H249R, T022A-S024R-S101G-S103A-V104I-P129E-G159E-S188D-A232V-Q245R-N248D-H249R, T022A-S024R-S101G-S103A-V104I-A158E-G159E-S188D-A232V-Q245R-N248D-H249R, T022A-S024R-S101G-S103A-V104I-P129E-A158E-G159E-S188D-A232V-N238R-Q245R-N248D, T022A-S101G-S103A-V104I-P129E-A158E-G159E-A232V-N238R-Q245R-N248D, T022A-S024R-S101G-S103A-V104I-P129E-A158E-G159E-A232V-Q245R-N248D-H249R, T022A-S024R-S101G-S103A-V104I-S128N-A158E-S188D-A232V-Q245R-N248D, T022A-S024R-S101G-S103A-V104I-P129E-A158E-A232V-Q245R-N248D, T022A-S024R-S101G-S103A-V104I-P129E-A232V-Q245R-N248D, T022A-S024R-S101G-S103A-V104I-P129E-S188D-A232V-Q245R-N248D, T022A-S101G-S103A-V104I-S128N-P129E-S188D-A232V-N238R-Q245R-N248D, T022A-S024R-S101G-S103A-V104I-S128N-S188D-A232V-Q245R-N248D, T022A-S024R-S101G-S103A-V104I-S128N-P129E-A158E-A232V-Q245R-N248D, T022A-S101G-S103A-V104I-S128N-P129E-A232V-N238R-Q245R-N248D, T022A-S024R-S101G-S103A-V104I-P129E-A158E-S188D-A232V-N238R-Q245R-N248D, T022A-S024R-S101G-S103A-V104I-A158E-G159E-S188D-A232V-Q245R-N248D, T022A-S101G-S103A-V104I-P129E-A158E-A232V-N238R-Q245R-N248D, T022A-S024R-S101G-S103A-V104I-P129E-A158E-A232V-Q245R-N248D-H249R, T022A-S101G-S103A-V104I-A158E-G159E-S188D-A232V-Q245R-N248D-H249R, T022A-S101G-S103A-V104I-A158E-S188D-A232V-Q245R-N248D, T022A-S101G-S103A-V104I-P129E-S188D-A232V-N238R-Q245R-N248D, T022A-S024R-S101G-S103A-V104I-A158E-G159E-S188D-A232V-N238R-Q245R-N248D, T022A-S024R-S101G-S103A-V104I-S128N-P129E-S188D-A232V-Q245R-N248D, T022A-S101G-S103A-V104I-P129E-G159E-A232V-N238R-Q245R-N248D, T022A-S101G-S 103A-V104I-P129E-A158E-A232V-Q245R-N248D-H249R, T022A-S101G-S103A-V104I-P129E-G159E-A232V-Q245R-N248D-H249R, T022A-S101G-S103A-V104I-G159E-S188D-A232V-Q245R-N248D-H249R, T022A-S101G-S103A-V104I-P129E-G159E-S188D-A232V-Q245R-N248D-H249R, T022A-S101G-S 103A-V104I-A158E-G159E-A232V-N238R-Q245R-N248D, T022A-S101G-S103A-V1041-P129E-A158E-G159E-A232V-Q245R-N248D-H249R, T022A-S024R-S101G-S103A-V104I-P129E-A158E-G159E-A232V-Q245R-N248D, T022A-S024R-S101G-S103A-V104I-A158E-A232V-Q245R-N248D-H249R, T022A-S024R-S101G-S103A-V104I-L148I-A158E-A232V-Q245R-N248D, T022A-S101G-S103A-V104I-G159E-S188D-A232V-N238R-Q245R-N248D, T022A-S024R-S101G-S 103A-V104I-P129E-A158E-G159E-S188D-A232V-Q245R-N248D-H249R, T022A-S101G-S103A-V104I-P129E-A232V-N238R-Q245R-N248D, T022A-S024R-S101G-S103A-V104I-S188D-A232V-Q245R-N248D-H249R, T022A-S101G-S 103A-V104I-A158E-G159E-A232V-Q245R-N248D-H249R, T022A-S024R-S101G-S103A-V104I-P129E-A232V-Q245R-N248D-H249R, T022A-S024R-S101G-S103A-V104I-P129E-L148I-A232V-Q245R-N248D, T022A-S024R-S101G-S103A-V104I-A158E-A232V-N238R-Q245R-N248D, T022A-S101G-S103A-V104I-S128N-G159E-A232V-Q245R-N248D, T022A-S101G-S103A-V104I-S128N-P129E-A158E-A232V-N238R-Q245R-N248D, T022A-S024R-S101G-S103A-V104I-S128N-P129E-A158E-S188D-A232V-Q245R-N248D-H249R, T022A-S024K-S101G-S 103A-V104I-S128N-A158E-G159E-A232V-Q245R-N248D, T022A-S101G-S103A-V104I-L148I-S188D-A232V-Q245R-N248D, T022A-S128N-A158E-H249R, T022A-N062E-A158E, T022A-S101A-A158E-R186H-H249R, A016S-T022A-A158E-R186H-E271F, T022A-S128N-A158E-R186H, T022A-S128N-R186H-S188D-, T022A-A158E-R186H-H249R-E271F, T022A-V104L-A158E-H249R, T022A-V104L-R186H-S188D-H249R, T022A-S101G-S103A-V104I-G159D-L217E-A232V-Q245R-N248D-E271F, T022A-N043R-S101G-S103A-V104I-G159D-S188D-L217E-A232V-Q245R-N248D-E271F, T022A-S101G-S103A-V104I-G159D-S188D-A232V-Q245R-N248D-E271F, T022A-N076D-S101G-S103A-V104I-G159D-S188D-A232V-Q245R-N248D-E271F, T022A-N043R-N076D-S101G-S 103A-V104I-G159D-S188D-A232V-Q245R-N248D-E271F, T022A-N076D-S101G-S103A-V104I-G159D-A232V-Q245R-N248D-E271F, T022A-S101G-S103A-V104I-G159D-A232V-Q245R-N248D-E271F, H017R-T022A-N076D-S101G-S103A-V104I-G159D-S188D-A232V-Q245R-N248D-E271F, T022A-N043R-S101G-S103A-V104I-G159D-A232V-Q245R-N248D-E271F, T022A-S101G-S103A-V104I-G159D-S188D-A232V-Q245R-N248D-H249R-E271F, H017R-T022A-N076D-S101G-S103A-V104I-G159D-A232V-Q245R-N248D-E271F, T022A-N076D-S101G-S103A-V104I-G159D-A232V-Q245R-N248D-H249R-E271F, T022A-S101G-G102A-S103A-V104I-G159D-S188D-A232V-Q245R-N248D-E271F, T022A-S024R-S101D-S103A-V104I-G118R-G159D-S188D-A232V-N248D-E271F, T022A-S024R-S103A-V104I-P129E-G159D-S188D-A232V-N248D-E271F, T022A-S024R-S103A-V104I-G118R-G159D-S188D-L217D-A232V-N248D, T022A-S024R-S101D-S103A-V104I-G118R-P129E-G159D-S188D-A232V-Q245R-N248D, T022A-S024R-S101D-S103A-V104I-G159D-S188D-A232V-Q245R-N248D, T022A-N043R-S103A-V104I-G118R-P129E-G159D-S188D-A232V-Q245R-N248D, T022A-N043R-S103A-V104I-G118R-S128I-P129E-G159D-S188D-A232V-N248D, T022A-N043R-S101D-S103A-V104I-G118R-P129E-G159D-S188D-A232V-N248D-E271F, T022A-S024R-N043R-S101D-S103A-V104I-G159D-S188D-A232V-Q245R-N248D, T022A-S103A-V104I-G159D-S188D-A232V-N248D, T022A-S024R-S103A-V104I-G118R-P129E-G159D-S188D-A232V-N248D-E271F, T022A-S024R-S103A-V104I-G159D-S188D-L217D-A232V-Q245R-N248D-E271F, T022A-N043R-N062E-S103A-V104I-G159D-S188D-A232V-Q245R-N248D-E271F, T022A-N043R-S103A-V104I-P129E-G159D-S188D-A232V-Q245R-N248D, T022A-S024R-S103A-V104I-G159D-S188D-L217D-A232V-N248D-E271F, T022A-S103A-V104I-G118R-G159D-S188D-L217D-A232V-Q245R-N248D, T022A-S024R-S101D-S103A-V104I-G118R-S128I-G159D-S188D-A232V-Q245R-N248D, T022A-S024R-N043R-S103A-V104I-G159D-S188D-L217D-A232V-N248D-E271F, T022A-N043R-S103A-V104I-G118R-G159D-S188D-L217D-A232V-N248D-E271F, T022A-N043R-S103A-V104I-G118R-G159D-S188D-A232V-N248D-E271F, T022A-S103A-V104I-G159D-S188D-L217D-A232V-Q245R-N248D-E271F, T022A-N043R-S103A-V104I-S128I-G159D-S188D-A232V-Q245R-N248D, T022A-S101D-S103A-V104I-G118R-G159D-S188D-L217D-A232V-Q245R-N248D-E271F, T022A-S103A-V104I-G118R-P129E-G159D-S188D-A232V-Q245R-N248D-E271F, T022A-S024R-N043R-S103A-V104I-G118R-G159D-S188D-L217D-A232V-N248D, T022A-N043R-S103A-V1041-S128I-P129E-G159D-S188D-A232V-Q245R-N248D, T022A-N043R-S103A-V104I-G118R-S128I-P129E-G159D-S188D-A232V-N248D-E271F, T022A-S024R-S103A-V104I-G118R-S128I-P129E-G159D-S188D-A232V-N248D, T022A-S024R-S103A-V104I-S128I-G159D-S188D-A232V-N248D, wherein each amino acid position is numbered according to the numbering of the corresponding amino acid position in the amino acid sequence of Bacillus amyloliquefaciens subtilisin BPN', shown in SEQ ID NO: 2. Preferred cold water proteases suitable for use in the present invention have the characteristics according to Test Method 6 as defined in d) above.

As used herein, the term "protease variant" means a variant of a parent protease, said parent protease's sequence being at least 95%, at least 96%, at least 97%, at least 98%, at least 99%, at least 99.5% or 100% identical to the amino acid sequence of SEQ ID NO:1, wherein each amino acid position is numbered according to the numbering of the corresponding amino acid position in the amino acid sequence of Bacillus amyloliquefaciens subtilisin BPN'.

As used herein, articles such as "a" and "an" when used in a claim, are understood to mean one or more of what is claimed or described.

As used herein, the terms "include", "includes" and "including" are meant to be non-limiting.

As used herein, the term "solid" includes granular, powder, bar and tablet product forms.

As used herein, the term "fluid" includes liquid, gel, paste and gas product forms.

As used herein, the term "situs" includes fabrics, garments, and/or hard surfaces.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Consumer Products

As described herein, the consumer products comprising the variant proteases disclosed herein find particular use in the cleaning industry, for example, laundry and dish detergents. These applications place enzymes under various environmental stresses. The variant proteases employed in the present invention provide advantages over many currently used enzymes, due, at least in part to, their stability under various conditions.

Indeed, there are a variety of wash conditions including varying detergent formulations, wash water volumes, wash water temperatures, and lengths of wash time, to which proteases involved in washing are exposed. In addition, detergent formulations used in different geographical areas have different concentrations of their relevant components present in the wash water. For example, European detergents typically have about 4500-5000 ppm of detergent components in the wash water, while Japanese detergents typically have approximately 667 ppm of detergent components in the wash water. In North America, particularly the United States, detergents typically have about 975 ppm of detergent components present in the wash water.

A low detergent concentration system includes detergents where less than about 800 ppm of the detergent components are present in the wash water. Japanese detergents are typically considered low detergent concentration system as they have approximately 667 ppm of detergent components present in the wash water.

A medium detergent concentration includes detergents where between about 800 ppm and about 2000ppm of the detergent components are present in the wash water. North American detergents are generally considered to be medium detergent concentration systems as they have approximately 975 ppm of detergent components present in the wash water. Brazil typically has approximately 1500 ppm of detergent components present in the wash water.

A high detergent concentration system includes detergents where greater than about 2000 ppm of the detergent components are present in the wash water. European detergents are generally considered to be high detergent concentration systems as they have approximately 4500-5000 ppm of detergent components in the wash water.

Latin American detergents are generally high suds phosphate builder detergents and the range of detergents used in Latin America can fall in both the medium and high detergent concentrations as they range from 1500 ppm to 6000 ppm of detergent components in the wash water. As mentioned above, Brazil typically has approximately 1500 ppm of detergent components present in the wash water. However, other high suds phosphate builder detergent geographies, not limited to other Latin American countries, may have high detergent concentration systems up to about 6000 ppm of detergent components present in the wash water.

In light of the foregoing, it is evident that concentrations of detergent compositions in typical wash solutions throughout the world varies from less than about 800 ppm of detergent composition ("low detergent concentration geographies"), for example about 667 ppm in Japan, to between about 800 ppm to about 2000 ppm ("medium detergent concentration geographies"), for example about 975 ppm in U.S. and about 1500 ppm in Brazil, to greater than about 2000 ppm ("high detergent concentration geographies"), for example about 4500 ppm to about 5000 ppm in Europe and about 6000 ppm in high suds phosphate builder geographies.

The concentrations of the typical wash solutions are determined empirically. For example, in the U.S., a typical washing machine holds a volume of about 64.4 L of wash solution. Accordingly, in order to obtain a concentration of about 975 ppm of detergent within the wash solution about 62.79 g of detergent composition must be added to the 64.4 L of wash solution. This amount is the typical amount measured into the wash water by the consumer using the measuring cup provided with the detergent.

As a further example, different geographies use different wash temperatures. The temperature of the wash water in Japan is typically less than that used in Europe. For example, the temperature of the wash water in North America and Japan is typically between about 10 and about 30°C (e.g., about 20°C), whereas the temperature of wash water in

Europe is typically between about 30 and about 60°C (*e.g.*, about 40°C). However, in the interest of saving energy, many consumers are switching to using cold water washing. In addition, in some further regions, cold water is typically used for laundry, as well as dish washing applications. In some embodiments, the "cold water washing" of the present invention utilizes washing at temperatures from about 10°C to about 40°C, or from about 20°C to about 30°C, or from about 15°C to about 25°C, as well as all other combinations within the range of about 15°C to about 35°C, and all ranges within 10°C to 40°C.

As a further example, different geographies typically have different water hardness. Water hardness is usually described in terms of the grains per gallon mixed $Ca^{2+}/Mg^{2+}$. Hardness is a measure of the amount of calcium ($Ca^{2+}$) and magnesium ($Mg^{2+}$) in the water. Most water in the United States is hard, but the degree of hardness varies. Moderately hard (60-120 ppm) to hard (121-181 ppm) water has 60 to 181 parts per million (parts per million converted to grains per U.S. gallon is ppm # divided by 17.1 equals grains per gallon) of hardness minerals.

| **Water** | **Grains per gallon** | **Parts per million** |
|---|---|---|
| Soft | less than 1.0 | less than 17 |
| Slightly hard | 1.0 to 3.5 | 17 to 60 |
| Moderately hard | 3.5 to 7.0 | 60 to 120 |
| Hard | 7.0 to 10.5 | 120 to 180 |
| Very hard | greater than 10.5 | greater than 180 |

European water hardness is typically greater than about 10.5 (for example about 10.5 to about 20.0) grains per gallon mixed $Ca^{2+}/Mg^{2+}$ (*e.g.*, about 15 grains per gallon mixed $Ca^{2+}/Mg^{2+}$). North American water hardness is typically greater than Japanese water hardness, but less than European water hardness. For example, North American water hardness can be between about 3 to about 10 grains, about 3 to about 8 grains or about 6 grains. Japanese water hardness is typically lower than North American water hardness, usually less than about 4, for example about 3 grains per gallon mixed $Ca^{2+}/Mg^{2+}$.

Accordingly, in some embodiments, the present invention provides consumer products that show surprising wash performance in at least one set of wash conditions (*e.g.*, water temperature, water hardness, and/or detergent concentration).

In some embodiments, the consumer products of the present invention are comparable in wash performance to other consumer products comprising other subtilisin proteases. In some embodiments, the consumer products of the present invention exhibit enhanced wash performance as compared to consumer products comprising subtilisin proteases currently commercially available. Thus, in some embodiments of the present invention, the consumer products comprising the variant proteases provided herein exhibit enhanced cleaning due, at least in part to, to enhanced oxidative stability, enhanced thermal stability, enhanced cleaning capabilities under various conditions, and/or enhanced chelator stability of the enzymes employed.

Consumer Products

In all of their forms, the compositions disclosed herein are consumer products.

In one aspect, a composition comprising an adjunct material and a cold water protease variant, said composition being a consumer product, is disclosed.

Test Method 2, Test Method 3, Test Method 4 and Test Method 6 are discussed *infra* in the section entitled "TEST METHODS".

In one aspect of said composition, said adjunct material may comprise an ingredient selected from the group consisting of: an encapsulate comprising a perfume, a hueing agent, surfactants, builders, chelating agents, dye transfer inhibiting agents, dispersants, additional enzymes, enzyme stabilizers, catalytic materials, bleach activators, hydrogen peroxide, sources of hydrogen peroxide, preformed peracids, polymeric dispersing agents, clay soil removal/antiredeposition agents, brighteners, suds suppressors, dyes, perfumes, structure elasticizing agents, fabric softeners, carriers, hydrotropes, processing aids, solvents, pigments and mixtures thereof.

In one aspect of said composition, said composition may comprise an adjunct material selected from the group consisting of:

a) perfume encapsulates;
b) fabric hueing agents;
c) cold-water soluble brighteners;

d) a bleach catalyst that may comprise a metal catalyst such as a transition metal catalyst, or more preferably, a material selected from the group consisting of iminium cations, iminium polyions; iminium zwitterions; modified amines; modified amine oxides; N-sulphonyl imines; N-phosphonyl imines; N-acyl imines; thiadiazole dioxides; perfluoroimines; cyclic sugar ketones and mixtures thereof;
e) first wash lipases;
f) bacterial cleaning cellulases;
g) Guerbet nonionic surfactants; and
h) mixtures thereof.

Preferred proteases comprise at least one, or even two or more charged mutations selected from the group consisting of N062E, S101D, P129E, A158E, G159D/E, and/or S188D. Such proteases are particularly preferred for incorporation into detergent compositions suitable for addition to water to make a wash liquor preferably having low ionic strength or low detergent concentration. Thus in a preferred aspect, these proteases will form part of a detergent composition that is added to water, either for a handwashing or machine washing process, typically within a washing machine, to form a wash liquor, whose conductivity is from about 0.1 mS/cm to about 3 mS/cm, from about 0.3 mS/cm to about 2.5 mS/cm, or even from about 0.5 mS/cm to about 2 mS/cm.

In a further aspect such proteases that comprise at least one, or even two or more charged mutations selected from the group consisting ofN062E, S101D, P129E, A158E, G159D/E, and/or S188D have a charge of 0, -1, -2, -3, -4 or -5, preferably 0, -1, -2 or -3, most preferably -1 or -2 relative to the enzyme of SEQ ID NO:1. Preferred mutations to arrive at a desired net charge comprise one, two or more mutations selected from the group consisting of:, S024R, G118R, Q245R, H249R and E271F.

In accordance with the present disclosure, particularly preferred proteases for use in the disclosure:

(a) comprise one or more charged mutations selected from the group consisting of N062E, S101D, P129E, A158E, G159D/E, , and/or S188D; and/or
(b) have a charge of 0, -1, -2, -3, -4 or -5, preferably 0, -1, -2 or -3, most preferably -1 or -2 relative to the enzyme of SEQ ID NO:1; and/or
(c) comprise one, two, three or more mutations to arrive at a desired net charge selected from the group comprising S024R, G118R, Q245R, H249R and/or E271F.

By "mutations to arrive at a desired net charge," it is intended to mean that when the enzyme variant is compared to the *Bacillus lentus* subtilisin GG36 protease, the total net charge of the variant relative to *Bacillus lentus* subtilisin GG36 can be adjusted to be within the preferred range by selecting one or more further mutations preferably selected from the mutations identified, for example listed in (c) above.

Preferably these preferred proteases form part of a detergent composition that is added to water, either in a hand or machine washing process, typically within a washing machine, to form a wash liquor, whose conductivity is from about 0.1 mS/cm to about 3 mS/cm, from about 0.3 mS/cm to about 2.5 mS/cm, or even from about 0.5 mS/cm to about 2 mS/cm.

Without wishing to be bound by theory it is believed that these mutations to arrive at a desired net charge provide enhanced overall protease performance by ensuring optimal charge of the molecule for low ionic strength conditions, or wash liquors comprising low detergent concentration - it is only through careful combination of certain mutations, of which these are preferred, that such preferred proteases can be obtained.

In another aspect the inventors have found that preferred proteases comprise at least one or two or more charged mutations selected from the group consisting of, S024R, N043R, Q245R and/or H249R. Such proteases are particularly preferred for incorporation into detergent compositions which will be added to water to make a wash liquor preferably having high ionic strength or high detergent concentration. For example these preferred proteases may form part of a detergent composition that is added to water, either for hand washing or machine washing, typically within a washing machine, to form a wash liquor, whose conductivity is from above about 3 mS/cm to about 30 mS/cm, from about 3.5 mS/cm to about 20 mS/cm, or even from about 4mS/cm to about 10 mS/cm.

In a further aspect such proteases comprise at least one, or two or even more charged mutations selected from the group consisting of S024R, N043R, Q245R and/or H249R have a charge of 0, +1, +2, +3, +4 or +5, preferably +1, +2 or +3, most preferably +2 relative to the enzyme of SEQ ID NO:1. Preferred mutations to arrive at a desired net charge comprise N076D.

Preferably these proteases form part of a detergent composition that is added to water either in a hand or machine washing process, typically within a washing machine, to form a wash liquor, whose conductivity is from above about 3 mS/cm to about 30 mS/cm, from about 3.5 mS/cm to about 20 mS/cm, or even from about 4mS/cm to about 10 mS/cm.

Without wishing to be bound by theory it is believed that these mutations to arrive at a desired net charge provide enhanced overall protease performance in high ionic strength or high detergent concentration conditions - it is only through careful combination of certain mutations, of which these are preferred, that such preferred proteases can be obtained.

In one aspect, of said composition, the protease variant comprises an amino acid sequence which differs from the amino acid sequence of SEQ ID NO:1 and wherein the total net charge of the protease variant is 0, +1, +2, +3, +4, +5, -1, -2, -3, -4, or -5 relative to the total net charge of the *Bacillus lentus* subtilisin GG36 protease, and wherein amino acid positions of the protease variant are numbered according to the numbering of corresponding amino acid positions in the amino acid sequence of *Bacillus amyloliquefaciens* subtilisin BPN' shown in SEQ ID NO:2 as determined by alignment of the protease variant amino acid sequence with the *Bacillus amyloliquefaciens* subtilisin BPN' amino acid sequence.

In one aspect of said composition, said composition comprises a second non-immunoequivalent protease selected from the group comprising:

a) subtilisins (EC 3.4.21.62);
b) trypsin-like or chymotrypsin-like proteases;
c) metalloproteases; and
d) mixtures thereof.

In one aspect of said composition, said composition comprises a second non-immunoequivalent protease selected from the group comprising:

a) subtilisins (EC 3.4.21.62) derived from B. subtilis, B. amyloliquefaciens, Bacillus pumilus and Bacillus gibsonii;
b) trypsin proteases and/or chymotrypsin proteases derived from Cellumonas;
c) metalloproteases derived from Bacillus amyloliquefaciens; and
d) mixtures thereof.

In one aspect of said composition, said composition comprises an additional enzyme selected from the group consisting of hemicellulases, peroxidases, proteases, cellulases, cellobiose dehydrogenases, xyloglucanases, xylanases, lipases, phospholipases, esterases, cutinases, pectinases, mannanases, pectate lyases, keratinases, reductases, oxidases, phenoloxidases, lipoxygenases, ligninases, pullulanases, tannases, pentosanases, lichenases glucanases, arabinosidases, hyaluronidase, chondroitinase, laccase, amylases and mixtures thereof.

In one aspect of said composition, said additional enzyme is selected from the group consisting of:

a) first-wash lipases;
b) alpha-amylases;
c) bacterial cleaning cellulases; and
d) mixtures thereof.

In one aspect of said composition, regarding said adjunct material(s), in said composition

a) said encapsulate comprises a perfume comprises a perfume micro capsule;
b) said hueing agent comprises a material selected from the group consisting of basic, acid, hydrophobic, direct and polymeric dyes, and dye-conjugates having a peak absorption wavelength of from 550nm to 650nm and mixtures thereof;
c) said detersive surfactant comprises a material selected from the group consisting of anionic detersive surfactants, non-ionic detersive surfactant, cationic detersive surfactants, zwitterionic detersive surfactants and amphoteric detersive surfactants and mixtures thereof;
d) said builder comprises a material selected from the group consisting of zeolites, phosphates and mixtures thereof;
e) said silicate salt comprises a material selected from the group consisting of sodium silictae, potassium silicate and mixtures thereof;
f) said brightener preferably comprises a material selected from the group consisting of cold-water soluble brighteners and mixtures thereof;
g) said carboxylate polymer comprises a material selected from the group consisting of maleate/acrylate random copolymer or polyacrylate homopolymer and mixtures thereof;
h) said soil release polymer comprises a material selected from the group consisting of terephthalate co-polymers and mixtures thereof;
i) said cellulosic polymer comprises a material selected from the group consisting of alkyl cellulose, alkyl alkoxyalkyl

cellulose, carboxyalkyl cellulose, alkyl carboxyalkyl cellulose and mixtures thereof;

j) said bleach catalyst comprises a transition metal catalyst or ligand for forming a transition metal catalyst, or preferably a material selected from the group consisting of iminium cations, iminium polyions; iminium zwitterions; modified amines; modified amine oxides; N-sulphonyl imines; N-phosphonyl imines; N-acyl imines; thiadiazole dioxides; perfluoroimines; cyclic sugar ketones and mixtures thereof;

k) said bleach activator comprises a material selected from the group consisting of dodecanoyl oxybenzene sulphonate, decanoyl oxybenzene sulphonate, decanoyl oxybenzoic acid or salts thereof, 3,5,5-trimethyl hexanoyloxybenzene sulphonate, tetraacetyl ethylene diamine (TAED), nonanoyloxybenzene sulphonate (NOBS) and mixtures thereof;

l) said source of hydrogen peroxide comprises a material selected from the group consisting of inorganic perhydrate salts, including alkali metal salts such as sodium salts of perborate (usually mono- or tetra-hydrate), percarbonate, persulphate, perphosphate, persilicate salts and mixtures thereof;

m) said chelant comprises a material selected from the group consisting of DTPA (Diethylene triamine pentaacetic acid), HEDP (Hydroxyethane diphosphonic acid), DTPMP (Diethylene triamine penta(methylene phosphonic acid)), ethylenediaminedisuccinic acid (EDDS), 1,2-Dihydroxybenzene-3,5-disulfonic acid disodium salt hydrate, derivatives of said chelants; and

n) mixtures thereof.

In one aspect of said composition, said composition comprises a fabric hueing agent selected from the group consisting of

a) dyes;
b) dye-clay conjugates comprising at least one cationic-basic dye and a smectite clay; and
c) mixtures thereof.

In one aspect of said composition, said composition comprises a fabric hueing agent selected from the group consisting of

a) small molecule dyes; polymeric dyes and mixtures thereof;
b) dye-clay conjugates comprising at least one cationic-basic dye and a smectite clay; and
c) mixtures thereof.

In one aspect of said solid laundry detergent composition, said composition comprises, based on total composition weight:

a) from about 0.0005 wt% to about 0.1 wt%, from about 0.001 wt% to about 0.05 wt%, or even from about 0.002 wt% to about 0.03 wt% of said protease variant; and
b) one or more of the following:

(i) from about 0.00003 wt% to about 0.1 wt% fabric hueing agent;
(ii) from about 0.001 wt% to about 5 wt %, perfume capsules;
(iii) from about 0.001 wt% to about 1 wt%, cold-water soluble brighteners;
(iv) from about 0.00003 wt% to about 0.1 wt% bleach catalysts;
(v) from about 0.00003 wt% to about 0.1 wt% first wash lipases;
(vi) from about 0.00003 wt% to about 0.1 wt% bacterial cleaning cellulases;
(vii) from about 0.05wt% to about 20 wt% Guerbet nonionic surfactants.

A composition according to any of the consumer products disclosed herein wherein said composition is a single or multi-compartment unit dose is disclosed.

A composition according to any of the consumer products disclosed herein wherein said composition is a multi-compartment unit dose, wherein the protease variant is in a different compartment to any source of hydrogen peroxide and/or chelant.

In the method of the present invention the aqueous wash liquor, comprises a composition as defined in the claims.

In one aspect, such consumer product may be a granular or powder laundry detergent.

In one aspect, any of the consumer products disclosed herein may be in the form of a multi-compartment unit dose. In one aspect of said multi-compartment unit dose, the protease variant may be in a different compartment to any additional enzymes and/or chelant.

In one aspect, such compositions may be a liquid laundry detergent, a dish washing product and/or a granular

or powder detergent.

In one aspect, any of the consumer products disclosed herein may be in the form of a multi-compartment unit dose. In one aspect of said multi-compartment unit dose, the protease may be in a different compartment to any additional enzymes and/or chelant.

The consumer product may take any form including a fluid or solid. The consumer product may be in the form of a unit dose pouch, especially when in the form of a liquid, and typically the consumer product is at least partially, or even completely, enclosed by a water-soluble pouch.

In one or more aspects of the aforementioned consumer product, such consumer product may have any combination of parameters and/or characteristics detailed above.

The charge of the protease variants is expressed relative to subtilisin GG36 protease wild-type having the amino acid sequence of SEQ ID NO:1. The amino acids that impart a single negative charge are D and E and those that impart a single positive charge are R, H and K. Any amino acid change versus SEQ ID NO:1 that changes a charge is used to calculate the charge of the protease variant. For example, introducing a negative charge mutation from a wild-type neutral position will add a net charge of -1 to the protease variant, whereas introducing a negative charge mutation (D or E) from a wild-type positive amino acid residue (R, H or K) will add a net charge of -2. Summing the charge changes from all the amino acid residues that are different for the protease variant versus subtilisin GG36 protease wild-type having the amino acid sequence of SEQ ID NO:1 gives the charge change of the protease variant.

The preferred charge range for proteases to be used in low conductivity laundry detergent solutions is -5, -4, -3, -2, -1, 0, particularly -2, -1;

The preferred charge range for proteases to be used in high conductivity laundry detergent solutions is +5, +4, +3, +2, +1, 0, particularly +2, +1. By correctly selecting the charge unexpectedly improved levels of cleaning performance can be obtained.

Low conductivity solutions are defined as having a conductivity of from about 0.1 mS/cm to about 3 mS/cm, from about 0.3 mS/cm to about 2.5 mS/cm, or even from about 0.5 mS/cm to about 2 mS/cm.

High conductivity having conductivity solutions are defined as having a conductivity of from about 3 mS/cm to about 30 mS/cm, from about 3.5 mS/cm to about 20 mS/cm, or even from about 4 mS/cm to about 10 mS/cm.

Methods for making cold water proteases

Several suitable methods are known in the art for generating modified polynucleotide sequences of the present invention, including but not limited to site-saturation mutagenesis, scanning mutagenesis, insertional mutagenesis, deletion mutagenesis, random mutagenesis, site-directed mutagenesis, and directed-evolution, as well as various other recombinatorial approaches. The commonly used methods include DNA shuffling (*See*, Stemmer, Proc Natl Acad Sci U S A. 25:10747-51 [1994]), methods based on non-homologous recombination of genes (*e.g*. ITCHY) (Ostermeier et al., Bioorg Med Chem. 7:2139-44 [1999]), SCRATCHY (Lutz et al. Proc Natl Acad Sci USA. 98:11248-53 [2001]), SHIPREC (Sieber et al., Nat Biotechnol., 19:456-60 [2001]),and NRR (Bittker et al., Nat Biotechnol., 20:1024-9 [2001]; Bittker et al., Proc Natl Acad Sci. USA. 101:7011-6 [2004]), and methods that rely on the use of oligonucleotides to insert random and targeted mutations, deletions and/or insertions (Ness et al., Nat Biotechnol. 20:1251-5 [2002]; Coco et al., Nat Biotechnol., 20:1246-50 [2002]; Zha et al., Chembiochem. 3:34-9 [2003], Glaser et al., J Immunol., 149:3903-13 [1992], Sondek and Shortle, Proc Natl Acad Sci USA 89:3581-5 [1992], Yáñez et al., Nucleic Acids Res., 32:e158 [2004], Osuna et al., Nucleic Acids Res., 32:e136 [2004], Gaytán et al., Nucleic Acids Res., 29:E9 [2001], and Gaytán et al., Nucleic Acids Res., 30:e84 [2002]).

In some embodiments, the full-length parent polynucleotide is ligated into an appropriate expression plasmid, and the following mutagenesis method is used to facilitate the construction of the modified protease of the present invention, although other methods may be used. The method is based on that described by Pisarchik *et al*. (Pisarchik et al., Prot. Eng. Des. Select., 20:257-265 [2007]). In some embodiments, an added advantage is provided, in that the restriction enzyme used herein cuts outside its recognition sequence, which allows digestion of practically any nucleotide sequence and precludes formation of a restriction site scar. First, as described herein, a naturally-occurring gene encoding the full-length protease is obtained and sequenced and scanned for one or more points at which it is desired to make a mutation (deletion, insertion, substitution, or a combination thereof) at one or more amino acids. Mutation of the gene in order to change its sequence to conform to the desired sequence is accomplished by primer extension in accord with generally known methods. Fragments to the left and to the right of the desired point(s) of mutation are amplified by PCR and to include the *Eam* 11041 restriction site. The left and right fragments are digested with *Eam* 11041 to generate a plurality of fragments having complementary three base overhangs, which are then pooled and ligated to generate a library of modified sequences containing one or more mutations. This method avoids the occurrence of frame-shift mutations. In addition, this method simplifies the mutagenesis process because all of the oligonucleotides can be synthesized so as to have the same restriction site, and no synthetic linkers are necessary to create the restriction sites as is required by some other methods.

A non-limiting set of methods used to make the cold water proteases are given in examples 31 to 43.

Suitable Fabric Hueing Agents

Fluorescent optical brighteners emit at least some visible light. In contrast, fabric hueing agents can alter the tint of a surface as they absorb at least a portion of the visible light spectrum. Suitable fabric hueing agents include dyes, dye-clay conjugates, and pigments that satisfy the requirements of Test Method 1 in the Test Method Section of the present specification. Suitable dyes include small molecule dyes and polymeric dyes. Suitable small molecule dyes include dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof.

Hueing dyes are formulated to deposit onto fabrics from the wash liquor so as to improve fabric whiteness perception. In one aspect, the hueing agent dye is blue or violet. In one aspect, the shading dye(s) may have a peak absorption wavelength of from 550nm to 650nm, or in one aspect from 570nm to 630nm. A combination of dyes which taken together have the visual effect on the human eye as a single dye having a peak absorption wavelength on polyester of from 550nm to 650nm, or from 570nm to 630nm. This may be provided for example by mixing a red and green-blue dye to yield a blue or violet shade.

Dyes are coloured organic molecules which are soluble in aqueous media that contain surfactants. Dyes are described in 'Industrial Dyes', Wiley VCH 2002, K .Hunger (editor). Dyes are listed in the Color Index International published by Society of Dyers and Colourists and the American Association of Textile Chemists and Colorists. Dyes are typically selected from the classes of basic, acid, hydrophobic, direct and polymeric dyes, and dye-conjugates. Those skilled in the art of detergent formulation are able to select suitable hueing dyes from these publications. Polymeric hueing dyes are commercially available, for example from Milliken, Spartanburg, South Carolina, USA.

Examples of suitable dyes are direct violet 7 , direct violet 9 , direct violet 11, direct violet 26, direct violet 31, direct violet 35, direct violet 40, direct violet 41, direct violet 51, direct violet 66, direct violet 99, acid violet 50, acid blue 9, acid violet 17, acid black 1 , acid red 17, acid blue 29, solvent violet 13, disperse violet 27 disperse violet 26, disperse violet 28, disperse violet 63 and disperse violet 77, basic blue 16, basic blue 65, basic blue 66, basic blue 67, basic blue 71, basic blue 159, basic violet 19, basic violet 35, basic violet 38, basic violet 48; basic blue 3 , basic blue 75, basic blue 95, basic blue 122, basic blue 124, basic blue 141, thiazolium dyes, reactive blue 19, reactive blue 163, reactive blue 182, reactive blue 96, Liquitint® Violet CT (Milliken, Spartanburg, USA) and Azo-CM-Cellulose (Megazyme, Bray, Republic of Ireland).

The aforementioned fabric hueing agents can be used in combination (any mixture of fabric hueing agents can be used). Suitable fabric hueing agents can be purchased from Aldrich, Milwaukee, Wisconsin, USA; Ciba Specialty Chemicals, Basel, Switzerland; BASF, Ludwigshafen, Germany; Dayglo Color Corporation, Mumbai, India; Organic Dyestuffs Corp., East Providence, Rhode Island, USA; Dystar, Frankfurt, Germany; Lanxess, Leverkusen, Germany; Megazyme, Wicklow, Ireland; Clariant, Muttenz, Switzerland; Avecia, Manchester, UK and/or made in accordance with the examples contained herein.

Further suitable hueing agents are described in more detail in US 7,208,459 B2.

Encapsulates

In one aspect of the aforementioned consumer product, said consumer product may comprise, based on total consumer product weight, from about 0.001 wt% to about 5 wt%, from about 0.01 wt% to about 2 wt%, or even from about 0.03 wt% to about 0.5 wt%, of said encapsulate comprising a perfume, in one aspect said encapsulated perfume comprises a perfume microcapsule. Suitable encapsulates typically comprise a core, a shell having an inner and outer surface, said shell encapsulating said core. In one aspect of said encapsulate, said core may comprise a material selected from the group consisting of perfumes; brighteners; dyes; insect repellants; silicones; waxes; flavors; vitamins; fabric softening agents; skin care agents in one aspect, paraffins; enzymes; anti-bacterial agents; bleaches; sensates; and mixtures thereof; and said shell may comprise a material selected from the group consisting of polyethylenes; polyamides; polystyrenes; polyisoprenes; polycarbonates; polyesters; polyacrylates; aminoplasts, in one aspect said aminoplast may comprise a polyureas, polyurethane, and/or polyureaurethane, in one aspect said polyurea may comprise polyoxymethyleneurea and/or melamine formaldehyde; polyolefins; polysaccharides, in one aspect said polysaccharide may comprise alginate and/or chitosan; gelatin; shellac; epoxy resins; vinyl polymers; water insoluble inorganics; silicone; and mixtures thereof.

In one aspect of said encapsulate, said core may comprise perfume.

In one aspect of said encapsulate, said shell may comprise melamine formaldehyde and/or cross linked melamine formaldehyde.

In a one aspect, suitable encapsulates may comprise a core material and a shell, said shell at least partially surrounding said core material, is disclosed. At least 75%, 85% or even 90% of said encapsulates may have a fracture

strength of from about 0.2 MPa to about 10 MPa, from about 0.4 MPa to about 5MPa, from about 0.6 MPa to about 3.5 MPa, or even from about 0.7 MPa to about 3MPa; and a benefit agent leakage of from 0% to about 30%, from 0% to about 20%, or even from 0% to about 5%.

In one aspect, at least 75%, 85% or even 90% of said encapsulates may have a particle size of from about 1 microns to about 80 microns, about 5 microns to 60 microns, from about 10 microns to about 50 microns, or even from about 15 microns to about 40 microns.

In one aspect, at least 75%, 85% or even 90% of said encapsulates may have a particle wall thickness of from about 30 nm to about 250 nm, from about 80 nm to about 180 nm, or even from about 100 nm to about 160 nm.

In one aspect, said encapsulates' core material may comprise a material selected from the group consisting of a perfume raw material and/or optionally a material selected from the group consisting of vegetable oil, including neat and/or blended vegetable oils including caster oil, coconut oil, cottonseed oil, grape oil, rapeseed, soybean oil, corn oil, palm oil, linseed oil, safflower oil, olive oil, peanut oil, coconut oil, palm kernel oil, castor oil, lemon oil and mixtures thereof; esters of vegetable oils, esters, including dibutyl adipate, dibutyl phthalate, butyl benzyl adipate, benzyl octyl adipate, tricresyl phosphate, trioctyl phosphate and mixtures thereof; straight or branched chain hydrocarbons, including those straight or branched chain hydrocarbons having a boiling point of greater than about 80 °C; partially hydrogenated terphenyls, dialkyl phthalates, alkyl biphenyls, including monoisopropylbiphenyl, alkylated naphthalene, including dipropylnaphthalene, petroleum spirits, including kerosene, mineral oil and mixtures thereof; aromatic solvents, including benzene, toluene and mixtures thereof; silicone oils; and mixtures there of.

In one aspect, said encapsulates' wall material may comprise a suitable resin including the reaction product of an aldehyde and an amine, suitable aldehydes include, formaldehyde. Suitable amines include melamine, urea, benzoguanamine, glycoluril, and mixtures thereof. Suitable melamines include, methylol melamine, methylated methylol melamine, imino melamine and mixtures thereof. Suitable ureas include, dimethylol urea, methylated dimethylol urea, urea-resorcinol, and mixtures thereof.

In one aspect, suitable formaldehyde scavengers may be employed with the encapsulates, for example, in a capsule slurry and/or added to a consumer product before, during or after the encapsulates are added to such consumer product.

Suitable capsules that can be made by following the teaching of USPA 2008/0305982 A1; and/or USPA 2009/0247449 A1. Alternatively, suitable capsules can be purchased from Appleton Papers Inc. of Appleton, Wisconsin USA.

In addition, the materials for making the aforementioned encapsulates can be obtained from Solutia Inc. (St Louis, Missouri U.S.A.), Cytec Industries (West Paterson, New Jersey U.S.A.), sigma-Aldrich (St. Louis, Missouri U.S.A.), CP Kelco Corp. of San Diego, California, USA; BASF AG of Ludwigshafen, Germany; Rhodia Corp. of Cranbury, New Jersey, USA; Hercules Corp. of Wilmington, Delaware, USA; Agrium Inc. of Calgary, Alberta, Canada, ISP of New Jersey U.S.A., Akzo Nobel of Chicago, IL, USA; Stroever Shellac Bremen of Bremen, Germany; Dow Chemical Company of Midland, MI, USA; Bayer AG of Leverkusen, Germany; Sigma-Aldrich Corp., St. Louis, Missouri, USA.

Method of Making Variant of Parent Protease

A non-limiting set of methods used to make the proteases is given in examples 31 to 43.

Protease Amino Acid Numbering, Enzyme Nomenclature and additional definitions

The numbering of amino acid positions used in this patent is the *Bacillus amyloliquefaciens* subtilisin BPN' numbering system. Each amino acid position of each protease variant, including each protease variant, is numbered according to the numbering of corresponding amino acid position in the amino acid sequence of *Bacillus amyloliquefaciens* subtilisin BPN' shown in Figure 1 as determined by alignment of the variant protease amino acid sequence with the *Bacillus amyloliquefaciens* subtilisin BPN' amino acid sequence.

An alternative numbering scheme is numbering the specific amino acid sequence of the B. lentus subtilisin GG36 protease, having the amino acid sequence of SEQ ID NO:1. None of the amino acid positions of the protease variants, including the protease variants, described herein are numbered using this alternative numbering scheme.

In describing protease variants herein, the following nomenclature is used for ease of reference: Original amino acid(s):position(s):substituted amino acid(s).

Mutations are named by the one letter code for the parent amino acid, followed by a three digit position number and then the one letter code for the variant amino acid. For example, mutating glycine (G) at position 87 to serine (S) is represented as "G087S" or "G87S". Multiple mutations are indicated by inserting a "-" between the mutations. Mutations at positions 87 and 90 are represented as either "G087S-A090Y" or "G87S-A90Y" or "G87S + A90Y" or "G087S + A090Y". For deletions, the one letter code "Z" is used. For an insertion relative to the parent sequence, the one letter code "Z" is on the left side of the position number. For a deletion, the one letter code "Z" is on the right side of the position

number. For insertions, the position number is the position number before the inserted amino acid(s), plus 0.01 for each amino acid. For example, an insertion of three amino acids alanine (A), serine (S) and tyrosine (Y) between position 87 and 88 is shown as "Z087.01A-Z087.02S-Z087.03Y." Thus, combining all the mutations above plus a deletion at position 100 is: "G087S- Z087.01A-Z087.02S-Z087.03Y-A090Y-A100Z."

In all cases, the accepted IUPAC single letter or triple letter amino acid abbreviation is employed. The single letter X refers to any of the twenty amino acids.

The term "wild-type" in reference to an amino acid sequence or nucleic acid sequence indicates that the amino acid sequence or nucleic acid sequence is native or naturally occurring sequence. As used herein, the term "naturally-occurring" refers to anything (*e.g*., proteins, amino acids, or nucleic acid sequences) that are found in nature (*i.e*., have not been manipulated by means of recombinant methods). As used herein, the term, "non-naturally occurring" refers to anything that is not found in nature (*e.g*., recombinant nucleic acids produced in the laboratory).

As used herein with regard to amino acid residue positions, "corresponding to" or "corresponds to" or "corresponds" refers to an amino acid residue at the enumerated position in a protein or peptide, or an amino acid residue that is analogous, homologous, or equivalent to an enumerated residue in a protein or peptide. As used herein, "corresponding region" generally refers to an analogous position along related proteins or a reference protein.

The terms "derived from" and "obtained from" refer to not only a protease produced or producible by a strain of the organism in question, but also a protease encoded by a DNA sequence isolated from such strain and produced in a host organism containing such DNA sequence. Additionally, the term refers to a protease which is encoded by a DNA sequence of synthetic and/or cDNA origin and which has the identifying characteristics of the protease in question. To exemplify, "proteases derived from *Bacillus*" refers to those enzymes having proteolytic activity which are naturally-produced by *Bacillus,* as well as to serine proteases like those produced by *Bacillus* sources but which through the use of genetic engineering techniques are produced by *non-Bacillus* organisms transformed with a nucleic acid encoding the serine proteases.

The term "identical" in the context of two nucleic acids or polypeptide sequences refers to the residues in the two sequences that are the same when aligned for maximum correspondence, as measured using one of the following sequence comparison or analysis algorithms.

As used herein, "homologous genes" refers to a pair of genes from different, but usually related species, which correspond to each other and which are identical or very similar to each other. The term encompasses genes that are separated by speciation (i.e., the development of new species) (e.g., orthologous genes), as well as genes that have been separated by genetic duplication (e.g., paralogous genes).

The term "mature" form of a protein, polypeptide, or peptide refers to the functional form of the protein, polypeptide, or peptide without the signal peptide sequence and propeptide sequence.

As used herein, "homology" refers to sequence similarity or identity, with identity being preferred. Homology may be determined using standard techniques known in the art (*See e.g.*, Smith and Waterman, Adv. Appl. Math. 2:482 [1981]; Needleman and Wunsch, J. Mol. Biol. 48:443 [1970\; Pearson and Lipman, Proc. Natl. Acad. Sci. USA 85:2444 [1988]; software programs such as GAP, BESTFIT, FASTA, and TFASTA in the Wisconsin Genetics Software Package (Genetics Computer Group, Madison, WI); and Devereux et al., Nucl. Acid Res. 12:387-395 [1984]). One example of a useful algorithm is PILEUP. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle (*See*, Feng and Doolittle, J. Mol. Evol. 35:351-360 [1987]). The method is similar to that described by Higgins and Sharp (See, Higgins and Sharp, CABIOS 5:151-153 [1989]). Useful PILEUP parameters including a default gap weight of 3.00, a default gap length weight of 0.10, and weighted end gaps. Another example of a useful algorithm is the BLAST algorithm, described by Altschul *et al*., (*See,* Altschul et al., J. Mol. Biol. 215:403-410 [1990]; and Karlin and Altschul, Proc. Natl. Acad. Sci. USA 90:5873-5787 [1993]). A particularly useful BLAST program is the WU-BLAST-2 program (*See,* Altschul et al., Meth. Enzymol. 266:460-480 [1996]). WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched. However, the values may be adjusted to increase sensitivity.

The percent sequence identity between a reference sequence and a test sequence of interest may be readily determined by one skilled in the art. The percent identity shared by polynucleotide or polypeptide sequences is determined by direct comparison of the sequence information between the molecules by aligning the sequences and determining the identity by methods known in the art. An example of an algorithm that is suitable for determining sequence similarity is the BLAST algorithm, (*See,* Altschul, et al., J. Mol. Biol., 215:403-410 [1990]). Software for performing BLAST analyses is publicly available through the National Center for Biotechnology Information. This algorithm involves first identifying high scoring sequence pairs (HSPs) by identifying short words of length W in the query sequence that either match or satisfy some positive-valued threshold score T when aligned with a word of the same length in a database sequence.

These initial neighborhood word hits act as starting points to find longer HSPs containing them. The word hits are expanded in both directions along each of the two sequences being compared for as far as the cumulative alignment score can be increased. Extension of the word hits is stopped when: the cumulative alignment score falls off by the quantity X from a maximum achieved value; the cumulative score goes to zero or below; or the end of either sequence is reached. The BLAST algorithm parameters W, T, and X determine the sensitivity and speed of the alignment. The BLAST program uses as defaults a wordlength (W) of 11, the BLOSUM62 scoring matrix (*See*, Henikoff and Henikoff, Proc. Natl. Acad. Sci. USA 89:10915 [1992]) alignments (B) of 50, expectation (E) of 10, M'5, N'-4, and a comparison of both strands.

The BLAST algorithm then performs a statistical analysis of the similarity between two sequences (*See e.g.*, Karlin and Altschul, *supra*). One measure of similarity provided by the BLAST algorithm is the smallest sum probability (P(N)), which provides an indication of the probability by which a match between two nucleotide or amino acid sequences would occur by chance. For example, a nucleic acid is considered similar to a serine protease nucleic acid of this disclosure if the smallest sum probability in a comparison of the test nucleic acid to a serine protease nucleic acid is less than about 0.1, more preferably less than about 0.01, and most preferably less than about 0.001. Where the test nucleic acid encodes a serine protease polypeptide, it is considered similar to a specified serine protease nucleic acid if the comparison results in a smallest sum probability of less than about 0.5, and more preferably less than about 0.2.

Percent "identical" or "identity" in the context of two or more nucleic acid or polypeptide sequences refers to two or more sequences that are the same or have a specified percentage of nucleic acid residues or amino acid residues, respectively, that are the same, when compared and aligned for maximum similarity, as determined using a sequence comparison algorithm or by visual inspection. "Percent sequence identity" or "% identity" or "% sequence identity or "% amino acid sequence identity" of a subject amino acid sequence to a reference (*i.e.*, query) amino acid sequence means that the subject amino acid sequence is identical (*i.e.*, on an amino acid-by-amino acid basis) by a specified percentage to the query amino acid sequence over a comparison length when the sequences are optimally aligned. Thus, 80% amino acid sequence identity or 80% identity with respect to two amino acid sequences means that 80% of the amino acid residues in two optimally aligned amino acid sequences are identical.

"Percent sequence identity" or "% identity" or "% sequence identity or "% nucleotide sequence identity" of a subject nucleic acid sequence to a reference (*i.e.* query) nucleic acid sequence means that the subject nucleic acid sequence is identical (*i.e.*, on a nucleotide-by-nucleotide basis for a polynucleotide sequence) by a specified percentage to the query sequence over a comparison length when the sequences are optimally aligned. Thus, 80% nucleotide sequence identity or 80% identity with respect to two nucleic acid sequences means that 80% of the nucleotide residues in two optimally aligned nucleic acid sequences are identical.

In some embodiments, the percent sequence identity or % sequence identity" or "% identity" of a subject sequence to a query sequence can be calculated by optimally aligning the two sequences and comparing the two optimally aligned sequences over the comparison length. The number of positions in the optimal alignment at which identical residues occur in both sequences are determined, thereby providing the number of matched positions, and the number of matched positions is then divided by the total number of positions of the comparison length (which, unless otherwise specified, is the length of the query sequence). The resulting number is multiplied by 100 to yield the percent sequence identity of the subject sequence to the query sequence.

"Optimal alignment" or "optimally aligned" refers to the alignment of two (or more) sequences giving the highest percent identity score. For example, optimal alignment of two protein sequences can be achieved by manually aligning the sequences such that the maximum number of identical amino acid residues in each sequence are aligned together or by using software programs or procedures described herein or known in the art. Optimal alignment of two nucleic acid sequences can be achieved by manually aligning the sequences such that the maximum number of identical nucleotide residues in each sequence are aligned together or by using software programs or procedures described herein or known in the art.

In some embodiments, two polypeptide sequences are deemed "optimally aligned" when they are aligned using defined parameters, such as a defined amino acid substitution matrix, gap existence penalty (also termed gap open penalty), and gap extension penalty, so as to achieve the highest similarity score possible for that pair of sequences. The BLOSUM62 scoring matrix (*See*, Henikoff and Henikoff, *supra*) is often used as a default scoring substitution matrix in polypeptide sequence alignment algorithms (*e.g.*, BLASTP). The gap existence penalty is imposed for the introduction of a single amino acid gap in one of the aligned sequences, and the gap extension penalty is imposed for each residue position in the gap. Exemplary alignment parameters employed are: BLOSUM62 scoring matrix, gap existence penalty=11, and gap extension penalty=1. The alignment score is defined by the amino acid positions of each sequence at which the alignment begins and ends (e.g., the alignment window), and optionally by the insertion of a gap or multiple gaps into one or both sequences, so as to achieve the highest possible similarity score.

Optimal alignment between two or more sequences can be determined manually by visual inspection or by using a computer, such as, but not limited to for example, the BLASTP program for amino acid sequences and the BLASTN program for nucleic acid sequences (*See e.g.*, Altschul et al., Nucleic Acids Res. 25(17):3389-3402 (1997);

*See also,* the National Center for Biotechnology Information (NCBI) website).

Non- immunoequivalent Enzyme and/or Additional Enzymes

The consumer products can comprise one or more enzymes which provide cleaning performance and/or fabric care benefits. Examples of suitable enzymes include, but are not limited to, hemicellulases, peroxidases, proteases, cellulases, xylanases, lipases, phospholipases, esterases, cutinases, pectinases, mannanases, pectate lyases, keratinases, reductases, oxidases, phenoloxidases, lipoxygenases, ligninases, pullulanases, tannases, pentosanases, malanases, β-glucanases, arabinosidases, hyaluronidase, chondroitinase, laccase, and amylases, or mixtures thereof. A typical combination is an enzyme cocktail that may comprise, for example, a protease and lipase in conjunction with amylase. When present in a consumer product, the aforementioned additional enzymes may be present at levels from about 0.00001% to about 2%, from about 0.0001% to about 1% or even from about 0.001% to about 0.5% enzyme protein by weight of the consumer product.

In one aspect suitable enzymes would include a protease. Suitable proteases include metalloproteases and serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:

(a) subtilisins (EC 3.4.21.62), including those derived from Bacillus, such as Bacillus lentus, B. alkalophilus, B. subtilis, B. amyloliquefaciens, Bacillus pumilus and Bacillus gibsonii described in US 6,312,936 B1, US 5,679,630, US 4,760,025, US 7,262,042 and WO09/021867.
(b) trypsin-type or chymotrypsin-type proteases, such as trypsin (*e.g.*, of porcine or bovine origin), including the Fusarium protease described in US5288627 and the chymotrypsin proteases derived from Cellumonas described in US PA 2008/0063774A1.
(c) metalloproteases, including those derived from Bacillus amyloliquefaciens described in US PA 2008/0293610A1.

Suitable proteases include those derived from Bacillus gibsonii or Bacillus Lentus.

Suitable commercially available protease enzymes include those sold under the trade names Alcalase®, Savinase®, Primase®, Durazym®, Polarzyme®, Kannase®, Liquanase®, Liquanase Ultra®, Savinase Ultra®, Ovozyme®, Neutrase®, Everlase® and Esperase® by Novozymes A/S (Denmark), those sold under the tradename Maxatase®, Maxacal®, Maxapem®, Properase®, Purafect®, Purafect Prime®, Purafect Ox®, FN3®, FN4®, Excellase® and Purafect OXP® by Genencor International, those sold under the tradename Opticlean® and Optimase® by Solvay Enzymes, those available from Henkel/ Kemira, namely BLAP (sequence shown in Figure 29 of US 5,352,604 with the folowing mutations S99D + S101 R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D) - all from Henkel/Kemira; and KAP (Bacillus alkalophilus subtilisin with mutations A230V + S256G + S259N) from Kao.

In one aspect, the consumer product may comprise a protease that is not immunoequivalent to the protease of this invention. For the purposes of this disclosure an immunoequivalent protease will have a high degree of identity (>80%) with BPN' and will cross-react with the same antibody. Suitable non-immunoequivalent enzymes will include those derived from Bacillus Lentus, Bacillus gibsonii and the metalloprotease derived from Bacillus amyloliquefaciens.

Suitable alpha-amylases include those of bacterial or fungal origin. Chemically or genetically modified mutants (variants) are included. A suitable alkaline alpha-amylase may be derived from a strain of Bacillus, such as Bacillus licheniformis, Bacillus amyloliquefaciens, Bacillus stearothermophilus, Bacillus subtilis, or other Bacillus sp., such as Bacillus sp. NCIB 12289, NCIB 12512, NCIB 12513, DSM 9375 (USP 7,153,818) DSM 12368, DSMZ no. 12649, KSM AP1378 (US 6,979,731), KSM K36 or KSM K38 (US 6,916,645). Suitable amylases include:

(a) the variants described in WO 94/02597, US6,297,037, US 7,378,264 and US5,763,385, especially the variants with substitutions in one or more of the following positions versus the enzyme listed as SEQ ID No. 2 in US 7,378,264: 15, 23, 105, 106, 124, 128, 133, 154, 156, 181 , 188, 190, 197, 202, 208, 209, 243, 264, 304, 305, 391, 408, and 444.
(b) the variants described in USP 5,856,164 and US 6,673,589, US 6,093,562, US 6,187,576 and US PA 2008/0193999A1, especially the variants with one or more substitutions in the following positions versus the AA560 enzyme listed as SEQ ID No. 12 in US PA 2008/0193999A1:
26, 30, 33, 82, 37, 106, 118, 128, 133, 149, 150, 160, 178, 182, 186, 193, 203, 214, 231, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, 484, preferably that also contain the deletions of D183* and G184*.

(c) variants exhibiting at least 90% identity with SEQ ID No. 4 in US PA 2008/0193999A1 the wild-type enzyme from Bacillus SP722, especially variants with deletions in the 183 and 184 positions and variants described in US 6,187,576.

(d) variants exhibiting at least 95% identity with the wild-type enzyme from Bacillus sp.707 (SEQ ID NO:7 in US 6,093, 562), especially those comprising one or more of the following mutations M202, M208, S255, R172, and/or M261. In one aspect, said amylase comprises one or more of M202L, M202V, M202S, M202T, M202I, M202Q, M202W, S255N and/or R172Q. Particularly suitable are those comprising the M202L or M202T mutations.

Suitable commercially available alpha-amylases include DURAMYL®, LIQUEZYME®, TERMAMYL®, TERMAMYL ULTRA®, NATALASE®, SUPRAMYL®, STAINZYME®, STAINZYME PLUS®, FUNGAMYL® and BAN® (Novozymes A/S, Bagsvaerd, Denmark), KEMZYM® AT 9000 Biozym Biotech Trading GmbH Wehlistrasse 27b A-1200 Wien Austria, RAPIDASE®, PURASTAR®, ENZYSIZE®, OPTISIZE HT PLUS® and PURASTAR OXAM® (Genencor International Inc., Palo Alto, California) and KAM® (Kao, 14-10 Nihonbashi Kayabacho, 1-chome, Chuo-ku Tokyo 103-8210, Japan). In one aspect, suitable amylases include NATALASE®, STAINZYME® and STAINZYME PLUS® and mixtures thereof.

In one aspect, such additional enzyme may be selected from the group consisting of: lipases, including "first cycle lipases" such as those described in U.S. Patent 6,939,702 B1 and US PA 2009/0217464. In one aspect, the lipase is a first-wash lipase, in one aspect, a variant of the wild-type lipase from Thermomyces lanuginosus comprising T231R and N233R mutations. The wild-type sequence is the 269 amino acids (amino acids 23 - 291) of the Swissprot accession number Swiss-Prot O59952 (derived from Thermomyces lanuginosus (Humicola lanuginosa)). Suitable lipases would include those sold under the tradenames Lipex®, Lipolex® and Lipoclean® by Novozymes, Bagsvaerd, Denmark.

In one aspect, the composition comprises a variant of Thermomyces lanuginosa lipase having >90% identity with the wild type amino acid and comprising substitution(s) at T231 and/or N233, in one aspect, T231R and N233R.

In one aspect, other enzymes include cellulases of bacterial or fungal origin. Chemically modified or protein engineered mutants are included. Suitable cellulases include cellulases from the genera Bacillus, Pseudomonas, Humicola, Fusarium, Thielavia, Acremonium, e.g., the fungal cellulases produced from Humicola insolens, Myceliophthora thermophila and Fusarium oxysporum disclosed in US 4,435,307, US 5,648,263, US 5,691,178, US 5,776,757 and US 5,691,178. Suitable cellulases include the alkaline or neutral cellulases having colour care benefits. Examples of such cellulases are cellulases described in US 5,520,838, US 5,948,672, US 5,919,691, US 6,001,639, WO 98/08940. Other examples are cellulase variants such as those described in US 6,114,296, US 5,457,046, US 5,457,046, US 5,686,593, US 5,763,254, US 6,117,664, US PA 2009/0170747A1 and PCT/DK98/00299. Commercially available cellulases include CELLUZYME®, and CAREZYME® (Novozymes A/S), CLAZINASE®, and PURADAX HA® (Genencor International Inc.), and KAC-500(B)® (Kao Corporation).

In one aspect, the cellulase may be a bacterial cleaning cellulase. Such bacterial cleaning cellulases are endo beta 1,4- glucanases and have a structure which does not comprise a class A Carbohydrate Binding Module (CBM). A class A CBM is defined according to A. B. Boraston et al. Biochemical Journal 2004, Volume 382 (part 3) pages 769-781. In particular, the cellulase does not comprise a class A CBM from families 1, 2a, 3, 5 and 10.

In one aspect, the bacterial cleaning cellulase may be a glycosyl hydrolase having enzymatic activity towards amorphous cellulose substrates, wherein the glycosyl hydrolase is selected from GH families 5, 7, 12, 16, 44 or 74. In one aspect, the cellulase may be a glycosyl hydrolase selected from GH family 5. In one aspect, the cellulase may be Celluclean®, supplied by Novozymes. This cellulase is described in more detail in US 7,141,403. The glycosyl hydrolase (GH) family definition is described in more detail in Biochem J. 1991, v280, 309-316.

Another suitable bacterial cleaning cellulase is a glycosyl hydrolase having enzymatic activity towards both xyloglucan and amorphous cellulose substrates, wherein the glycosyl hydrolase is selected from GH families 5, 12, 44 or 74. In one aspect, the glycosyl hydrolase selected from GH family 44. The glycosyl hydrolase enzyme may belong to glycosyl hydrolase family 44.

Suitable glycosyl hydrolases may be selected from the group consisting of: GH family 44 glycosyl hydrolases from *Paenibacillus polyxyma* (wild-type) such as XYG1006 described in US 7,361,736 or are variants thereof; GH family 12 glycosyl hydrolases from Bacillus licheniformis (wild-type) such as Seq. No. ID: 1 described in US 6,268,197 or are variants thereof; GH family 5 glycosyl hydrolases from Bacillus agaradhaerens (wild type) or variants thereof; GH family 5 glycosyl hydrolases from Paenibacillus (wild type) such as XYG1034 and XYG 1022 described in US 6,630,340 or variants thereof; GH family 74 glycosyl hydrolases from Jonesia sp. (wild type) such as XYG1020 described in WO 2002/077242 or variants thereof; and GH family 74 glycosyl hydrolases from Trichoderma Reesei (wild type), such as the enzyme described in more detail in Sequence ID NO. 2 of US 7,172,891, or variants thereof.

Suitable glycosyl hydrolases may be selected from the group consisting of: GH family 44 glycosyl hydrolases from *Paenibacillus polyxyma* (wild-type) such as XYG1006 or are variants thereof.

Suitable bacterial cleaning cellulases are sold under the tradenames Celluclean® and Whitezyme® (Novozymes A/S, Bagsvaerd, Denmark).

In one aspect, the composition may comprise a fungal cleaning cellulase belonging to Glycosyl Hydrolase family 45 having a molecular weight of from 17kDa to 30 kDa, for example the endoglucanases sold under the tradename Biotouch® NCD, DCC and DCL (AB Enzymes, Darmstadt, Germany).

Other suitable enzymes include pectate lyases sold under the tradenames Pectawash®, Pectaway® and mannanases sold under the tradenames Mannaway® (all from Novozymes A/S, Bagsvaerd, Denmark), and Purabrite® (Genencor International Inc., Palo Alto, California).

In one aspect, the composition comprises a Guerbet nonionic surfactant. The Guerbet nonionic surfactant is a secondary alcohol-based detersive surfactant having the formula:

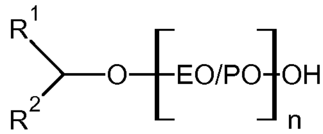

wherein $R^1$ = linear or branched, substituted or unsubstituted, saturated or unsaturated $C_{2-8}$ alkyl;
wherein $R^2$ = linear or branched, substituted or unsubstituted, saturated or unsaturated $C_{2-8}$ alkyl,
wherein the total number of carbon atoms present in $R^1$ + $R^2$ moieties is in the range of from 7 to 13; wherein EO/PO are alkoxy moieties selected from ethoxy, propoxy, or mixtures thereof;
wherein n is the average degree of alkoxylation and is in the range of from 4 to 10.

Builders

Commercially available laundry detergents comprise strong inorganic builder, with either phosphate builder typically sodium tripolyphosphate (STPP), or zeolite typically sodium aluminosilicate builder being used as the predominant strong builder. Generally such strong builders are present at relatively high levels such as 15 to 20 wt% or even higher, for example even up to 40 wt%. In accordance with a preferred aspect of the present invention, the amount of strong builder selected from phosphate and/or zeolite builder is no greater than 10 wt% based on the total weight of the detergent composition, preferably below 8 wt%, or even more preferably below 5 or 4 or 3 or 2 or 1 wt% .

Thus, preferred compositions of the invention are low builder compostions comprising from 0 wt% to 10wt% zeolite builder, and 0 wt% to 10 wt% phosphate builder, the total amount of phosphate and/or zeolite not exceeding 10 wt%, and preferably being below 10 wt% as described above. Preferably the compositions of the invention comprise from 0 wt% to 8 wt%, or from 0 wt% to 5 or 4 wt%, or from 0 wt% to 3 or even less than 2 wt% zeolite builder. It may even be preferred for the composition to be essentially free from zeolite builder. By essentially free from zeolite builder it is typically meant that the composition comprises no deliberately added zeolite builder. This is especially preferred if it is desirable for the composition to be very highly soluble, to minimise the amount of water-insoluble residues (for example, which may deposit on fabric surfaces), and also when it is highly desirable to have transparent wash liquor. Zeolite builders include zeolite A, zeolite X, zeolite P and zeolite MAP.

The compositions of the invention may comprise from 0 wt% to 10 wt% phosphate builder. The composition preferably comprises from 0 wt% to 8 wt%, or from 0 wt% to 5 or 4 wt%, or from 0 wt% to 3 or even 2 wt% phosphate builder. It may even be preferred for the composition to be essentially free from phosphate builder. By essentially free from phosphate builder it is typically meant that the composition comprises no deliberately added phosphate builder. This is especially preferred if it is desirable for the composition to have a very good environmental profile. Phosphate builders include sodium tripolyphosphate.

In a further preferred aspect of the invention, the total level of weak builders selected from layered silicate (SKS-6), citric acid, citrate salts and nitrilo triacetic acid or salt thereof is below 15 wt%, more preferably below 8 wt%, more preferably below 4 wt% or even below 3 or 2 wt% based on the total weight of the detergent composition. Typically the level of each of layered silicate, citric acid, citrate salts and nitrilo triacetic acid or salt thereof will be below 10 wt% or even below 5 wt% or wt% based on the total weight of the composition.

Although builders bring several benefits to the formulator, their main role is to sequester divalent metal ions (such as calcium and magnesium ions) from the wash solution that would otherwise interact negatively with the surfactant system. Builders are also effective at removing metal ions and inorganic soils from the fabric surface too, leading to improved removal of particulate and beverage stains. It would therefore be expected that reduction of their levels would negatively impact on cleaning performance and therefore, preparation of detergent compositions that are effective with the claimed reduced levels of phosphate and zeolite builders is surprising.

Reserve Alkalinity

As used herein, the term "reserve alkalinity" is a measure of the buffering capacity of the detergent composition (g/NaOH/100g detergent composition) determined by titrating a 1% (w/v) solution of detergent composition with hydrochloric acid to pH 7.5 i.e in order to calculate Reserve Alkalinity as defined herein:

$$\textbf{Reserve Alkalinity} \text{ (to pH 7.5) as \% alkali in g NaOH/100 g product} = \frac{T \times M \times 40 \times Vol}{10 \times Wt \times Aliquot}$$

| | | |
|---|---|---|
| T | = | titre (ml) to pH 7.5 |
| M | = | Molarity of HCl = 0.2 |
| 40 | = | Molecular weight of NaOH |
| Vol | = | Total volume (ie. 1000 ml) |
| W | = | Weight of product (10 g) |
| Aliquot | = | (100 ml) |

Obtain a 10g sample accurately weighed to two decimal places, of fully formulated detergent composition. The sample should be obtained using a Pascall sampler in a dust cabinet. Add the 10g sample to a plastic beaker and add 200 ml of carbon dioxide-free deionised water. Agitate using a magnetic stirrer on a stirring plate at 150 rpm until fully dissolved and for at least 15 minutes. Transfer the contents of the beaker to a 1 litre volumetric flask and make up to 1 litre with deionised water. Mix well and take a 100 mls ± 1 ml aliquot using a 100 mls pipette immediately. Measure and record the pH and temperature of the sample using a pH meter capable of reading to ±0.01pH units, with stirring, ensuring temperature is 21°C +/- 2°C. Titrate whilst stirring with 0.2M hydrochloric acid until pH measures exactly 7.5. Note the millilitres of hydrochloric acid used. Take the average titre of three identical repeats. Carry out the calculation described above to calculate RA to pH 7.5.

The RA of the detergent compositions of the invention may preferably be greater than 7.5 and preferably greater than 8. The RA may be greater than 9 or even greater than 9.5 or 10 or higher. The RA may be up to 20 or higher.

Adequate reserve alkalinity may be provided, for example, by one or more of alkali metal silicates (excluding crystalline layered silicate), typically amorphous silicate salts, generally 1.2 to 2.2 ratio sodium salts, alkali metal typically sodium carbonate, bicarbonate and/or sesquicarbonates. STPP and persalts such as perborates and percarbonates also contribute to alkalinity. Buffering is necessary to maintain an alkaline pH during the wash process counteracting the acidity of soils, especially fatty acids liberated by the lipase enzyme.

The detergent composition preferably comprises from 0 wt% to 50 wt% silicate salt, more usually 5 to 30 wt% silicate salt, or 7 to 20 wt% silicate salt, usually sodium silicate.

In order to provide the preferred reserve alkalinity the detergent compositions of the invention may comprise a carbonate salt, typically from 1 wt% to 70 wt%, or from 5 wt% to 50 wt% or from 10 wt% to 30 wt% carbonate salt. Preferred carbonate salts are sodium carbonate and/or sodium bicarbonate and/or sodium sesquicarbonate. The carbonate salt may be incorporated into the detergent composition wholly or partially via a mixed salt such as Burkeite. A highly preferred carbonate salt is sodium carbonate. Preferably, the composition may comprise from 5 wt% to 50 wt% sodium carbonate, or from 10 to 40 wt% or even 15 to 35 wt% sodium carbonate. It may also be desired for the composition to comprise from 1wt% to 20 wt% sodium bicarbonate, or even 2 to 10 or 8 wt%.

If zeolite is present, it may be desired for the weight ratio of sodium carbonate and/or sodium silicate to zeolite builder to be at least 5:1, preferably at least 10:1, or at least 15:1, or at least 20:1 or even at least 25:1

The carbonate salt, or at least part thereof, is typically in particulate form, typically having a weight average particle size in the range of from 200 to 500 micrometers. However, it may be preferred for the carbonate salt, or at least part thereof, to be in micronised particulate form, typically having a weight average particle size in the range of from 4 to 40 micrometers; this is especially preferred when the carbonate salt, or at least part thereof, is in the form of a co-particulate admixture with a detersive surfactant, such as an alkoxylated anionic detersive surfactant.

In order to provide the required reserve alkalinity, preferably the levels of carbonate and/or silicate salts, typically sodium carbonate and sodium silicate will be from 10 to 70 wt%, or from 10 or even 15 to 50 wt% based on the total weight of the composition.

Adjunct materials

In addition to materials previously disclosed herein, and while not essential for the purposes of the present

invention, the non-limiting list of adjuncts illustrated hereinafter are suitable for use in the instant consumer products and may be desirably incorporated in certain embodiments of the invention, for example to assist or enhance cleaning performance, for treatment of the substrate to be cleaned, or to modify the aesthetics of the consumer product as is the case with perfumes, colorants, dyes or the like. The levels of any such adjuncts incorporated in any consumer product are in addition to any materials previously recited for incorporation. The precise nature of these additional components/adjunct materials, and levels of incorporation thereof, will depend on the physical form of the consumer product and the nature of the cleaning operation for which it is to be used. Suitable adjunct materials include, but are not limited to, surfactants, builders, chelating agents, dye transfer inhibiting agents, dispersants, additional enzymes, and enzyme stabilizers, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids, solvents and/or pigments. In addition to the disclosure below, suitable examples of such other adjuncts and levels of use are found in U.S. Patent Nos. 5,576,282, 6,306,812 B1 and 6,326,348 B1.

As stated, the adjunct ingredients are not essential to Applicants' consumer products. Thus, certain embodiments of Applicants' consumer products do not contain one or more of the following adjuncts materials: surfactants, builders, chelating agents, dye transfer inhibiting agents, dispersants, additional enzymes, and enzyme stabilizers, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids, solvents and/or pigments. However, when one or more adjuncts are present, such one or more adjuncts may be present as detailed below:

Bleaching Agents - The consumer products of the present invention may comprise one or more bleaching agents. Suitable bleaching agents other than bleaching catalysts include photobleaches, bleach activators, hydrogen peroxide, sources of hydrogen peroxide, pre-formed peracids and mixtures thereof. In general, when a bleaching agent is used, the consumer products of the present invention may comprise from about 0.1% to about 50% or even from about 0.1% to about 25% bleaching agent by weight of the subject consumer product. Examples of suitable bleaching agents include:

(1) photobleaches for example sulfonated zinc phthalocyanine sulfonated aluminium phthalocyanines, xanthene dyes and mixtures thereof;

(2) preformed peracids: Suitable preformed peracids include, but are not limited to, compounds selected from the group consisting of percarboxylic acids and salts, percarbonic acids and salts, perimidic acids and salts, peroxymonosulfuric acids and salts, for example, Oxone®, and mixtures thereof. Suitable percarboxylic acids include hydrophobic and hydrophilic peracids having the formula R-(C=O)O-O-M wherein R is an alkyl group, optionally branched, having, when the peracid is hydrophobic, from 6 to 14 carbon atoms, or from 8 to 12 carbon atoms and, when the peracid is hydrophilic, less than 6 carbon atoms or even less than 4 carbon atoms; and M is a counterion, for example, sodium, potassium or hydrogen;

(3) sources of hydrogen peroxide, for example, inorganic perhydrate salts, including alkali metal salts such as sodium salts of perborate (usually mono- or tetra-hydrate), percarbonate, persulphate, perphosphate, persilicate salts and mixtures thereof. In one aspect of the invention the inorganic perhydrate salts are selected from the group consisting of sodium salts of perborate, percarbonate and mixtures thereof. When employed, inorganic perhydrate salts are typically present in amounts of from 0.05 to 40 wt%, or 1 to 30 wt% of the overall consumer product and are typically incorporated into such consumer products as a crystalline solid that may be coated. Suitable coatings include, inorganic salts such as alkali metal silicate, carbonate or borate salts or mixtures thereof, or organic materials such as water-soluble or dispersible polymers, waxes, oils or fatty soaps; and

(4) bleach activators having R-(C=O)-L wherein R is an alkyl group, optionally branched, having, when the bleach activator is hydrophobic, from 6 to 14 carbon atoms, or from 8 to 12 carbon atoms and, when the bleach activator is hydrophilic, less than 6 carbon atoms or even less than 4 carbon atoms; and L is leaving group. Examples of suitable leaving groups are benzoic acid and derivatives thereof - especially benzene sulphonate. Suitable bleach activators include dodecanoyl oxybenzene sulphonate, decanoyl oxybenzene sulphonate, decanoyl oxybenzoic acid or salts thereof, 3,5,5-trimethyl hexanoyloxybenzene sulphonate, tetraacetyl ethylene diamine (TAED) and nonanoyloxybenzene sulphonate (NOBS). Suitable bleach activators are also disclosed in US 6,380,144. While any suitable bleach activator may be employed, in one aspect of the invention the subject consumer product may comprise NOBS, TAED or mixtures thereof.

When present, the peracid and/or bleach activator is generally present in the consumer product in an amount of from about 0.1 to about 60 wt%, from about 0.5 to about 40 wt % or even from about 0.6 to about 10 wt% based on the consumer product. One or more hydrophobic peracids or precursors thereof may be used in combination with one or more hydrophilic peracid or precursor thereof.

The amounts of hydrogen peroxide source and peracid or bleach activator may be selected such that the molar

ratio of available oxygen (from the peroxide source) to peracid is from 1:1 to 35:1, or even 2:1 to 10:1.

Surfactants - The consumer products according to the present invention may comprise a surfactant or surfactant system wherein the surfactant can be selected from nonionic surfactants, anionic surfactants, cationic surfactants, ampholytic surfactants, zwitterionic surfactants, semi-polar nonionic surfactants and mixtures thereof. When present, surfactant is typically present at a level of from about 0.1% to about 60%, from about 1% to about 50% or even from about 5% to about 40% by weight of the subject consumer product.

Suitable anionic detersive surfactants include sulphate and sulphonate detersive surfactants.

Suitable sulphonate detersive surfactants include alkyl benzene sulphonate, in one aspect, $C_{10-13}$ alkyl benzene sulphonate. Suitable alkyl benzene sulphonate (LAS)may be obtained, by sulphonating commercially available linear alkyl benzene (LAB); suitable LAB includes low 2-phenyl LAB, such as those supplied by Sasol under the tradename Isochem® or those supplied by Petresa under the tradename Petrelab®, other suitable LAB include high 2-phenyl LAB, such as those supplied by Sasol under the tradename Hyblene®. A suitable anionic detersive surfactant is alkyl benzene sulphonate that is obtained by DETAL catalyzed process, although other synthesis routes, such as HF, may also be suitable.

Suitable sulphate detersive surfactants include alkyl sulphate, in one aspect, $C_{8-18}$ alkyl sulphate, or predominantly $C_{12}$ alkyl sulphate.

Another suitable sulphate detersive surfactant is alkyl alkoxylated sulphate, in one aspect, alkyl ethoxylated sulphate, in one aspect, a $C_{8-18}$ alkyl alkoxylated sulphate, in another aspect,a $C_{8-18}$ alkyl ethoxylated sulphate, typically the alkyl alkoxylated sulphate has an average degree of alkoxylation of from 0.5 to 20, or from 0.5 to 10, typically the alkyl alkoxylated sulphate is a $C_{8-18}$ alkyl ethoxylated sulphate having an average degree of ethoxylation of from 0.5 to 10, from 0.5 to 7, from 0.5 to 5 or even from 0.5 to 3.

The alkyl sulphate, alkyl alkoxylated sulphate and alkyl benzene sulphonates may be linear or branched, substituted or un-substituted.

The detersive surfactant may be a mid-chain branched detersive surfactant, in one aspect, a mid-chain branched anionic detersive surfactant, in one aspect, a mid-chain branched alkyl sulphate and/or a mid-chain branched alkyl benzene sulphonate, for example a mid-chain branched alkyl sulphate. In one aspect, the mid-chain branches are $C_{1-4}$ alkyl groups, typically methyl and/or ethyl groups.

Suitable non-ionic detersive surfactants are selected from the group consisting of: $C_8$-$C_{18}$ alkyl ethoxylates, such as, NEODOL® non-ionic surfactants from Shell; $C_6$-$C_{12}$ alkyl phenol alkoxylates wherein the alkoxylate units may be ethyleneoxy units, propyleneoxy units or a mixture thereof; $C_{12}$-$C_{18}$ alcohol and $C_6$-$C_{12}$ alkyl phenol condensates with ethylene oxide/propylene oxide block polymers such as Pluronic® from BASF; $C_{14}$-$C_{22}$ mid-chain branched alcohols; $C_{14}$-$C_{22}$ mid-chain branched alkyl alkoxylates, typically having an average degree of alkoxylation of from 1 to 30; alkylpolysaccharides, in one aspect, alkylpolyglycosides; polyhydroxy fatty acid amides; ether capped poly(oxyalkylated) alcohol surfactants; and mixtures thereof.

Suitable non-ionic detersive surfactants include alkyl polyglucoside and/or an alkyl alkoxylated alcohol.

In one aspect, non-ionic detersive surfactants include alkyl alkoxylated alcohols, in one aspect $C_{8-18}$ alkyl alkoxylated alcohol, for example a $C_{8-18}$ alkyl ethoxylated alcohol, the alkyl alkoxylated alcohol may have an average degree of alkoxylation of from 1 to 50, from 1 to 30, from 1 to 20, or from 1 to 10. In one aspect, the alkyl alkoxylated alcohol may be a $C_{8-18}$ alkyl ethoxylated alcohol having an average degree of ethoxylation of from 1 to 10, from 1 to 7, more from 1 to 5 or from 3 to 7. The alkyl alkoxylated alcohol can be linear or branched, and substituted or un-substituted.

Suitable cationic detersive surfactants include alkyl pyridinium compounds, alkyl quaternary ammonium compounds, alkyl quaternary phosphonium compounds, alkyl ternary sulphonium compounds, and mixtures thereof.

Suitable cationic detersive surfactants are quaternary ammonium compounds having the general formula:

$$(R)(R_1)(R_2)(R_3)N^+ X^-$$

wherein, R is a linear or branched, substituted or unsubstituted $C_{6-18}$ alkyl or alkenyl moiety, $R_1$ and $R_2$ are independently selected from methyl or ethyl moieties, $R_3$ is a hydroxyl, hydroxymethyl or a hydroxyethyl moiety, X is an anion which provides charge neutrality, suitable anions include: halides, for example chloride; sulphate; and sulphonate. Suitable cationic detersive surfactants are mono-$C_{6-18}$ alkyl mono-hydroxyethyl di-methyl quaternary ammonium chlorides. Highly suitable cationic detersive surfactants are mono-$C_{8-10}$ alkyl mono-hydroxyethyl di-methyl quaternary ammonium chloride, mono-$C_{10-12}$ alkyl mono-hydroxyethyl di-methyl quaternary ammonium chloride and mono-Cio alkyl mono-hydroxyethyl di-methyl quaternary ammonium chloride.

Builders - The consumer products of the present invention may comprise one or more detergent builders or builder systems. When a builder is used, the subject consumer product will typically comprise at least about 1%, from about 2% to about 60% or even from about 5% to about 10% builder by weight of the subject consumer product. The composition may even be substantially free of builder; substantially free means "no deliberately added" zeolite and/or phosphate. Typical zeolite builders include zeolite A, zeolite P and zeolite MAP. A typical phosphate builder is sodium

tri-polyphosphate.

Chelating Agents - The consumer products herein may contain a chelating agent. Suitable chelating agents include copper, iron and/or manganese chelating agents and mixtures thereof. When a chelating agent is used, the subject consumer product may comprise from about 0.005% to about 15% or even from about 3.0% to about 10% chelating agent by weight of the subject consumer product. Suitable chelants include DTPA (Diethylene triamine pentaacetic acid), HEDP (Hydroxyethane diphosphonic acid), DTPMP (Diethylene triamine penta(methylene phosphonic acid)), 1,2-Dihydroxybenzene-3,5-disulfonic acid disodium salt hydrate, ethylenediamine, diethylene triamine, ethylenediaminedisuccinic acid (EDDS), N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP) and derivatives thereof.

Dye Transfer Inhibiting Agents - The consumer products of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in a subject consumer product, the dye transfer inhibiting agents may be present at levels from about 0.0001% to about 10%, from about 0.01% to about 5% or even from about 0.1% to about 3% by weight of the consumer product.

Brighteners - The consumer products of the present invention can also contain additional components that may tint articles being cleaned, such as fluorescent brighteners.

The composition may comprise C.I. fluorescent brightener 260, preferably in alpha-crystalline form having the following structure:

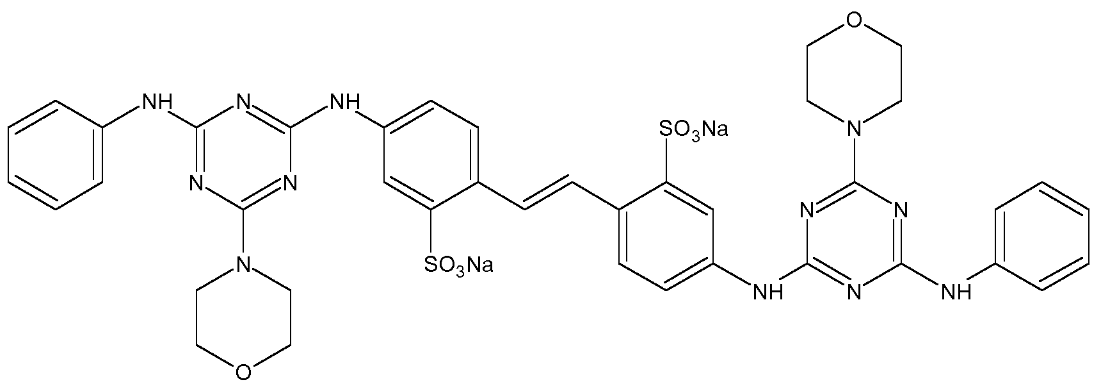

In one aspect, the brightener is a soluble brightener, such as the C.I. fluorescent brightener 260 in alpha-crystalline form.

In one aspect the brightener is predominantly in alpha-crystalline form, which means that typically at least 50wt%, at least 75wt%, at least 90wt%, at least 99wt%, or even substantially all, of the C.I. fluorescent brightener 260 is in alpha-crystalline form.

The brightener is preferably in micronized particulate form, having a weight average primary particle size of from 3 to 30 micrometers, from 3 micrometers to 20 micrometers, or from 3 to 10 micrometers.

The composition may comprise C.I. fluorescent brightener 260 in beta-crystalline form, and the weight ratio of: (i) C.I. fluorescent brightener 260 in alpha-crystalline form, to (ii) C.I. fluorescent brightener 260 in beta-crystalline form may be at least 0.1, or at least 0.6.

BE680847 relates to a process for making C.I fluorescent brightener 260 in alpha-crystalline form.

Suitable fluorescent brightener levels include lower levels of from about 0.01, from about 0.05, from about 0.1 or even from about 0.2 wt % to upper levels of 0.5 or even 0.75 wt %.

Carboxylate polymer - The consumer products of the present invention may also include one or more carboxylate polymers such as a maleate/acrylate random copolymer or polyacrylate homopolymer. In one aspect, the carboxylate polymer is a polyacrylate homopolymer having a molecular weight of from 4,000 Da to 9,000 Da, or from 6,000 Da to 9,000 Da.

Soil release polymer - The consumer products of the present invention may also include one or more soil release polymers having a structure as defined by one of the following structures (I), (II) or (III):

(I) $-[(OCHR^1\text{-}CHR^2)_a\text{-}O\text{-}OC\text{-}Ar\text{-}CO\text{-}]_d$

(II) $-[(OCHR^3\text{-}CHR^4)_b\text{-}O\text{-}OC\text{-}sAr\text{-}CO\text{-}]_e$

(III) $-[(OCHR^5-CHR^6)_c-OR^7]_f$

wherein:

a, b and c are from 1 to 200;
d, e and f are from 1 to 50;
Ar is a 1,4-substituted phenylene;
sAr is 1,3-substituted phenylene substituted in position 5 with $SO_3Me$;
Me is Li, K, Mg/2, Ca/2, Al/3, ammonium, mono-, di-, tri-, or tetraalkylammonium wherein the alkyl groups are $C_1$-$C_{18}$ alkyl or $C_2$-$C_{10}$ hydroxyalkyl, or mixtures thereof;
$R^1$, $R^2$, $R^3$, $R^4$, $R^5$ and $R^6$ are independently selected from H or $C_1$-$C_{18}$ n- or iso-alkyl; and
$R^7$ is a linear or branched $C_1$-$C_{18}$ alkyl, or a linear or branched $C_2$-$C_{30}$ alkenyl, or a cycloalkyl group with 5 to 9 carbon atoms, or a $C_8$-$C_{30}$ aryl group, or a $C_6$-$C_{30}$ arylalkyl group.

Suitable soil release polymers are polyester soil release polymers such as Repel-o-tex polymers, including Repel-o-tex SF, SF-2 and SRP6 supplied by Rhodia. Other suitable soil release polymers include Texcare polymers, including Texcare SRA100, SRA300, SRN100, SRN170, SRN240, SRN300 and SRN325 supplied by Clariant. Other suitable soil release polymers are Marloquest polymers, such as Marloquest SL supplied by Sasol.

Cellulosic polymer - The consumer products of the present invention may also include one or more cellulosic polymers including those selected from alkyl cellulose, alkyl alkoxyalkyl cellulose, carboxyalkyl cellulose, alkyl carboxyalkyl cellulose. In one aspect, the cellulosic polymers are selected from the group comprising carboxymethyl cellulose, methyl cellulose, methyl hydroxyethyl cellulose, methyl carboxymethyl cellulose, and mixures thereof. In one aspect, the carboxymethyl cellulose has a degree of carboxymethyl substitution from 0.5 to 0.9 and a molecular weight from 100,000 Da to 300,000 Da.

Bleach Catalysts - The consumer products of the present invention may also include one or more bleach catalysts, such as transitiona metal catalysts or ligands capable of forming a transition metal catalyst for example as described in EP1109965, or EP1240378 or 9,or bleach catalysts capable of accepting an oxygen atom from a peroxyacid and/or salt thereof, and transferring the oxygen atom to an oxidizeable substrate. Suitable bleach catalysts include, but are not limited to: iminium cations and polyions; iminium zwitterions; modified amines; modified amine oxides; N-sulphonyl imines; N-phosphonyl imines; N-acyl imines; thiadiazole dioxides; perfluoroimines; cyclic sugar ketones and mixtures thereof, as described in USPA 2007/0173430 A1.

In another aspect, the laundry detergent composition comprises a bleach ingredient, the bleach ingredient have a $logP_{o/w}$ no greater than 0, no greater than -0.5, no greater than -1.0, no greater than -1.5, no greater than -2.0, no greater than -2.5, no greater than -3.0, or even no greater than -3.5. The method for determining $logP_{o/w}$ is described in more detail below.

Typically, the bleach ingredient is capable of generating a bleaching species having a $X_{SO}$ of from 0.01 to about 0.30, from 0.05 to about 0.25, or even from about 0.10 to 0.20. The method for determining $X_{SO}$ is described in more detail below. For example, bleaching ingredients having an isoquinolinium structure are capable of generating a bleaching species that has an oxaziridinium structure. In this example, the $X_{SO}$ is that of the oxaziridinium bleaching species.

Without wishing to be bound by theory, the inventors believe that controlling the electophilicity and hydrophobicity in this above described manner enables the bleach ingredient to be delivered substantially only to areas of the fabric that are more hydrophobic, and that contain electron rich soils, including visible chromophores, that are susceptible to bleaching by highly electrophilic oxidants.

In one aspect, the bleach catalyst has a structure corresponding to general formula below:

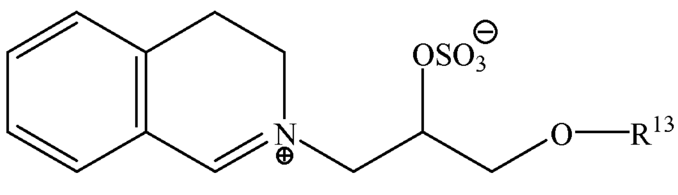

wherein $R^{13}$ is selected from the group consisting of 2-ethylhexyl, 2-propylheptyl, 2-butyloctyl, 2-pentylnonyl, 2-hexyldecyl, n-dodecyl, n-tetradecyl, n-hexadecyl, n-octadecyl, isononyl, iso-decyl, iso-tridecyl and iso-pentadecyl;

Method of determining $logP_{o/w}$

Log $P_{o/w}$ is determined according to the method found in Brooke, D. N., Dobbs, A. J., Williams, N, Ecotoxicology and Environmental Safety (1986) 11(3): 251-260.

Method of determining Xso

The parameter Xso is determined according to the method described in Adam, W., Haas, W., Lohray, B. B. Journal of the American Chemical Society (1991) 113(16) 6202-6208.

Silicate salts - The consumer products of the present invention can also contain silicate salts, such as sodium or potassium silicate. The composition may comprise from 0wt% to less than 10wt% silicate salt, to 9wt%, or to 8wt%, or to 7wt%, or to 6wt%, or to 5wt%, or to 4wt%, or to 3wt%, or even to 2wt%, and preferably from above 0wt%, or from 0.5wt%, or even from 1wt% silicate salt. A suitable silicate salt is sodium silicate.

Dispersants - The consumer products of the present invention can also contain dispersants. Suitable water-soluble organic materials include the homo- or co-polymeric acids or their salts, in which the polycarboxylic acid comprises at least two carboxyl radicals separated from each other by not more than two carbon atoms.

Enzyme Stabilizers - Enzymes for use in detergents can be stabilized by various techniques. The enzymes employed herein can be stabilized by the presence of water-soluble sources of calcium and/or magnesium ions in the finished consumer products that provide such ions to the enzymes. In case of aqueous consumer products comprising protease, a reversible protease inhibitor, such as a boron compound, or compounds such as calcium formate, sodium formate and 1,2-propane diol can be added to further improve stability.

Catalytic Metal Complexes - Applicants' compositions may include catalytic metal complexes. One type of metal-containing bleach catalyst is a catalyst system comprising a transition metal cation of defined bleach catalytic activity, such as copper, iron, titanium, ruthenium, tungsten, molybdenum, or manganese cations, an auxiliary metal cation having little or no bleach catalytic activity, such as zinc or aluminum cations, and a sequestrate having defined stability constants for the catalytic and auxiliary metal cations, particularly ethylenediaminetetraacetic acid, ethylenediaminetetra(methylenephosphonic acid) and water-soluble salts thereof. Such catalysts are disclosed in U.S. 4,430,243. If desired, the compositions herein can be catalyzed by means of a manganese compound. Such compounds and levels of use are well known in the art and include, for example, the manganese-based catalysts disclosed in U.S. 5,576,282. Cobalt bleach catalysts useful herein are known, and are described, for example, in U.S. 5,597,936; U.S. 5,595,967. Such cobalt catalysts are readily prepared by known procedures, such as taught for example in U.S. 5,597,936, and U.S. 5,595,967.

Compositions herein may also suitably include a transition metal complex of ligands such as bispidones (US 7,501,389) and/or macropolycyclic rigid ligands - abbreviated as "MRLs". As a practical matter, and not by way of limitation, the compositions and processes herein can be adjusted to provide on the order of at least one part per hundred million of the active MRL species in the aqueous washing medium, and will typically provide from about 0.005 ppm to about 25 ppm, from about 0.05 ppm to about 10 ppm, or even from about 0.1 ppm to about 5 ppm, of the MRL in the wash liquor. Suitable transition-metals in the instant transition-metal bleach catalyst include, for example, manganese, iron and chromium. Suitable MRLs include 5,12-diethyl-1,5,8,12-tetraazabicyclo[6.6.2]hexadecane.

Suitable transition metal MRLs are readily prepared by known procedures, such as taught for example in U.S. 6,225,464.

Solvents - Suitable solvents include water and other solvents such as lipophilic fluids. Examples of suitable lipophilic fluids include siloxanes, other silicones, hydrocarbons, glycol ethers, glycerine derivatives such as glycerine ethers, perfluorinated amines, perfluorinated and hydrofluoroether solvents, low-volatility nonfluorinated organic solvents, diol solvents, other environmentally-friendly solvents and mixtures thereof.

Processes of Making Consumer Products

The consumer products of the present invention can be formulated into any suitable form and prepared by any process chosen by the formulator, non-limiting examples of which are described in Applicants' examples and in U.S. 4,990,280; U.S. 20030087791A1; U.S. 20030087790A1; U.S. 20050003983A1; U.S. 20040048764A1; U.S. 4,762,636; U.S. 6,291,412; U.S. 20050227891A1; EP 1070115A2; U.S. 5,879,584; U.S. 5,691,297; U.S. 5,574,005; U.S. 5,569,645; U.S. 5,565,422; U.S. 5,516,448; U.S. 5,489,392; U.S. 5,486,303.

Method of Use

The present disclosure includes a method for cleaning and /or treating a situs *inter alia* a fabric surface. Such method includes the steps of contacting an embodiment of Applicants' cleaning consumer product, in neat form or diluted in a wash liquor, with at least a portion of a situs then optionally rinsing such fabric surface. The situs may be subjected

to a washing step prior to the aforementioned rinsing step. For purposes of the present disclosure, washing includes but is not limited to, scrubbing, and mechanical agitation. Accordingly, the present disclosure includes a method for laundering a fabric. The method comprises the steps of contacting a fabric to be laundered with a said cleaning laundry solution comprising at least one embodiment of Applicants' consumer product. The fabric may comprise most any fabric capable of being laundered in normal consumer use conditions. The solution typically has a pH of from about 7 to about 11. The consumer products may be employed at concentrations of from about 500 ppm to about 15,000 ppm in solution. The water temperatures typically range from about 5 °C to about 90 °C. The water to fabric ratio is typically from about 1:1 to about 30:1.

In one aspect, a situs treated with any of the consumer products disclosed herein is disclosed.

TEST METHODS

Test Method 1

A protocol to define whether a dye or pigment material is a fabric hueing agent for the purpose of the invention is given here:

1.) Fill two tergotometer pots with 800ml of Newcastle upon Tyne, UK, City Water (~12 grains per US gallon total hardness, supplied by Northumbrian Water, Pity Me, Durham, Co. Durham, UK).
2) Insert pots into tergotometer, with water temperature controlled at 30°C and agitation set at 40rpm for the duration of the experiment.
3) Add 4.8g of IEC-B detergent (IEC 60456 Washing Machine Reference Base Detergent Type B), supplied by wfk, Brüggen-Bracht, Germany, to each pot.
4) After two minutes, add 2.0mg active colorant to the first pot.
5) After one minute, add 50g of flat cotton vest (supplied by Warwick Equest, Consett, County Durham, UK), cut into 5cm x 5cm swatches, to each pot.
6) After 10 minutes, drain the pots and re-fill with (16°C) having a water hardness of 14.4 English Clark Degrees Hardness with a 3:1 Calcium to Magnesium molar ratio.
7) After 2 minutes rinsing, remove fabrics.
8) Repeat steps 3-7 for a further three cycles using the same treatments.
9) Collect and line dry the fabrics indoors for 12 hours.
10) Analyse the swatches using a Hunter Miniscan spectrometer fitted with D65 illuminant and UVA cutting filter, to obtain Hunter a (red-green axis) and Hunter b (yellow-blue axis) values.
11) Average the Hunter a and Hunter b values for each set of fabrics. If the fabrics treated with colorant under assessment show an average difference in hue of greater than 0.2 units on either the a axis or b axis, it is deemed to be a fabric hueing agent for the purpose of the invention.

Test Methods 2 - 6

Any deviations from the protocols provided are indicated in the pertinent Examples.

The assays were performed using a Biomek FX Robot (Beckman Coulter) or a multichannel pipettor (e.g., Rainin PipetLite, Mettler-Toledo) and a SpectraMAX MTP Reader (type 340; Molecular Devices).

TCA Assay for Protein Content Determination in 96-well Microtiter Plates

B. subtilis cultures were grown 2-3 days at 37 °C, shaking at 250-300 rpm with humidified aeration. The cells were removed from the enzyme-containing culture supernatant, by centrifugation and/or filtration. The protease concentration was determined using a TCA precipitation assay. An aliquot (20-25 ul) of culture supernatant was transferred to a 96-well flat bottom microtiter plate (MTP; Costar 9017 medium binding clear polystyrene plate) containing 100 μL/well of 0.25 N HCl. The "baseline" read was determined by light scattering/absorbance reading at 405 nm following a 5 sec mix. 100 μL/well of 30% (w/v) trichloroacetic acid (TCA) was added to the HCl-containing plate and incubated for 10 minutes at room temperature to facilitate protein precipitation. The light scattering/absorbance at 405 nm of this "test" plate was determined after a 5 sec mix. The turbidity/light scattering increase in the samples correlates to the total amount of precipitable protein in the culture supernatant. The calculations were performed by subtracting the "baseline" reading (obtained after addition of HCl) from the "test" reading (obtained after addition of TCA) to provide a relative measure of total protein present. If desired, a standard curve can be created by calibrating the TCA readings with AAPF protease assays (see below) of clones with known specific activity. However, the TCA results are linear with respect to protein concentration from 50 to 500 parts per million (ppm) of protein (where 1 ppm corresponds to 1 mg/L) and can

thus be plotted directly against enzyme performance for the purpose of choosing variants with desired performance.

AAPF Protease Assay in 96-well Microtiter Plates

In order to determine the protease activity of the serine protease variants, the hydrolysis of N-succinyl-L-alanyl-L-alanyl-L-prolyl-L-phenyl-p-nitroanilide (suc-AAPF-pNA) was measured. The reagent solutions used were: 100 mM Tris/HCl, pH 8.6, containing 0.005% TWEEN®-80 (Tris dilution buffer); 100 mM Tris buffer, pH 8.6, containing 1 mM CaCl2 and 0.005% TWEEN®-80 (Tris/Ca buffer); and 160 mM suc-AAPF-pNA in DMSO (suc-AAPF-pNA stock solution) (Sigma: S-7388). To prepare a suc-AAPF-pNA working solution, 1 ml suc-AAPF-pNA stock solution was added to 100 ml Tris/Ca buffer and mixed well for at least 10 seconds. The assay was performed by adding 10 μl of diluted protease solution to each well of a 96-well MTP, immediately followed by the addition of 190 μl of 1 mg/ml suc-AAPF-pNA working solution. The solutions were mixed for 5 sec, and the absorbance change in kinetic mode (25 readings in 5 minutes) was read at 405 nm in an MTP reader, at 25°C. The protease activity was expressed as AU (activity = ΔOD•min-1 ml-1).

Eglin C Inhibition Assay

As described herein, serine protease concentration and specific activity was determined by titration with an inhibitor called eglin c. Eglin c from the leech Hirudo medicinalis is a tight-binding protein inhibitor of subtilisins and ASP protease (Heinz et al., Biochemistry, 31: 8755-66 [1992]), and can therefore be used to measure protease enzyme concentration, which in turn permits specific activity to be calculated. The gene for eglin c was synthesized and expressed in E. coli by standard methods. Its properties and inhibitory potency were the same as eglin c purchased from Sigma.

(i) Concentration Determination of an Eglin C Stock Solution

A sample of Bacillus lentus subtilisin of known specific activity was diluted in 100 mM Tris buffer, pH 8.6, containing 1 mM CaCl2 and 0.005% TWEEN®-80 (Tris/Ca buffer), to a concentration appropriate for AAPF protease assay described above. Several dilutions of the eglin c stock solution were also made in the Tris/Ca buffer. An aliquot of each diluted eglin c solution was mixed with an equal volume of the diluted Bacillus lentus subtilisin solution. An aliquot of the Tris/Ca buffer only, without eglin c, was also mixed with an equal volume of the diluted Bacillus lentus subtilisin solution, in order to measure uninhibited subtilisin activity in the absence of eglin c. The mixed solutions were incubated at room temperature for 15-30 minutes and the protease activity of each sample was then measured by AAPF assay described above. Using the known specific activity of Bacillus lentus subtilisin, the concentration of active protease in each sample was determined. The concentration of eglin c in each sample was then calculated based on the decrease of the observed protease activity as compared to the uninhibited subtilisin sample that was mixed with Tris/Ca buffer only (without eglin c). Thus, using the known dilutions and volumes of the eglin c solutions, the concentration of eglin c in the stock solution was determined.

(ii) Concentration and Specific Activity Determination of Subtilisin Variants

Samples of subtilisin variants were diluted in 100 mM Tris buffer, pH 8.6, containing 1 mM $CaCl_2$ and 0.005% TWEEN®-80 (Tris/Ca buffer). Several dilutions of the eglin c stock solution of known concentration were also made in the Tris/Ca buffer. An aliquot of each diluted eglin c solution was mixed with an equal volume of a subtilisin variant solution. The mixed solutions were incubated at room temperature for 15-30 minutes and the protease activity of each sample was then measured by AAPF assay. Using the observed decrease of the protease activity upon addition of each eglin c sample and the known concentration of the eglin c, the concentration of the eglin c necessary for the complete inhibition of each subtilisin enzyme variant was calculated. This concentration is equivalent to the enzyme concentration in the sample. An aliquot of the Tris/Ca buffer only, without eglin c, was also mixed with each subtilisin variant sample and the protease activity in the absence of eglin c was measured by AAPF assay. The specific activity of the subtilisin variants was then calculated using the enzyme concentrations as determined above.

BMI Microswatch Assay (BMI assay)

Pre-rinsed and punched blood, milk and ink (BMI) stained microswatches (EMPA116) of 5.5 millimeter circular diameter in 96 well microtiter plates (MTP; Corning 3641) were obtained from Center for Testmaterials BV (Vlaardingen, The Netherlands).
Detergents were prepared by mixing for at least 30 minutes in 2 mM sodium carbonate, buffered to pH 10.3 with the appropriate level of water hardness (3:1 Ca:Mg. - $CaCl_2$ : $MgCl_2$•$6H_2O$) in Milli-Q water as described in Table 1-1 and Table 6-4. The detergents were aliquoted into 50 ml conical tubes (Falcon), centrifuged to remove precipitate, and chilled

on ice for 30 minutes prior to use.
Enzyme concentrations were equalized to a desired fixed concentration ranging from 20-50 ppm relative to a standard of purified GG36 (enzyme of SEQ ID NO:1). The specific activity of GG36 using AAPF as a substrate was used to convert baseline subtracted TCA values into enzyme concentration in ppm. Once enzyme concentration was determined in ppm, a simple formula was used to calculate the volume of each variant required to add to a fixed volume of buffer (300-600 µL) in order to achieve the desired stock enzyme concentration:

$$x=(\text{target ppm})(v_b)/(y-\text{target ppm})$$

Where x=volume enzyme, y= enzyme concentration, $v_b$= buffer volume
A Perkin-Elmer Janus robot with a Versispan 8 channel arm was used to dispense variable volumes of enzyme from the source plate (Axygen half deep well plate with pooled harvested variants used in the TCA enzyme concentration assay) into the buffer-filled destination plate using conductive tips. Samples were mixed three times by pipetting up and down. The accuracy of the enzyme dilutions was validated by measuring the AAPF activity of the equalized plate and comparing it to that of the source plate, to veryify that the correct dilutions had been made.

After equalization, 5-15 µL of enzyme solution was added to a detergent-filled microswatch plate to reach a final volume of ~200 µL. In some instances, the enzyme samples were not equalized, and were instead all diluted equally from the stock plate to give a working range of 0.1-5 ppm. Optimal target concentrations for each assay were determined from a dose response curve measuring cleaning activity over this range for a given detergent.

The MTP was sealed with foil (Bio-Rad) and incubated in iEMS incubator/shaker (Thermo/Labsystems) pre-set to 16°C in a cold room set to 4oC or at 32°C on the benchtop for 30 minutes at 1400 rpm. Following incubation, 120 µL of supernatant was transferred to a fresh MTP (Corning 9017) and read at 600 nm using the SpectraMax reader. True absorbance readings were obtained by subtracting a blank control (no enzyme) from each value.

A performance index (PI) was calculated for each variant. The performance index is the ratio of the absorbance of the supernatant produced by variant enzyme cleaning to the absorbance produced by GG36 cleaning at a fixed enzyme concentration. For the equalized plates, PI values were calculated by dividing the absorbance of a variant by that of the control on a given plate. For non-equalized plates, a standard curve (*e.g.* Langmuir or four parameter logistic nonlinear regression model fit) is generated from the activity and enzyme concentration of the controls. Using this standard curve, the performance of the variants can be directly compared to the control at any enzyme concentration. The PI is determined by dividing the absorbance of the variants by the calculated absorbance for the control at the same enzyme concentration.
A performance index (PI) that is greater than 1 (PI>1) indicates superior cleaning by a variant as compared to the standard (e.g., GG36), while a PI of 1 (PI=1) identifies a variant that performs the same as the standard, and a PI that is less than 1 (PI<1) identifies a variant that performs worse than the standard.

TABLE 1-1: Final detergent, water hardness, and buffer concentrations used for BMI microswatch assays

| Detergent | Final detergent Concentration (g/L) | Final Water Hardness* (gpg) | Final Sodium Carbonate Buffer Concentration (mM) |
|---|---|---|---|
| Example 26 | 0.808 | 6 | 2 |
| Example 27 | 1 | 3 | 2 |
| Example 28 | 2.3 | 12 | 2 |
| Example 29 | 5.9 | 12 | 2 |
| Example 30 | 8.3 | 12 | 2 |

* (3:1 Ca:Mg) Concentration as detailed in text.

TEST METHOD 2 - For Test Method 2, the BMI microswatch assay is run using the detergent of Example 29. The detergent is dissolved in water that has a hardness of 12gpg and adjusted to a temperature of 16°C. Performance of the variant enzymes is then determined as per the BMI microswatch assay described. The performance index is

determined by comparing the performance of the variant with that of the enzyme of SEQ ID NO:1, with in all cases the enzyme dosage range being 0.1-5ppm. Enzymes having a performance index of 1.1 or greater are viewed to be cold water proteases.

TEST METHOD 3 - For Test Method 3, the BMI microswatch assay is run using the detergent of Example 26. The detergent is dissolved in water that has a hardness of 6gpg and adjusted to a temperature of 16°C. Performance of the variant enzymes is then determined as per the BMI microswatch assay described. The performance index is determined by comparing the performance of the variant with that of the enzyme of SEQ ID NO:1, with in all cases the enzyme dosage range being 0.1-5ppm. Enzymes having a performance index of 1.1 or greater are viewed to be cold water proteases.

TEST METHOD 4 - For Test Method 4, the BMI microswatch assay is run using the detergent of Example 26. The detergent is dissolved in water that has a hardness of 6gpg and adjusted to a temperature of 16°C. Performance of the variant enzymes is then determined as per the BMI microswatch assay described. The performance index is determined by comparing the performance of the variant with that of a reference enzyme, said reference enzyme being the enzyme of SEQ ID NO:1 consisting the A158E mutation, with in all cases the enzyme dosage range being 0.1-5ppm. Enzymes having a performance index of 1.0 or greater are viewed to be cold water proteases.

TEST METHOD 5 - Electrical conductivity of an aqueous solution is assayed according to the standard method ASTM D1125 and reported in units of milliSiemens/cm, abbreviated to mS/cm in this patent.

TEST METHOD 6 - For Test Method 6, the BMI microswatch assay is run using one of the detergents of Examples 36a - 36n. The detergent is dissolved in water that has a hardness as specified in Table 6-4 and adjusted to a temperature of 16°C. Performance of the variant enzymes is then determined as per the BMI microswatch assay described. The performance index is determined by comparing the performance of the variant with that of the enzyme of SEQ ID NO:1, with in all cases the enzyme dosage range being 0.1-5ppm. Enzymes having a performance index of 1.1 or greater are viewed to be cold water proteases.

EXAMPLE

Examples 1-8

Liquid laundry detergent compositions suitable for front-loading automatic washing machines.

| **Ingredient** | **Composition (wt% of composition)** | | | | | | | |
|---|---|---|---|---|---|---|---|---|
| | **1** | **2** | **3** | **4** | **5** | **6** | **7** | **8** |
| Alkylbenzene sulfonic acid | 7 | 11 | 4.5 | 1.2 | 1.5 | 12.5 | 5.2 | 4 |
| Sodium $C_{12-14}$ alkyl ethoxy 3 sulfate | 2.3 | 3.5 | 4.5 | 4.5 | 7 | 18 | 1.8 | 2 |
| $C_{14-15}$ alkyl 8-ethoxylate | 5 | 8 | 2.5 | 2.6 | 4.5 | 4 | 3.7 | 2 |
| $C_{12}$ alkyl dimethyl amine oxide | - | - | 0.2 | - | - | - | - | - |
| $C_{12-14}$ alkyl hydroxyethyl dimethyl ammonium chloride | - | - | - | 0.5 | - | - | - | - |
| $C_{12-18}$ Fatty acid | 2.6 | 4 | 4 | 2.6 | 2.8 | 11 | 2.6 | 1.5 |
| Citric acid | 2.6 | 3 | 1.5 | 2 | 2.5 | 3.5 | 2.6 | 2 |
| Protease* | 0.05 | 0.03 | 0.04 | 0.03 | 0.04 | 0.03 | 0.03 | 0.02 |
| Amylase (Natalase®) | 0.1 | 0.2 | 0.15 | - | 0.05 | 0.5 | 0.1 | 0.2 |
| Mannanase (Mannaway®) | 0.05 | 0.1 | 0.05 | - | - | 0.1 | 0.04 | - |
| Random graft co-polymer[1] | 1 | 0.2 | 1 | 0.4 | 0.5 | 2.7 | 0.3 | 1 |

(continued)

| Ingredient | Composition (wt% of composition) | | | | | | | |
|---|---|---|---|---|---|---|---|---|
| | 1 | 2 | 3 | 4 | 5 | 6 | 7 | 8 |
| A compound having the following general structure: bis($(C_2H_5O)(C_2H_4O)n)(CH_3)$ -$N^+$-$C_xH_{2x}$-$N^+$-$(CH_3)$-bis($(C_2H_5O)(C_2H_4O)n)$, wherein n = from 20 to 30, and x = from 3 to 8, or sulphated or sulphonated variants thereof | 0.4 | 2 | 0.4 | 0.6 | 1.5 | 1.8 | 0.7 | 0.3 |
| Ethoxylated Polyethylenimine [2] | - | - | - | - | - | 0.5 | - | - |
| Amphiphilic alkoxylated grease cleaning polymer [3] | 0.1 | 0.2 | 0.1 | 0.2 | 0.3 | 0.3 | 0.2 | 0.3 |
| Diethoxylated poly (1,2 propylene terephthalate short block soil release polymer. | - | - | - | - | - | - | 0.3 | - |
| Diethylenetriaminepenta(met hylenephosphonic) acid | 0.2 | 0.3 | - | - | 0.2 | - | 0.2 | 0.3 |
| Hydroxyethane diphosphonic acid | - | - | 0.45 | - | - | 1.5 | - | 0.1 |
| FWA | 0.1 | 0.2 | 0.1 | - | - | 0.2 | 0.05 | 0.1 |
| Solvents (1,2 propanediol, ethanol), stabilizers | 3 | 4 | 1.5 | 1.5 | 2 | 4.3 | 2 | 1.5 |
| Hydrogenated castor oil derivative structurant | 0.4 | 0.4 | 0.3 | 0.1 | 0.3 | - | 0.4 | 0.5 |
| Boric acid | 1.5 | 2.5 | | 1.5 | 1.5 | 0.5 | 1.5 | 1.5 |
| Na formate | - | - | - | 1 | - | - | - | - |
| Reversible protease inhibitor[4] | - | - | 0.00 2 | - | - | - | - | - |
| Perfume | 0.5 | 0.7 | 0.5 | 0.5 | 0.8 | 1.5 | 0.5 | 0.8 |
| Perfume MicroCapsules slurry (30%am) | 0.2 | 0.3 | 0.7 | 0.2 | 0.05 | 0.4 | 0.9 | 0.7 |
| Ethoxylated thiophene Hueing Dye[5] | 0.005 | 0.007 | 0.01 0 | 0.008 | 0.008 | 0.00 7 | 0.007 | 0.008 |
| Buffers (sodium hydroxide, Monoethano lamine) | To pH 8.2 | | | | | | | |
| Water and minors (antifoam, aesthetics) | To 100% | | | | | | | |

Examples 9-16

Liquid laundry detergent compositions suitable for top-loading automatic washing machines.

| Ingredient | Composition (wt% of composition) | | | | | | | |
|---|---|---|---|---|---|---|---|---|
| | **9** | **10** | **11** | **12** | **13** | **14** | **15** | **16** |
| $C_{12-15}$ Alkylethoxy(1.8)sulfate | 20.1 | 15.1 | 20.0 | 15.1 | 13.7 | 16.7 | 10.0 | 9.9 |
| $C_{11.8}$ Alkylbenzene sulfonate | 2.7 | 2.0 | 1.0 | 2.0 | 5.5 | 5.6 | 3.0 | 3.9 |
| $C_{16-17}$ Branched alkyl sulfate | 6.5 | 4.9 | | 4.9 | 3.0 | 9.0 | 2.0 | |
| $C_{12-14}$ Alkyl -9-ethoxylate | 0.8 | 0.8 | 0.8 | 0.8 | 8.0 | 1.5 | 0.3 | 11.5 |
| $C_{12}$ dimethylamine oxide | | | 0.9 | | | | | |
| Citric acid | 3.8 | 3.8 | 3.8 | 3.8 | 3.5 | 3.5 | 2.0 | 2.1 |
| $C_{12-18}$ fatty acid | 2.0 | 1.5 | 2.0 | 1.5 | 4.5 | 2.3 | | 0.9 |
| Protease* | 0.1 | 0.2 | 0.1 | 0.1 | 0.1 | 0.1 | 0.1 | 0.1 |
| Amylase (Natalase®) | 0.7 | 0.3 | 0.6 | 0.3 | 0.6 | 0.4 | | |
| Amylase (Termamyl Ultra®) | | | | | | | | 1.1 |
| Mannanase (Mannaway®) | 0.1 | | | | | 0.1 | | |
| Pectate Lyase (Pectawash®) | 0.1 | | | | | 0.2 | | |
| Borax | 3.0 | 3.0 | | | 2.0 | 3.0 | 3.0 | 3.3 |
| Na & Ca formate | 0.2 | 0.2 | | 0.2 | 0.2 | | 0.7 | |
| A compound having the following general structure: bis(($C_2H_5O$)($C_2H_4O$)n)($CH_3$)-$N^+$-$C_xH_{2x}$-$N^+$-($CH_3$)-bis(($C_2H_5O$)($C_2H_4O$)n), wherein n = from 20 to 30, and x = from 3 to 8, or sulphated or sulphonated variants thereof | 1.6 | 1.6 | 3.0 | 1.6 | 2.0 | 1.6 | 1.3 | 1.2 |
| Random graft co-polymer[1] | 0.4 | 0.2 | 1.0 | 0.5 | 0.6 | 1.0 | 0.8 | 1.0 |
| Diethylene triamine pentaacetic acid | 0.4 | 0.4 | 0.4 | 0.4 | 0.2 | 0.3 | 0.8 | |
| Tinopal AMS-GX | 0.2 | 0.2 | 0.2 | 0.2 | 0.2 | 0.3 | 0.1 | |
| Tinopal CBS-X | | | | | | 0.1 | | 0.2 |
| Amphiphilic alkoxylated grease cleaning polymer [3] | 1.0 | 1.3 | 1.3 | 1.4 | 1.0 | 1.1 | 1.0 | 1.0 |
| Texcare 240N (Clariant) | | | | 1.0 | | | | |
| Ethanol | 2.6 | 2.6 | 2.6 | 2.6 | 1.8 | 3.0 | 1.3 | |
| Propylene Glycol | 4.6 | 4.6 | 4.6 | 4.6 | 3.0 | 4.0 | 2.5 | |

(continued)

| Ingredient | Composition (wt% of composition) | | | | | | | |
|---|---|---|---|---|---|---|---|---|
| | 9 | 10 | 11 | 12 | 13 | 14 | 15 | 16 |
| Diethylene glycol | 3.0 | 3.0 | 3.0 | 3.0 | 3.0 | 2.7 | 3.6 | |
| Polyethylene glycol | 0.2 | 0.2 | 0.2 | 0.2 | 0.1 | 0.3 | 0.1 | 1.4 |
| Monoethanolamine | 2.7 | 2.7 | 2.7 | 2.7 | 4.7 | 3.3 | 1.7 | 0.4 |
| Triethanolamine | | | | | | | | 0.9 |
| NaOH | to pH 8.3 | to pH 8.3 | to pH 8.3 | to pH 8.3 | to pH 8.3 | to pH 8.3 | to pH 8.3 | to pH 8.5 |
| Suds suppressor | | | | | | | | |
| Dye | 0.01 | 0.01 | 0.01 | | 0.01 | 0.01 | 0.01 | 0.0 |
| Perfume | 0.5 | 0.5 | 0.5 | 0.5 | 0.7 | 0.7 | 0.8 | 0.6 |
| Perfume MicroCapsules slurry (30%am) | 0.2 | 0.5 | 0.2 | 0.3 | 0.1 | 0.3 | 0.9 | 1.0 |
| Ethoxylated thiophene Hueing Dye[5] | 0.003 | 0.002 | 0.002 | 0.005 | 0.002 | 0.004 | 0.004 | 0.003 |
| Water | balance | balance | balance | balance | balance | balance | balance | balance |

Examples 17-22

The following are granular detergent compositions suitable for laundering fabrics.

| | **17** | **18** | **19** | **20** | **21** | **22** |
|---|---|---|---|---|---|---|
| Linear alkylbenzenesulfonate with aliphatic carbon chain length $C_{11}$-$C_{12}$ | 15 | 12 | 20 | 10 | 12 | 13 |
| Other surfactants | 1.6 | 1.2 | 1.9 | 3.2 | 0.5 | 1.2 |
| Phosphate builder(s) | 2 | 3 | 4 | | | |
| Zeolite | | 1 | | 1 | 4 | 1 |
| Silicate | 4 | 5 | 2 | 3 | 3 | 5 |
| Sodium Carbonate | 2 | 5 | 5 | 4 | 0 | 3 |
| Polyacrylate (MW 4500) | 1 | 0.6 | 1 | 1 | 1.5 | 1 |
| Carboxymethyl cellulose (Finnfix BDA ex CPKelco) | 1 | - | 0.3 | - | 1.1 | - |
| Celluclean® (15.6mg/g) | 0.23 | 0.17 | 0.5 | 0.2 | 0.2 | 0.6 |
| Protease* | 0.23 | 0.17 | 0.05 | 0.2 | 0.03 | 0.1 |
| Stainzyme Plus® (14mg/g) | 0.23 | 0.17 | 0.5 | 0.2 | 0.2 | 0.6 |
| Mannaway 4.0T (4mg/g) | 0.1 | | | 0.1 | | 0.1 |
| Lipex 100T (18.6mg/g) | 0.2 | | 0.1 | | 0.3 | |
| Fluorescent Brightener(s) | 0.16 | 0.06 | 0.16 | 0.18 | 0.16 | 0.16 |
| Diethylenetriamine pentaacetic acid or Ethylene diamine tetraacetic acid | 0.6 | | 0.6 | 0.25 | 0.6 | 0.6 |
| $MgSO_4$ | 1 | 1 | 1 | 0.5 | 1 | 1 |
| Bleach(es) and Bleach activator(s) | 6.88 | | 6.12 | 2.09 | 1.17 | 4.66 |
| Ethoxylated thiophene Hueing Dye[5] | 0.002 | 0.001 | 0.003 | 0.003 | - | - |
| Direct Violet 9 ex Ciba Specialty Chemicals | | | | 0.0006 | 0.0004 | 0.0006 |

(continued)

| | 17 | 18 | 19 | 20 | 21 | 22 |
|---|---|---|---|---|---|---|
| Sulfate/Citric Acid/Sodium Bicarbonate/Moisture/ perfume | Balance to 100% | | | | | |

[1]Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units.

[2]Polyethylenimine (MW = 600) with 20 ethoxylate groups per -NH.

[3]Amphiphilic alkoxylated grease cleaning polymer is a polyethylenimine (MW = 600) with 24 ethoxylate groups per -NH and 16 propoxylate groups per -NH

[4]Reversible Protease inhibitor of structure:

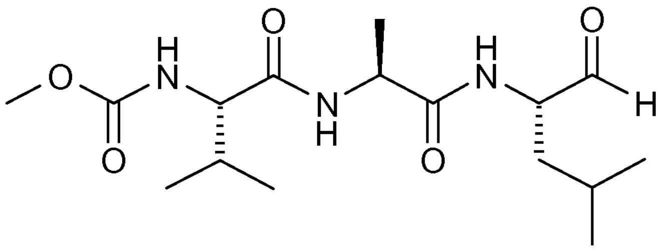

[5]Ethoxylated thiophene Hueing Dye is as described in US 7,208,459 B2.

*Remark: all enzyme levels expressed as % enzyme raw material, except for protease (of this invention) which is expressed as % of active protein added to the product..

Examples 23- 25

The following are liquid laundry detergent compositions suitable for top-loading automatic washing machines (23 and 24) and front loading washing machines (25).

| Ingredient | Composition (wt% of composition) | | |
|---|---|---|---|
| | 23 | 24 | 25 |
| $C_{12-15}$ Alkylethoxy(1.8)sulfate | 14.7 | 11.6 | |
| $C_{11.8}$ Alkylbenzene sulfonate | 4.3 | 11.6 | 8.3 |
| $C_{16-17}$ Branched alkyl sulfate | 1.7 | 1.29 | |
| $C_{12-14}$ Alkyl -9-ethoxylate | 0.9 | 1.07 | |
| $C_{12}$ dimethylamine oxide | 0.6 | 0.64 | |
| Citric acid | 3.5 | 0.65 | 3 |
| $C_{12-18}$ fatty acid | 1.5 | 2.32 | 3.6 |
| Sodium Borate (Borax) | 2.5 | 2.46 | 1.2 |
| Sodium $C_{12-14}$ alkyl ethoxy 3 sulfate | | | 2.9 |
| $C_{14-15}$ alkyl 7-ethoxylate | | | 4.2 |
| $C_{12-14}$ Alkyl -7-ethoxylate | | | 1.7 |
| Ca formate | 0.09 | 0.09 | |

(continued)

| Ingredient | Composition (wt% of composition) | | |
|---|---|---|---|
| | 23 | 24 | 25 |
| A compound having the following general structure: bis(($C_2H_5O$)($C_2H_4O$)n)($CH_3$)-$N^+$-$C_xH_{2x}$-$N^+$-($CH_3$)-bis(($C_2H_5O$)($C_2H_4O$)n), wherein n = from 20 to 30, and x = from 3 to 8, or sulphated or sulphonated variants thereof | | | 1.2 |
| Random graft co-polymer[1] | | 1.46 | 0.5 |
| Ethoxylated Polyethylenimine [2] | 1.5 | 1.29 | |
| Diethylene triamine pentaacetic acid | 0.34 | 0.64 | |
| Diethylene triamine penta(methylene phosphonic acid) | | | 0.3 |
| Tinopal AMS-GX | | 0.06 | |
| Tinopal CBS-X | 0.2 | 0.17 | |
| Amphiphilic alkoxylated grease cleaning polymer[3] | 1.28 | 1 | 0.4 |
| Ethanol | 2 | 1.58 | 1.6 |
| Propylene Glycol | 3.9 | 3.59 | 1.3 |
| Diethylene glycol | 1.05 | 1.54 | |
| Polyethylene glycol | 0.06 | 0.04 | |
| Monoethano lamine | 3.05 | 2.41 | 0.4 |
| NaOH | 2.44 | 1.8 | |
| Sodium Cumene Sulphonate | | | 1 |
| Sodium Formate | | 0.11 | |
| Water, Aesthetics (Dyes, perfumes) and Minors (Enzymes, solvents, structurants) | balance | balance | balance |

[1]Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units.
[2]Polyethylenimine (MW = 600) with 20 ethoxylate groups per -NH.
[3]Amphiphilic alkoxylated grease cleaning polymer is a polyethylenimine (MW = 600) with 24 ethoxylate groups per -NH and 16 propoxylate groups per -NH

Examples 26- 30

The following are granular laundry detergent compositions suitable for top-loading automatic washing machines (26-28) and front loading washing machines (29-30).
The protease of this invention is separately added to these formulations.

| | 26 | 27 | 28 | 29 | 30 |
|---|---|---|---|---|---|
| **Surfactants** | | | | | |
| $C_{16-17}$ Branched alkyl sulfate | 3.55 | | | | |
| $C_{12-14}$ alkyl sulphate | | | 1.5 | | |
| Sodium linear alkylbenzenesulfonate with aliphatic chain length $C_{11}$-$C_{12}$ | 9.6 | 15.8 | 10.6 | 7.5 | 9 |
| Sodium $C_{14/15}$ alcohol ethoxy - 3 - sulfate | 1.15 | | | 2.88 | |

(continued)

| | 26 | 27 | 28 | 29 | 30 |
|---|---|---|---|---|---|
| **Surfactants** | | | | | |
| Sodium $C_{14/15}$ alkyl sulphate | 2.37 | | | | |
| $C_{14/15}$ alcohol ethoxylate with average 7 moles of ethoxylation | | | | 1.17 | 1 |
| mono-$C_{8-10}$ alkyl mono-hydroxyethyl di-methyl quaternary ammonium chloride | | | | | 0.45 |
| Di methyl hydroxyl ethyl lauryl ammonium chloride | | | 0.18 | | |
| Zeolite A | 13.9 | 4.7 | 0.01 | 2.9 | 1.8 |
| Sodium Silicate 1.6.ratio | 4 | 0.2 | | 4 | 4 |
| Sodium Silicate 2.35.ratio | | | 8 | | |
| Citric Acid | | | | 2.5 | 1.4 |
| Sodium tripolyphosphate | | | 5 | | |
| Sodium Carbonate | 24.1 | 30 | 16.9 | 24.4 | 21 |
| Nonanoyloxybenzenesuplhonate | 5.78 | 2.81 | 0.96 | | |
| Oxaziridinium-based bleach booster | | | | 0.03 | 0.017 |
| Tetrasodium S,S,-ethylenediaminedisuccinate | | | | 0.2 | |
| Diethylenetriamine penta (methylene phosphonic acid), heptasodium salt | 0.61 | | | | 0.33 |
| Hydroxyethane dimethylene phosphonic acid | | | | 0.29 | 0.45 |
| Ethylene diamine tetraacetate | | | 0.27 | | |
| MgSO4 | | | 0.47 | 0.5994 | 0.782 |
| Sodium Percarbonate | 7 | 4.4 | | 15.9 | 19.1 |
| Tetra Acetyl Ethylene Diamine | | | | 3.3 | 4.6 |
| Sodium Perborate Monohydrate | | | 1.2 | | |
| Carboxymethyl cellulose (e.g. Finnfix BDA ex CPKelco) | 0.1 | | 0.17 | 1.69 | 0.23 |
| Sodium Acrylic acid/maleic acid co-polymer (70/30) | 0.0236 | 3.8 | | 2 | 2.5 |
| Sodium polyacrylate (Sokalan PA30 CL) | 4 | | 0.84 | | |
| Terephthalate polymer | | | | 0.23 | |
| Polyethylene glycol/vinyl acetate random graft co polymer | | | 0.89 | 0.89 | 0.91 |
| Photobleach- zinc phthalocyanine tetrasulfonate | | | 0.005 | 0.001 | 0.002 |
| C.I.Fluorescent Brightener 260 | 0.11 | 0.15 | 0.04 | 0.23 | 0.15 |
| C.I.Fluorescent Brightener 351 (Tinopal ® CBS) | | | 0.1 | | |
| Suds suppressor granule | | 0.25 | | 0.07 | 0.04 |
| Hyrdophobically modified carboxy methyl cellulose (Finnifix ® SH-1) | | | 0.019 | 0.028 | |
| Bentonite | | | 8.35 | | |
| Miscellaneous (Dyes, perfumes, process aids, moisture and sodium sulphate) | Balance | Balance | Balance | Balance | Balance |

Notes for examples 26 - 30:

Surfactant ingredients can be obtained from BASF, Ludwigshafen, Germany (Lutensol®); Shell Chemicals, London, UK; Stepan, Northfield, Illinois, USA; Huntsman, Huntsman, Salt Lake City, Utah, USA; Clariant, Sulzbach, Germany (Praepagen®).

Zeolite can be obtained from Industrial Zeolite (UK) Ltd, Grays, Essex, UK.

Citric acid and sodium citrate can be obtained from Jungbunzlauer, Basel, Switzerland. Sodium percarbonate, sodium carbonate, sodium bicarbonate and sodium sesquicarbonate can be obtained from Solvay, Brussels, Belgium.

Acrylate/maleate copolymers can be obtained from BASF, Ludwigshafen, Germany. Carboxymethylcellulose and hydrophobically modified carboxymethyl cellulose can be obtained from CPKelco, Arnhem, The Netherlands.

C.I. Fluorescent Brightener 260 can be obtained from 3V Sigma, Bergamo, Italy as Optiblanc® Optiblanc® 2M/G, Optiblanc® 2MG/LT Extra, or Optiblanc® Ecobright.

Tetrasodium S,S-ethylenediamine disuccinate can be obtained from Innospec, Ellesmere Port, UK.

Terephthalate co-polymer can be obtained from Clariant under the tradename Repelotex SF 2. 1-Hydroxyethane -1,1-diphosphonic acid can be obtained from Thermphos, Vlissingen-Oost, The Netherlands.

Oxaziridinium-based bleach booster has the following structure, where R1 = 2-butyloctyl, and was produced according to US 2006/0089284A1.

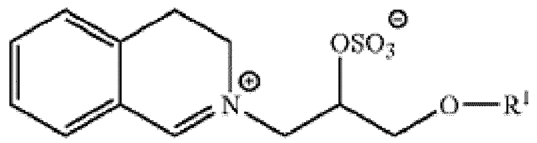

Enzymes Natalase ®, Termamyl ®, Stainzyme Plus®, Celluclean® and Mannaway ®, can be obtained from Novozymes, Bagsvaerd, Denmark.

Zinc phthalocyanine tetrasulfonate can be obtained from Ciba Specialty Chemicals, Basel, Switzerland, as Tinolux® BMC.

Suds suppressor granule can be obtained from Dow Corning, Barry, UK.

Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units.

Example 31

Generation of GG36 Site Evaluation Libraries (SELs)

The construction of GG36 SELs described in this example was performed by GENEART using their proprietary methods and technology platform for gene optimization, gene synthesis, library generation and analysis (WO 2004/059556A3, European Patent Nos. 0 200 362 and 0 201 184; and US Patent Nos. 4,683,195, 4,683,202 and 6,472,184). The GG36 SELs were produced at positions pre-selected by the inventors using the pHPLT-GG36 *B. subtilis* expression plasmid (*See*, FIG. 2). This *B. subtilis* expression plasmid contains the GG36 expression cassette shown below, the *B. licheniformis* LAT promoter (Plat), and additional elements from pUB110 (McKenzie et al., Plasmid, 15:93-103, 1986) including a replicase gene (reppUB), a neomycin/kanamycin resistance gene (neo) and a bleomycin resistance marker (bleo) (Figure 4 in US Patent No. 6,566,112).

DNA sequence of GG36 (signal sequence is shown in lower case letters, propeptide in lower case, underlined text, and GG36 mature sequence in uppercase letters)

gtgagaagcaaaaaattgtggatcgtcgcgtcgaccgcactactcatttctgttgctttcagttcatcgatcgcatcggctgctgaagaagcaa
aagaaaaatatttaattggctttaatgagcaggaagctgtcagtgagtttgtagaacaagtagaggcaaatgacgaggtcgccattctctctga
ggaagaggaagtcgaaattgaattgcttcatgaatttgaaacgattcctgttttatccgttgagttaagcccagaagatgtggacgcgcttgag
ctcgatccagcgatttcttatattgaagaggatgcagaagtaacgacaatgGCGCAATCAGTGCCATGGGGAATTAG
CCGTGTGCAAGCCCCAGCTGCCCATAACCGTGGATTGACAGGTTCTGGTGTAAAAGT
TGCTGTCCTCGATACAGGTATTTCCACTCATCCAGACTTAAATATTCGTGGTGGCGCT
AGCTTTGTACCAGGGGAACCATCCACTCAAGATGGGAATGGGCATGGCACGCATGT
GGCCGGGACGATTGCTGCTTTAAACAATTCGATTGGCGTTCTTGGCGTAGCGCCGAG
CGCGGAACTATACGCTGTTAAAGTATTAGGGGCGAGCGGTTCAGGTTCGGTCAGCTC
GATTGCCCAAGGATTGGAATGGGCAGGGAACAATGGCATGCACGTTGCTAATTTGA
GTTTAGGAAGCCCTTCGCCAAGTGCCACACTTGAGCAAGCTGTTAATAGCGCGACTT

CTAGAGGCGTTCTTGTTGTAGCGGCATCTGGAAATTCAGGTGCAGGCTCAATCAGCT
ATCCGGCCCGTTATGCGAACGCAATGGCAGTCGGAGCTACTGACCAAAACAACAAC
CGCGCCAGCTTTTCACAGTATGGCGCAGGGCTTGACATTGTCGCACCAGGTGTAAAC
GTGCAGAGCACATACCCAGGTTCAACGTATGCCAGCTTAAACGGTACATCGATGGCT
ACTCCTCATGTTGCAGGTGCAGCAGCCCTTGTTAAACAAAAGAACCCATCTTGGTCC
AATGTACAAATCCGCAATCATCTAAAGAATACGGCAACGAGCTTAGGAAGCACGAA
CTTGTATGGAAGCGGACTTGTCAATGCAGAAGCTGCAACTCGTTAA SEQ ID NO:4 is the full sequence, including propeptide and signal peptide.

Protein sequence of GG36 (signal sequence is shown in lower case letters, propeptide in lower case, underlined text, and GG36 mature protease sequence in uppercase letters)

vrskklwivastallisvafsssiasaaeeakekyligfneqeavsefveqveandevailseeeeveiellhefetipvlsvelspedvdal
eldpaisyieedaevttmAQSVPWGISRVQAPAAHNRGLTGSGVKVAVLDTGISTHPDLNIRGGA
SFVPGEPSTQDGNGHGTHVAGTIAALNNSIGVLGVAPSAELYAVKVLGASGSGSVSSIAQ
GLEWAGNNGMHVANLSLGSPSPSATLEQAVNSATSRGVLVVAASGNSGAGSISYPARY
ANAMAVGATDQNNNRASFSQYGAGLDIVAPGVNVQSTYPGSTYASLNGTSMATPHVA GAAALVKQKNPSWSNVQIRNHLKNTATSLGSTNLYGSGLVNAEAATR SEQ ID NO:1 is the mature sequence and SEQ ID NO:3 also includes the signal and propeptide.

The method of mutagenesis was based on the codon-specific mutation approach in which all possible amino acid substitutions are simultaneously created at a specific codon of interest using forward and reverse mutagenesis primers that contain a degenerate codon, NNS ((A,C,T or G), (A,C,T or G), (C or G)) at the site of interest. To construct each of the GG36 SELs, three PCR reactions were performed: two mutagenesis reactions (primary PCR1 and PCR2) to introduce the mutated codon of interest in the mature GG36 DNA sequence using the NNS forward and reverse mutagenesis primers (25-45 nucleotides long), and a third reaction to fuse the two mutagenesis PCR products together to construct the pHPLT-GG36 expression vector having the desired mutated codons in the mature GG36 sequence.

The primer sequences used in this Example are provided below:

| | |
|---|---|
| CGCGCTT**GAGCTC**GATCCAGCGATTTC | SacI-Fw |
| GTCTCC**AAGCTT**TAACGAGTTGCAG | HindIII-Rv |
| GCAATTC**AGATCT**TCCTTCAGGTTATGACC | pHPLT-BglII-Fw |
| GCATCGA**AGATCT**GATTGCTTAACTGCTTC | pHPLT-BglII-Rv |

The Phusion High-Fidelity DNA Polymerase (Finnzymes catalog no. F-530L) was used for all PCRs, and the reactions were executed according to manufacturer's protocols that were supplied with the polymerase. In particular, for primary PCR 1, 1 $\mu$L (10 $\mu$M) of each of the pHPLT-BglII-Fw primer and a NNS reverse mutagenesis primer were used, and for primary PCR 2, 1 $\mu$L (10 $\mu$M) of the pHPLT-BglII-Rv primer and a NNS forward mutagenesis primer were used. Each reaction also included 1 $\mu$L of the pHPLT-GG36 plasmid template DNA (0.1 - 1 ng/ $\mu$L). An MJ Research PTC-200 Peltier thermal cycler was used for the PCRs. The reactions yielded two fragments of approximately 2 to 3 kb having approximately 30 nucleotide overlap surrounding the GG36 codon of interest. The fragments obtained were fused in a third PCR similar to the ones described above using 1 $\mu$L of primary PCR 1 reaction mix, 1 $\mu$L of primary PCR 2 reaction mix and 1 $\mu$L (10 $\mu$M) of each of the forward and reverse SacI-Fw and HindIII-Rv primers. The amplified linear 859 bp fragment encoding the GG36 variant gene was purified (using QIAGEN® Qiaquick PCR purification kit) and digested with the SacI and HindIII restriction enzymes to create cohesive ends on both sides of the fusion fragment. About 50ng of plasmid pHPLT-GG36 was also purified after digestion with SacI and HindIII, resulting in a 3.9kb vector backbone fragment. The digested vector fragment was ligated with 50ng of the digested 859bp fragment encoding the variant enzyme using the T4 DNA ligase (Invitrogen) following the manufacturer's protocol for cloning of cohesive ends. Subsequently, the ligation mixture was used to transform *B. subtilis* cells ($\Delta$*aprE*, $\Delta$*nprE*, *oppA*, $\Delta$*spoIIE*, *degUHy32*, $\Delta$*amyE*::[*xylR,pxylA-comK]*) as described (WO 2002/014490).

To express the variant proteins for further biochemical analyses, the *B. subtilis* strains carrying the GG36 variant plasmids were inoculated into microtiter plates containing 150$\mu$l Luria broth medium supplemented with 10$\mu$g/ml neomycin. Plates were grown overnight at 37°C with 300 rpm shaking and 80% humidity using Enzyscreen lids for microtiter plates (Enzyscreen). Ten microliters from the overnight culture plate were used to inoculate a new microtiter plate containing 190$\mu$l of MBD medium (a MOPS based defined medium) with 10ug/ml neomycin. MBD medium was prepared essentially as known in the art (*See*, Neidhardt et al., J. Bacteriol., 119: 736-747 [1974]), except that $NH_4Cl$, $FeSO_4$, and $CaCl_2$ were omitted from the base medium, 3 mM $K_2HPO_4$ was used, and the base medium was supplemented with 60 mM urea, and 100ml of a solution made of 210 g/L glucose, and 350 g/L maltodextrin. The micronutrients were

made up as a 100X stock solution containing in one liter, 400 mg $FeSO_4$ $7H_2O$, 100 mg $MnSO_4$ $.H_2O$, 100 mg $ZnSO_4$ $7H_2O$, 50 mg $CuCl_2$ $2H_2O$, 100 mg $CoCl_2$ $6H_2O$, 100 mg $NaMoO_4$ $2H_2O$, 100 mg $Na_2B_4O_7$ $10H_2O$, 10 ml of 1M $CaCl_2$, and 10 ml of 0.5 M sodium citrate. The MBD medium containing microtiter plates were grown for 68 hours at 37°C, 300 rpm, and 80% humidity using Enzyscreen lids (Enzyscreen) for determining protein expression. The next day, cultures were filtered through a micro-filter plate (0.22μm; Millipore) and the resulting filtrate was used for biochemical analysis. The TCA, LAS/EDTA and BMI microswatch assays were carried out as described in the Test Methods Section. Performance indices were also calculated as described under the BMI assay description in the Test Methods Section, and they are shown in Table 2-1.

TABLE 2-1: Performance index (PI) values of GG36 variants (for reference only) relative to GG36 in the BMI microswatch assay using Test Method 3

| **Mutation versus SEQ ID NO:1 (BPN' numbering)** | **BMI, Detergent of Example 26 16C, PI** |
|---|---|
| Q2S | 1.1 |
| V4R | 1.1 |
| V4S | 1.1 |
| R10S | 1.1 |
| P14K | 1.1 |
| A16S | 1.1 |
| T22A | 1.1 |
| T22R | 1.1 |
| S24R | 1.2 |
| G25V | 1.1 |
| V26F | 1.1 |
| L42I | 1.2 |
| P52F | 1.2 |
| P52E | 1.1 |
| P52N | 1.1 |
| N62E | 1.1 |
| N62Q | 1.1 |
| V68A | 1.1 |
| V68C | 1.1 |
| T71G | 1.2 |
| I72C | 1.2 |
| A74C | 1.1 |
| L75A | 1.1 |
| L75F | 1.1 |
| S78R | 1.1 |
| E89P | 1.2 |
| E89T | 1.2 |
| E89G | 1.1 |
| E89H | 1.1 |
| E89W | 1.1 |
| Y91N | 1.3 |

(continued)

| Mutation versus SEQ ID NO:1 (BPN' numbering) | BMI, Detergent of Example 26 16C, PI |
|---|---|
| K94N | 1.1 |
| G100S | 1.1 |
| S101A | 1.2 |
| S101N | 1.1 |
| S101G | 1.1 |
| S101D | 1.1 |
| S103G | 1.3 |
| S103N | 1.2 |
| V104L | 1.1 |
| V104I | 1.1 |
| A108I | 1.1 |
| L111V | 1.5 |
| E112V | 1.2 |
| G115K | 1.1 |
| N117F | 1.1 |
| V121F | 1.1 |
| S128D | 1.2 |
| S128F | 1.1 |
| S128L | 1.1 |
| S128N | 1.1 |
| P129E | 1.1 |
| L148I | 1.1 |
| A158E | 1.1 |
| G159E | 1.1 |
| S160D | 1.2 |
| S166D | 1.2 |
| N185E | 1.1 |
| R186H | 1.1 |
| S188E | 1.2 |
| S188D | 1.1 |
| V203E | 1.2 |
| Y209S | 1.2 |
| Y209N | 1.2 |
| Y209F | 1.1 |
| Y209T | 1.1 |
| Y209E | 1.1 |
| Y209H | 1.1 |
| Y209G | 1.1 |

(continued)

| Mutation versus SEQ ID NO:1 (BPN' numbering) | BMI, Detergent of Example 26 16C, PI |
|---|---|
| P210R | 1.1 |
| S212I | 1.2 |
| Y214F | 1.1 |
| A215N | 1.1 |
| A215D | 1.1 |
| A215E | 1.1 |
| L217E | 1.1 |
| L217N | 1.1 |
| T224A | 1.1 |
| A230E | 1.1 |
| A231I | 1.1 |
| Q236F | 1.1 |
| N238R | 1.1 |
| N238K | 1.1 |
| P239K | 1.2 |
| P239G | 1.2 |
| P239R | 1.1 |
| N248V | 1.1 |
| H249R | 1.1 |
| L250I | 1.1 |
| L262D | 1.1 |
| Y263F | 1.1 |
| S265F | 1.1 |
| L267V | 1.2 |
| L267N | 1.1 |
| N269I | 1.1 |
| E271I | 1.1 |
| E271H | 1.1 |
| A272F | 1.2 |

TABLE 2-2: Performance index (PI) values of GG36 variants (for reference only) relative to GG36 in the BMI microswatch assay using the detergent of Example 29 The performance index of the below variants was assayed using Test Method 2 at 16C (for reference only)

| Mutations versus SEQ ID NO:1 (BPN' numbering) | PI |
|---|---|
| V4R | 1.1 |
| H17R | 1.1 |
| N18R | 1.1 |
| G20R | 1.1 |

(continued)

| Mutations versus SEQ ID NO:1 (BPN' numbering) | PI |
|---|---|
| T22R | 1.1 |
| S24R | 1.1 |
| S24W | 1.1 |
| G25R | 1.1 |
| N43R | 1.1 |
| N43A | 1.1 |
| G46R | 1.1 |
| P52F | 1.1 |
| P52N | 1.1 |
| T57R | 1.1 |
| Q59A | 1.1 |
| N62Q | 1.1 |
| T71G | 1.2 |
| L75R | 1.1 |
| N76D | 1.1 |
| S78R | 1.1 |
| L82R | 1.1 |
| P86W | 1.1 |
| E89P | 1.2 |
| E89W | 1.1 |
| E89T | 1.1 |
| E89I | 1.1 |
| E89H | 1.1 |
| E89V | 1.1 |
| V104L | 1.1 |
| S106V | 1.1 |
| S106G | 1.1 |
| G115R | 1.1 |
| G118I | 1.2 |
| V121F | 1.1 |
| S144R | 1.1 |
| N185I | 1.1 |
| D197F | 1.1 |
| Y209N | 1.1 |
| Y209S | 1.1 |
| L217E | 1.1 |
| A231I | 1.1 |
| P239R | 1.1 |

(continued)

| Mutations versus SEQ ID NO:1 (BPN' numbering) | PI |
|---|---|
| P239S | 1.1 |
| W241R | 1.1 |
| S242R | 1.1 |
| S242L | 1.1 |
| N243R | 1.2 |
| V244R | 1.1 |
| N248I | 1.1 |
| H249R | 1.1 |
| N252R | 1.1 |
| T253R | 1.1 |
| E271T | 1.2 |
| E271V | 1.1 |
| E271L | 1.1 |
| E271H | 1.1 |
| E271F | 1.1 |
| E271P | 1.1 |

EXAMPLE 32

Construction and cleaning performance of NHJ1 and WCE1 set of GG36 variants

The NHJ1 and WCE1 set of GG36 variants described herein were constructed at DNA 2.0, Inc. (Menlo Park, CA) with their proprietary methods using the pHPLT-GG36 *B. subtilis* expression plasmid described above (Figure 2). The variants were expressed in *B. subtilis* cells (genotype: ΔaprE, ΔnprE, amyE::xylRPxylAcomK-phleo) as described in Example 31, and were further characterized using the TCA and BMI microswatch assays as described in the Test Methods Section.

TABLE 3-1: Performance index (PI) values of NHJ1 variants relative to GG36 in Test Method 3

| Mutations versus SEQ ID NO:1 (BPN' numbering) | PI determined in detergent of Example 26 at 16C |
|---|---|
| T022A-N062E | 1.2 |
| T022A-P129E | 1.1 |
| T022A-L111V | 1.1 |
| T022A-S188D | 1.1 |
| A016S-T022A | 1.1 |
| T022A-E089P | 1.1 |
| T022A-G159E | 1.1 |
| T022A-A158E | 1.1 |

EXAMPLE 33

Construction and Cleaning Performance of NHJ4 Set of GG36 Variants

The NHJ4 set of GG36 variants described in Table 4-4 below were constructed using the pHPLT-GG36 *B.*

*subtilis* expression plasmid (Figure 2) using PCR fusion or the QuikChange® Multi Site-directed mutagenesis kit ("QCMS kit"; Stratagene) as described below.

a) Construction of NHJ4 variants by QuikChange® Multi Site-Directed Mutagenesis (QCMS)

Variants created by the QuikChange® Multi Site-Directed Mutagenesis are shown in Table 4-4. The parent plasmid pHPLT-GG36 (template DNA) was methylated using two micrograms of DNA and the Dam methylase (New England Biolabs), according to the manufacturer's instructions. Site-directed mutants were made by a QuikChange® Multi Site-Directed Mutagenesis Kit ("QCMS kit"; Stratagene) following the manufacturer's protocol *(See,* Table 4-1 for primer sequences). For efficient transformation of *B. subtilis*, DNA from the QCMS reaction was amplified by rolling circle amplification (RCA) using the Illustra Templiphi kit (GE Healthcare) according to the manufacturer's protocol. One microliter of 10-fold diluted amplified DNA was used to transform 50 μL of competent *B. subtilis* cells (genotype: ΔaprE, ΔnprE, amyE::xylRPxylAcomK-phleo). The transformation mixture was shaken at 37°C for 1 hour. Ten microliter aliquots of the transformation mixture were plated on skim milk (1.6%) Luria agar plates supplemented with 10 μg/ml of neomycin. Subsequently, the colonies with halos were inoculated in 120 μl of Luria broth media containing 10μg/mL neomycin for plasmid DNA extraction (QIAprep Spin Miniprep kit, Qiagen). The extracted plasmids were sequenced to confirm the presence of the desired mutations.

Table 4.1 Primer Sequences Used in Construction of NHJ4 Variants with QCMS method

| Mutations Introduced (BPN' Numbering) | Primer Name | Primer Sequence (5'-phosphorylated) |
|---|---|---|
| S101A S103G V104L | P5939 | AAAGTATTAGGGGCGAGCGGTGCAGGTGGACTTAGCTCGA TTGCCCAAGGATTG (SEQ ID NO:5) |
| G159E | P5940 | CATCTGGAAATTCAGGTGCAGAATCAATCAGCTATCCGGCC CGTTA (SEQ ID NO:6) |
| T22A | P5941 | CTGCCCATAACCGTGGATTGGCAGGTTCTGGTGTAAAAGTT GCTG (SEQ ID NO:7) |
| Y209E | P5942 | AGGTGTAAACGTGCAGAGCACAGAACCAGGTTCAACGTAT GCCAG(SEQ ID NO:8) |
| E271F | P5943 | GAAGCGGACTTGTCAATGCATTCGCTGCAACTCGTTAAAGC TTG (SEQ ID NO:9) |
| S101A | P5944 | AAAGTATTAGGGGCGAGCGGTGCAGGTTCGGTCAGCTCGAT TGCCCAA (SEQ ID NO:10) |
| S103G | P5945 | TATTAGGGGCGAGCGGTTCAGGTGGAGTCAGCTCGATTGCC CAAGGA (SEQ ID NO:11) |
| L111V | P5946 | GTCAGCTCGATTGCCCAAGGAGTAGAATGGGCAGGGAACA ATGGCA (SEQ ID NO:12) |
| S128N | P5947 | CGTTGCTAATTTGAGTTTAGGAAACCCTTCGCCAAGTGCCA CACTTGA (SEQ ID NO:13) |
| N62E | P5948 | GAACCATCCACTCAAGATGGGGAAGGGCATGGCACGCATG TG (SEQ ID NO:14) |

(continued)

Table 4.1 Primer Sequences Used in Construction of NHJ4 Variants with QCMS method

| Mutations Introduced (BPN' Numbering) | Primer Name | Primer Sequence (5'-phosphorylated) |
|---|---|---|
| S188D | P5949 | ACCAAAACAACAACCGCGCCGACTTTTCACAGTATGGCGCAGGGCTT (SEQ ID NO:15) |

b) Construction of NHJ4 variants by extension PCR

Ten combinatorial mutants of GG36 were created by extension PCR (Table 4-4). The list of mutations introduced in pHPLT-GG36 plasmid and primers used for this purpose is shown in Table 4-2. To create each mutant, several fragments (Table 4-3) were amplified by primers shown in Table 4-2. Each PCR amplification reaction contained 30 pmol of each primer and 100 ng of the DNA template, pHPLT-GG36 plasmid. Amplifications were carried out using Vent DNA polymerase (New England Biolabs). The PCR reaction (20 μL) was initially heated at 95°C for 2.5 min followed by 30 cycles of denaturation at 94°C for 15 sec., annealing at 55°C for 15 sec. and extension at 72°C for 1 min. Following amplification, 2 to 4 PCR fragments (Table 4-3) for each variant were gel-purified, using a QIAGEN® gel-band purification kit and mixed (50 ng of each fragment). These mixtures served as DNA templates for the extension PCR by primers P5954 and P5955 to generate the full-length gene fragment. The PCR conditions were the same as described above, except the extension phase, which was carried out at 72°C for 2 min. The full-length DNA fragment was gel-purified using a QIAGEN® gel-band purification kit, digested with the *Bam*HI and *Hin*dIII restriction enzymes, and ligated with the pHPLT-GG36 vector digested with the same restriction enzymes. The ligation mixtures were amplified using rolling circle amplification and transformed in *B. subtilis* cells as described for the QCMS method above. The GG36 variants obtained were sequenced to confirm the presence of the desired mutations. Variants created by the extension PCR are shown in Table 4-4.

TABLE 4-2: List of Primers Used for Construction of NHJ4 Variants with Extension PCR

| Mutation (BPN' numbering) | Primer name | Forward or Reverse | Primer Sequence |
|---|---|---|---|
| | P5950 flanking | Forward | CATATGAGTTATGCAGTTTGTAG (SEQ ID NO:16) |
| | P5951 flanking | Reverse | TGTTTTTCTTGGAATTGTGCTGT (SEQ ID NO:17) |
| | P5954 flanking | Forward | CAGTTTGTAGAATGCAAAAAGTG (SEQ ID NO:18) |
| | P5955 flanking | Reverse | GACAAGGTAAAGGATAAAACAGC (SEQ ID NO:19) |
| T22A | P5956 | Forward | CATAACCGTGGATTGGCAGGTTCT GGTGTAAAAGTTGCTG (SEQ ID NO:20) |
| T22A | P5957 | Reverse | ACTTTTACACCAGAACCTGCCAAT CCACGGTTATGGGCAG (SEQ ID NO:21) |
| S103G | P5960 | Forward | GCGAGCGGTTCAGGTGGAGTCAG CTCGATTGCCCAAGGA (SEQ ID NO:24) |
| S103G | P5961 | Reverse | TGGGCAATCGAGCTGACTCCACCT GAACCGCTCGCCCCTA (SEQ ID NO:25) |
| S103G L111V | P5962 | Forward | GTGGAGTCAGCTCGATTGCCCAAG GAGTAGAATGGGCAGGGAACAATGGCAT (SEQ ID NO:26) |
| S103G L111V | P5963 | Reverse | CATTCTACTCCTTGGGCAATCGAG CTGACTCCACCTGAACCGCTCGCCCCTA (SEQ ID NO:27) |

(continued)

TABLE 4-2: List of Primers Used for Construction of NHJ4 Variants with Extension PCR

| Mutation (BPN' numbering) | Primer name | Forward or Reverse | Primer Sequence |
|---|---|---|---|
| S101G S103A V104I | P5964 | Forward | GCGAGCGGTGGAGGTGCGATCAGCTC GATTGCCCAAGGATTG (SEQ ID NO:28) |
| S101G S103A V104I | P5965 | Reverse | CTTGGGCAATCGAGCTGATCGCACCT CCACCGCTCGCCCCTAATACTTTA (SEQ ID NO:29) |
| S101A S103G V104L | P5966 | Forward | GCGAGCGGTGCAGGTGGACTTAGCTC GATTGCCCAAGGATTG (SEQ ID NO:30) |
| S101A S103G V104L | P5967 | Reverse | CTTGGGCAATCGAGCTAAGTCCACCT GCACCGCTCGCCCCTAATACTTTA (SEQ ID NO:31) |
| S101A | P5968 | Forward | TATTAGGGGCGAGCGGTGCAGGTTCGG TCAGCTCGATTGC (SEQ ID NO:32) |
| S101A | P5969 | Reverse | ATCGAGCTGACCGAACCTGCACCGCTC GCCCCTAATACTTTA (SEQ ID NO:33) |
| S128N | P5970 | Forward | CTAATTTGAGTTTAGGAAACCCTTCGC CAAGTGCCACACTT (SEQ ID NO:34) |
| S128N | P5971 | Reverse | GCACTTGGCGAAGGGTTTCCTAAACTC AAATTAGCAACGTG (SEQ ID NO:35) |
| G159D | P5972 | Forward | GAAATTCAGGTGCAGACTCAATCAGCT ATCCGGCCCGTT (SEQ ID NO:36) |
| G159D | P5973 | Reverse | GGATAGCTGATTGAGTCTGCACCTGAA TTTCCAGATGC (SEQ ID NO:37) |
| G159E | P5974 | forward | GAAATTCAGGTGCAGAATCAATCAGCT ATCCGGCCCGTT (SEQ ID NO:38) |
| G159E | P5975 | reverse | GGATAGCTGATTGATTCTGCACCTGAAT TTCCAGATGC (SEQ ID NO:39) |
| Y209E | P5976 | forward | AACGTGCAGAGCACAGAACCAGGTTCA ACGTATGCCAGCTT (SEQ ID NO:40) |

(continued)

TABLE 4-2: List of Primers Used for Construction of NHJ4 Variants with Extension PCR

| Mutation (BPN' numbering) | Primer name | Forward or Reverse | Primer Sequence |
|---|---|---|---|
| Y209E | P5977 | reverse | CATACGTTGAACCTGGTTCTGTGCTCTG CACGTTTACACC (SEQ ID NO:41) |
| L111V | P5978 | forward | TCGATTGCCCAAGGAGTAGAATGGGCA GGGAACAATGGCAT (SEQ ID NO:42) |
| L111V | P5979 | reverse | CATTGTTCCCTGCCCATTCTACTCCTTGG GCAATCGAGCTGAC (SEQ ID NO:43) |

TABLE 4-3: Combinatorial Variants Created by Extension PCR

| Variant # | Variants (BPN numbering) | Fragment | PCR1 Fragments |
|---|---|---|---|
| NHJ4-1 | S101G S103A V104I | 1<br>2 | P5950+P5965<br>P5964+P5951 |
| NHJ4-2 | S101G S103A V104I G159D | 3<br>4<br>5 | P5950+P5965<br>P5964+P5973<br>P5972+P5951 |
| NHJ4-3 | S101A S103G V104L | 6<br>7 | P5950+P5967<br>P5966+P5951 |
| NHJ4-4 | S101A S103G V104L G159E | 8<br>9<br>10 | P5950+P5967<br>P5966+P5975<br>P5974+P5951 |
| NHJ4-5 | S101A S103G V104L T22A | 11<br>12<br>13 | P5950+P5957<br>P5956+P5967<br>P5966+P5951 |
| NHJ4-10 | T22A S101A Y209E | 14<br>15<br>16<br>17 | P5950+P5957<br>P5956+P5969<br>P5968+P5977<br>P5976+P5951 |
| NHJ4-12 | T22A S103G G159E | 21<br>22<br>23<br>24 | P5950+P5957<br>P5956+P5961<br>P5960+P5975<br>P5974+P5951 |
| NHJ4-20 | S101A S103G V104L S128N | 29<br>30<br>31 | P5950+P5967<br>P5966+P5971<br>P5970+P5951 |

To express the NHJ4 set of variant proteins for further biochemical analyses, the *B. subtilis* strains carrying the variant plasmids were inoculated into microtiter plates containing 150μl Luria broth medium supplemented with 10μg/ml neomycin. The cultures were grown up for protein expression as described in Example 31, and they were filtered through a micro-filter plate (0.22μm; Millipore) also as described in Example 31. The resulting filtrate was used for biochemical analysis. The eglin c inhibition assay for protein content determination and BMI microswatch assays tested in various

detergents were carried out as described in the Test Methods section. Performance indices are also calculated as described under the BMI microswatch assay description in the Test Methods section.

| TABLE 4-4: NHJ4 Multiple Mutation Variants. Performance Index (PI) Relative to GG36 (determined by Test Method 3 - detergent of Example 26, 16C) | | |
|---|---|---|
| **Mutations versus SEQ ID NO:1 (BPN' numbering)** | PI | Variant Name (method for construction) |
| T22A-L111V-G159E | 1.4 | NHJ4-13 (QCMS) |
| T22A-S103G-G159E | 1.2 | NHJ4-12 (Extension PCR) |
| T22A-S128N-E271F-Y209E | 1.2 | NHJ4-14 (QCMS) |
| T22A-Y209E-E271F | 1.2 | NHJ4-7 (QCMS) |
| T22A-S101A-Y209E | 1.1 | NHJ4-10 (Extension PCR) |
| T22A-L111V-S128N | 1.1 | NHJ4-17 (QCMS) |
| T22A-S101A-G159E | 1.1 | NHJ4-24 (QCMS) |
| T22A-S101A-S103G-V104L | 1.1 | NHJ4-5 (Extension PCR) |

EXAMPLE 34

Construction and Cleaning Performance of NHJ3 Set of GG36 Variants

The NHJ3 set of variants described herein are based on a variant of GG36 (referred to as GG36-9) containing the following mutations: S101G, S103A, V104I, G159D, A232V, Q236H, Q245R, N248D, and N252K (BPN' numbering). These variants were created using the QuikChange® Lightning Multi Site-Directed Mutagenesis Kit (QCLMS kit; Stratagene), with the pRA68 plasmid (*See*, Figure 3) as the DNA template. Plasmid pRA68 was derived from the pBN3 vector (*See*, Babé et al., Biotech. Appl. Biochem. 27:117-124 [1998]).

The DNA sequence of GG36-9 variant (the signal sequence is shown in lower case letters, propeptide in lower case, underlined text, and GG36-9 mature sequence in uppercase letters) is provided below:

Gtgagaagcaaaaaattgtggatcgtcgcgtcgaccgcactactcatttctgttgcttttagttcatcgatcgcatcggctgctgaagaagcaa
aagaaaaatatttaattggctttaatgagcaggaagctgtcagtgagtttgtagaacaagtagaggcaaatgacgaggtcgccattctctctga
ggaagaggaagtcgaaattgaattgcttcatgaatttgaaacgattcctgttttatccgttgagttaagcccagaagatgtggacgcgcttgaac
tcgatccagcgatttcttatattgaagaggatgcagaagtaacgacaatgGCGCAATCAGTGCCATGGGGAATTAG
CCGTGTGCAAGCCCCGGCTGCCCATAACCGTGGATTGACAGGTTCTGGTGTAAAAGT
TGCTGTCCTCGATACAGGTATTTCCACTCATCCAGACTTAAATATTCGTGGTGGCGCT
AGCTTTGTACCAGGGGAACCATCCACTCAAGATGGGAATGGGCATGGCACGCATGT
GGCCGGGACGATTGCTGCTCTAAACAATTCGATTGGCGTACTTGGCGTAGCGCCGAG
CGCGGAACTATACGCTGTTAAAGTATTAGGGGCGAGCGGTGGGGGCGCCATCAGCT
CGATTGCCCAAGGATTGGAATGGGCAGGGAACAATGGCATGCACGTTGCTAATTTG
AGTTTAGGAAGCCCTTCGCCAAGTGCCACACTTGAGCAAGCTGTTAATAGCGCGACT
TCTAGGGGCGTTCTTGTTGTAGCGGCATCTGGAAATTCGGGTGCAGACTCAATCAGC
TATCCGGCCCGTTATGCGAACGCAATGGCAGTCGGAGCTACTGACCAAAACAACAA
CCGCGCCAGCTTTTCACAGTATGGCGCAGGGCTTGACATCGTCGCACCAGGTGTAAA
CGTGCAGAGCACATACCCAGGTTCAACGTATGCCAGCTTAAACGGTACATCGATGGC
TACTCCTCATGTTGCAGGTGCAGCAGTCCTTGTTAAACATAAGAACCCATCTTGGTCC
AATGTACGAATCCGCGATCATCTAAAGAAAACGGCAACGAGCTTAGGAAGCACGAA
CTTGTATGGAAGCGGACTTGTCAATGCCGAAGCTGCAACTCGTTAA (SEQ ID NO:44)

The protein sequence of the GG36-9 variant (the signal sequence is shown in lower case letters, propeptide in lower case, underlined text, and GG36-9 mature protease sequence in uppercase letters) is provided below:

vrskklwivastallisvafsssiasaaeeakekyligfneqeavsefveqveandevailseeeeveiellhefetipvlsvelspedvdal
eldpaisyieedaevttmAQSVPWGISRVQAPAAHNRGLTGSGVKVAVLDTGISTHPDLNIRGGA
SFVPGEPSTQDGNGHGTHVAGTIAALNNSIGVLGVAPSAELYAVKVLGASGGGAISSIAQ
GLEWAGNNGMHVANLSLGSPSPSATLEQAVNSATSRGVLVVAASGNSGADSISYPARY
ANAMAVGATDQNNNRASFSQYGAGLDIVAPGVNVQSTYPGSTYASLNGTSMATPHVA
GAAVLVKHKNPSWSNVRIRDHLKKTATSLGSTNLYGSGLVNAEAATR (SEQ ID NO:45)

To create the NHJ3 variants using the QCLMS kit, mutagenic primers were designed as shown in Table 5-1 for each of the variants. The mutagenesis reaction for each variant consisted of 0.5 μl pRA68 plasmid DNA (168ng/μL), 0.5μl forward "f" mutagenic primer (25μM), 0.5μl reverse "r" mutagenesis primer (25μM), 1ul dNTPs (supplied in the QCLMS kit), 1.5μl Quik solution (supplied in the QCLMS kit), 1μl Enzyme blend (supplied in the QCLMS kit), and 39.5μl of distilled, deionized water to make up a 50 μL reaction volume as per the manufacturer's instructions. The cycling program was 1cycle at 95°C for 2 minutes, 18 cycles of 95°C for 20 seconds, 60°C for 10 seconds and 68°C for 3 minutes, 22 seconds, and a final cycle of 68°C for 5 minutes. Next, 1μL of DpnI restriction enzyme supplied in the kit was used to digest the plasmid DNA in the reaction, and then 2 μL of the reaction was used to transform TOP 10 *E.coli* competent cells (Invitrogen). The *E.coli* transformants were selected on LB plates containing 50 ug/mL(ppm) carbenicillin after overnight growth at 37°C. Plasmid DNA was extracted from 4-8 *E. coli* colonies grown in LA medium containing 50 ug/mL(ppm) carbenicillin using the QIAprep spin miniprep kit (Qiagen). The plasmids were sequenced to confirm the

presence of the desired mutations. The variant plasmids were then transformed into *B. subtilis* cells as described in Example 31. The *B. subtilis* variant strains were grown up as described in Example 31 for further biochemical analysis, such as protein content determination using the eglin c inhibition assay (Test Methods section) and the BMI microswatch cleaning assay (Test Methods section). The cleaning performance of the NHJ3 variants is shown in Tables 5-2 and 5-3.

TABLE 5-1: List of Mutagenic Primers Used to Create the NHJ3 Set of Variants (* "f" refers to forward primer and "r" refers to reverse primer)

| Variant | Mutation Incorporated (BPN' numbering) | Mutagenic Primer Name | Mutagenic Primer Sequence 5' to 3' |
|---|---|---|---|
| NHJ3-1 | G101S | p138_G101S f* | GTATTAGGGGCGAGCGGTGGCGGCGCCATCAG CTCGATTGC (SEQ ID NO:46) |
| | | p139_G101S r* | GCAATCGAGCTGATGGCGCCGCCACCGCTCGCC CCTAATAC (SEQ ID NO:47) |
| | | | |
| NHJ3-2 | A103S | p140_A1013 f | GGGGCGAGCGGTGGGGGCGGCATCAGCTCGATT GCCCAAG (SEQ ID NO:48) |
| | | p141_A1013 r | CTTGGGCAATCGAGCTGATGCCGCCCCCACCGCT CGCCCC (SEQ ID NO:49) |
| NHJ3-3 | I104V | p142_I104V f | GGGCGAGCGGTGGGGGCGCCGTTAGCTCGATTGC CCAAGGATTG (SEQ ID NO:50) |
| | | p143_I104V r | CAATCCTTGGGCAATCGAGCTAACGGCGCCCCCA CCGCTCGCCC (SEQ ID NO:51) |
| | | | |
| NHJ3-4 | D159G | p144_D159G f | GCATCTGGAAATTCGGGTGCAGGCTCAATCAGCT ATCCGGCCCGT (SEQ ID NO:52) |
| | | p145_D159G r | ACGGGCCGGATAGCTGATTGAGCCTGCACCCGAA TTTCCAGATGC (SEQ ID NO:53) |
| | | | |
| NHJ3-5 | V232A | p146_V232A f | CTCATGTTGCAGGTGCAGCAGCACTTGTTAAACAT AAGAACCC (SEQ ID NO:54) |
| | | p147_V232A r | GGGTTCTTATGTTTAACAAGTGCTGCTGCACCTGC AACATGAG (SEQ ID NO:55) |
| | | | |

(continued)

TABLE 5-1: List of Mutagenic Primers Used to Create the NHJ3 Set of Variants (* "f" refers to forward primer and "r" refers to reverse primer)

| Variant | Mutation Incorporated (BPN' numbering) | Mutagenic Primer Name | Mutagenic Primer Sequence 5' to 3' |
|---|---|---|---|
| NHJ3-6 | H236Q | p148_H236Q f | GTGCAGCAGTCCTTGTTAAACAAAAGAACCCATC TTGGTCCAAT (SEQ ID NO:56) |
| | | p149_H236Q r | ATTGGACCAAGATGGGTTCTTTTGTTTAACAAGG ACTGCTGCAC (SEQ ID NO:57) |
| | | | |
| NHJ3-7 | R245Q | p150_R245Q f | CCATCTTGGTCCAATGTACAAATCCGCGATCATC TAAAGAAAAC (SEQ ID NO:58) |
| | | p151_R245Q r | GTTTTCTTTAGATGATCGCGGATTTGTACATTGG ACCAAGATGG (SEQ ID NO:59) |
| NHJ3-8 | D248N | P152_D248N f | GGTCCAATGTACGAATCCGCAATCATCTAAAGA AAACGGCAAC (SEQ ID NO:60) |
| | | P153_D248N r | GTTGCCGTTTTCTTTAGATGATTGCGGATTCGTA CATTGGACC (SEQ ID NO:61) |
| | | | |
| NHJ3-9 | K252N | P154_K252N f | GAATCCGCGATCATCTAAAGAATACGGCAACGA GCTTAGGAAG (SEQ ID NO:62) |
| | | P155_K252N r | CTTCCTAAGCTCGTTGCCGTATTCTTTAGATGATC GCGGATTC (SEQ ID NO:63) |

## EXAMPLE 35

Construction and cleaning performance of NHJ5 set of GG36 variants

The NHJ5 set of variants described herein are based on a variant of GG36 (referred to as GG36-7) containing the following mutations: S101G, S103A, V104I, G159D, A232V, Q245R, N248D (BPN' numbering). These variants were created as described in Example 34 using the OuikChange® Lightning Multi Site-Directed Mutagenesis Kit (QCLMS kit; Stratagene) with the pRA96 plasmid as the DNA template (Figure 4; plasmid pRA96 was derived from the pBN3 vector, which is described in Babe et al, Biotech. Appl. Biochem. 27:117-124, 1998). The mutations incorporated and the sequences of the primers used for introducing the mutations in GG36-7 are shown in Table 6-1. The variants were generated using the methods described in Example 34.The *B. subtilis* variant strains were grown up as described in Example 31 for further biochemical analysis, such as protein content determination using the eglin c inhibition assay (Test Methods section) and the BMI microswatch cleaning assay (Test Methods section). The cleaning performance of the NHJ5 set of variants is shown in Tables 6-2 and 6-3.

SEQ ID NO: 64 DNA sequence of GG36-7 variant (signal sequence is shown in lower case letters, propeptide in lower case, underlined text, and GG36-7 mature protease sequence in uppercase letters)

Gtgagaagcaaaaaattgtggatcgtcgcgtcgaccgcactactcatttctgttgcttttagttcatcgatcgcatcggctgctgaagaagcaa
aagaaaaatatttaattggctttaatgagcaggaagctgtcagtgagtttgtagaacaagtagaggcaaatgacgaggtcgccattctctctga
ggaagaggaagtcgaaattgaattgcttcatgaatttgaaacgattcctgttttatccgttgagttaagcccagaagatgtggacgcgcttgaac
tcgatccagcgatttcttatattgaagaggatgcagaagtaacgacaatgGCGCAATCAGTGCCATGGGGAATTAG
CCGTGTGCAAGCCCCGGCTGCCCATAACCGTGGATTGACAGGTTCTGGTGTAAAAGT
TGCTGTCCTCGATACAGGTATTTCCACTCATCCAGACTTAAATATTCGTGGTGGCGCT
AGCTTTGTACCAGGGGAACCATCCACTCAAGATGGGAATGGGCATGGCACGCATGT
GGCCGGGACGATTGCTGCTCTAAACAATTCGATTGGCGTACTTGGCGTAGCGCCGAG
CGCGGAACTATACGCTGTTAAAGTATTAGGGGCGAGCGGTGGGGGCGCCATCAGCT
CGATTGCCCAAGGATTGGAATGGGCAGGGAACAATGGCATGCACGTTGCTAATTTG
AGTTTAGGAAGCCCTTCGCCAAGTGCCACACTTGAGCAAGCTGTTAATAGCGCGACT
TCTAGGGGCGTTCTTGTTGTAGCGGCATCTGGAAATTCGGGTGCAGACTCAATCAGC
TATCCGGCCCGTTATGCGAACGCAATGGCAGTCGGAGCTACTGACCAAAACAACAA
CCGCGCCAGCTTTTCACAGTATGGCGCAGGGCTTGACATCGTCGCACCAGGTGTAAA
CGTGCAGAGCACATACCCAGGTTCAACGTATGCCAGCTTAAACGGTACATCGATGGC
TACTCCTCATGTTGCAGGTGCAGCAGTCCTTGTTAAACAAAAGAACCCATCTTGGTC
CAATGTACGAATCCGCGATCATCTAAAGAATACGGCAACGAGCTTAGGAAGCACGA
ACTTGTATGGAAGCGGACTTGTCAATGCCGAAGCTGCAACTCGT

SEQ ID NO: 65 Protein sequence of GG36-7 variant (signal sequence is shown in lower case letters, propeptide in lower case, underlined text, and GG36-7 mature protease sequence in uppercase letters)

vrskklwivastallisvafsssiasaaeeakekyligfneqeavsefveqveandevailseeeeveiellhefetipvlsvelspedvdaleldpaisyieedaevttmAQSVPWGISRVQAPAAHNRGLTGSGVKVAVLDTGISTHPDLNIRGGASFVPGEPSTQDGNGHGTHVAGTIAALNNSIGVLGVAPSAELYAVKVLGASGGGAISSIAQGLEWAGNNGMHVANLSLGSPSPSATLEQAVNSATSRGVLVVAASGNSGADSISYPARYANAMAVGATDQNNNRASFSQYGAGLDIVAPGVNVQSTYPGSTYASLNGTSMATPHVAGAAVLVKQKNPSWSNVRIRDHLKNTATSLGSTNLYGSGLVNAEAATR

Table 6-1: List of primers used to create the NHJ5 set of variants

| Primer # | Mutations used GG36 numbering (BPN' numbering shown in parentheses) | Primer sequence |
|---|---|---|
| 168 | H243R (H249R) | GTACGAATCCGCGATAGACTAAAGAATACGGCAACGAG (SEQ ID NO:66) |
| 170 | E265F (E271F) | GCGGACTTGTCAATGCCTTTGCTGCAACTCGTTAAAGCTTACAT (SEQ ID NO:67) |
| 172 | D157E (D159E) | GGAAATTCGGGTGCAGAATCAATCAGCTATCCGGCCCGTTA (SEQ ID NO:68) |
| 174 | A156E (A158E) | CTGGAAATTCGGGTGAAGACTCAATCAGCTATCCGGCC (SEQ ID NO:69) |
| 176 | A156E-D157G (A158E-D159E) | CGGCATCTGGAAATTCGGGTGAAGGCTCAATCAGCTATCCGGCCCGTTATG (SEQ ID NO:70) |
| 178 | T22A | CATAACCGTGGATTGGCAGGTTCTGGTGTAAAAGTTGCTGTC (SEQ ID NO:71) |
| 180 | N60E (N62E) | TCCACTCAAGATGGGGAAGGGCATGGCACGCATGTGGC (SEQ ID NO:72) |
| 182 | N232R (N238R) | CTTGTTAAACAAAAGAGACCATCTTGGTCCAATGTACGAATC (SEQ ID NO:73) |
| 186 | T247R (T253R) | AATGTACGAATCCGCAGACATCTAAAGAATACGGCAACGAGC (SEQ ID NO:74) |
| 188 | S24R | CGATCATCTAAAGAATAGAGCAACGAGCTTAGGAAGCACGAAC (SEQ ID NO:75) |
| 190 | N74D (N76D) | GTGGATTGACAGGTAGAGGTGTAAAAGTTGCTGTCCTCGATA (SEQ ID NO:76) |

TABLE 6-2: Cleaning performance of NHJ5 variants using Test Method 3. Performance Index (PI) Relative to GG36.

| Mutations versus SEQ ID NO:1 (BPN' numbering) | PI | Variant Name |
|---|---|---|
| T22A-S101G-S103A-V104I-G159D-A232V-Q245R-N248D-H249R | 1.3 | NHJ5-11 |

(continued)

| Mutations versus SEQ ID NO:1 (BPN' numbering) | PI | Variant Name |
|---|---|---|
| T22A-S101G-S103A-V104I-G159D-A232V-Q245R-N248D-E271F | 1.3 | NHJ5-12 |

**EXAMPLE 36**

The below table (6-4) exemplifies the detergent concentrations and buffers used while Examples 36a - 36n show the detergent types used.

| TABLE 6-4: Final Detergent, Water Hardness, and Buffer Concentrations Used for BMI Microswatch Assays | | | |
|---|---|---|---|
| **Detergent Composition** | **Final Detergent Concentration (g/L)** | **Final Water Hardness* (gpg)** | **Final Sodium Carbonate Buffer Concentration (mM)** |
| 36a | 0.75 | 6 | 2 |
| 36b | 0.808 | 6 | 2 |
| 36c | 0.808 | 6 | 2 |
| 36d | 2.25 | 12 | 2 |
| 36e | 1 | 3 | 2 |
| 36f | 1.2 | 12 | 2 |
| 36g | 3.96 | 12 | 2 |
| 36h | 7.69 | 20 | 2 |
| 36i | 5 | 10 | 2 |
| 36j | 7.69 | 20 | 2 |
| 36k | 7.69 | 20 | 2 |
| 361 | 6.15 | 10 | 2 |
| 36m | 7.69 | 20 | 2 |
| 36n | 6.15 | 20 | 2 |

The following are granular laundry detergent compositions. The protease of this invention is separately added to these formulations.

| | 36a | 36b | 36c | 36d | 36e |
|---|---|---|---|---|---|
| **Surfactants** | | | | | |
| $C_{10}$ Nonionic | | | | 0.1843 | |
| $C_{16-17}$ Branched alkyl sulfate | 3.53 | 3.53 | 3.53 | | |
| $C_{12-14}$ alkyl sulphate | | | | | |
| Sodium linear alkylbenzenesulfonate with aliphatic chain length $C_{11}$-$C_{12}$ | 8.98 | 8.98 | 8.98 | 13.58 | 14.75 |
| Sodium $C_{14/15}$ alcohol ethoxy – 3 - sulfate | 1.28 | 1.28 | 1.28 | | |
| Sodium $C_{14/15}$ alkyl sulphate | 2.36 | 2.36 | 2.36 | | |
| $C_{14/15}$ alcohol ethoxylate with average 7 moles of ethoxylation | | | | | |
| mono-$C_{8-10}$ alkyl mono-hydroxyethyl di-methyl quaternary ammonium chloride | | | | | |
| Di methyl hydroxyl ethyl lauryl ammonium chloride | | | | 0.1803 | |
| Zeolite A | 15.31 | 15.31 | 15.31 | | 4.47 |
| Bentonite | | | | 8.35 | |
| Sodium Silicate 1.6.ratio | | | | | 0.16 |
| Sodium Silicate 2.0.ratio | 3.72 | 3.72 | 3.72 | 8.41 | |
| Sodium Silicate 2.35.ratio | | | | | |
| Citric Acid | | | | 0.0066 | |
| Sodium tripolyphosphate | | | | 5.06 | |

| | | | | | |
|---|---|---|---|---|---|
| Sodium Carbonate | 26.1 | 26.18 | 26.1 | 15.9 | 29.0 |
| Nonanoyloxybenzenesuplhonate | 5.78 | 5.78 | 5.78 | 1.17 | 1.86 |
| Oxaziridinium-based bleach booster | 0.037 | 0.037 | 0.037 | | |
| Tetrasodium S,S,-ethylenediaminedisuccinate | | | | | |
| Diethylenetriamine penta (methylene phosphonic acid), heptasodium salt | 0.62 | 0.62 | 0.62 | | |
| Hydroxyethane dimethylene phosphonic acid | | | | | |
| Ethylene diamine tetraacetate | | | | 0.2701 | |
| MgSO4 | 0.056 | 0.056 | 0.056 | 0.47 | |
| Sodium Percarbonate | | 7.06 | 7.06 | | 3.64 |
| Tetra Acetyl Ethylene Diamine | | | | | |
| Sodium Perborate Monohydrate | | | | 1.47 | |
| Carboxymethyl cellulose (e.g. Finnfix BDA ex CPKelco) | 0.38 | 0.38 | 0.38 | 0.173 | |
| Sodium Acrylic acid/maleic acid co-polymer (70/30) | 3.79 | 3.78 | 3.79 | | 3.64 |
| Sodium polyacrylate (Sokalan PA30 CL) | 3.78 | 3.78 | 3.78 | 0.842 | |
| Terephthalate polymer | | | | | |
| Polyethylene glycol/vinyl acetate random graft co polymer | | | | 0.89 | |
| Photobleach- zinc phthalocyanine tetrasulfonate | | | | | |
| C.I.Fluorescent Brightener 260 | 0.1125 | 0.1125 | 0.1125 | 0.043 | 0.15 |
| C.I.Fluorescent Brightener 351 (Tinopal ® CBS) | | | | 0.0952 | |
| Suds suppressor granule | 0.015 | 0.015 | 0.015 | | 0.031 |
| Hyrdophobically modified carboxy methyl cellulose (Finnifix ® SH-1) | | | | | |
| Bentonite | | | | | |
| Miscellaneous (Dyes, perfumes, process aids, moisture and sodium sulphate) | Balance | Balance | Balance | Balance | Balance |

| | **36f** | **36g** | **36h** | **36i** | **36j** |
|---|---|---|---|---|---|
| **Surfactants** | | | | | |
| $C_{10}$ Nonionic | 0.1142 | 0.2894 | 0.1885 | 0.1846 | 0.1885 |
| $C_{16-17}$ Branched alkyl sulfate | | | | | |
| $C_{12-14}$ alkyl sulphate | | | | | |
| Sodium linear alkylbenzenesulfonate with aliphatic chain length $C_{11}$-$C_{12}$ | 12.94 | 15.69 | 9.01 | 8.42 | 9.51 |
| Sodium $C_{14/15}$ alcohol ethoxy – 3 - sulfate | | | | | |
| Sodium $C_{14/15}$ alkyl sulphate | | | | | |

| | | | | | |
|---|---|---|---|---|---|
| $C_{12/14}$ alcohol ethoxylate with average 7 moles of ethoxylation | 2.9 | | | | |
| $C_{12/14}$ alcohol ethoxylate with average 3 moles of ethoxylation | | | | 2.44 | |
| $C_{14/15}$ alcohol ethoxylate with average 7 moles of ethoxylation | | | 0.97 | 1.17 | 0.97 |
| mono-$C_{8-10}$ alkyl mono-hydroxyethyl di-methyl quaternary ammonium chloride | | | 0.45 | | |
| Di methyl hydroxyl ethyl lauryl ammonium chloride | | 0.195 | | | 0.45 |
| Zeolite A | 2.01 | 0.39 | 1.83 | 2.58 | 0.59 |
| Sodium Silicate 1.6.ratio | | | 4.53 | 5.62 | 4.53 |
| Sodium Silicate 2.0.ratio | | 10.1 | | | |
| Sodium Silicate 2.35.ratio | 7.05 | | | | |
| Citric Acid | | | 1.4 | 1.84 | 1.0 |
| Sodium tripolyphosphate | | 5.73 | | | |
| Sodium Carbonate | 12.65 | 15.93 | 21.0 | 27.31 | 20.2 |
| Nonanoyloxybenzenesuplhonate | | 1.73 | | | |
| Oxaziridinium-based bleach booster | | | 0.0168 | 0.0333 | 0.024 |
| Tetrasodium S,S,-ethylenediaminedisuccinate | | | | | |
| Diethylenetriamine penta (methylene phosphonic acid), heptasodium salt | | | 0.327 | | 0.3272 |
| Hydroxyethane dimethylene phosphonic acid | | | 0.45 | 0.2911 | 0.45 |
| Ethylene diamine tetraacetate | | 0.28 | | 0.1957 | |
| MgSO4 | | 0.54 | 0.79 | 0.6494 | 0.793 |
| Sodium Percarbonate | | | 19.1 | 15.85 | 22.5 |
| Tetra Acetyl Ethylene Diamine | | | 4.554 | 3.71 | 5.24 |
| Sodium Perborate Monohydrate | | 5.55 | | | |
| Carboxymethyl cellulose (e.g. Finnfix BDA ex CPKelco) | 0.62 | 0.21 | 0.23 | 1.07 | 0.2622 |
| Sodium Acrylic acid/maleic acid co-polymer (70/30) | 0.40 | 2.61 | 2.5 | 2.00 | 1.75 |
| Sodium polyacrylate (Sokalan PA30 CL) | | | 0.0055 | 0.011 | 0.008 |
| Terephthalate polymer | | | | 0.231 | |
| Polyethylene glycol/vinyl acetate random graft co polymer | 0.55 | 1.40 | 0.911 | 0.8924 | 0.911 |
| Photobleach- zinc phthalocyanine tetrasulfonate | | | | | |
| C.I.Fluorescent Brightener 260 | 0.1174 | 0.048 | 0.1455 | 0.2252 | 0.1455 |
| C.I.Fluorescent Brightener 351 (Tinopal ® CBS) | | 0.1049 | | | |
| Suds suppressor granule | | | 0.04 | 0.0658 | 0.04 |

| | | | | | |
|---|---|---|---|---|---|
| Hyrdophobically modified carboxy methyl cellulose (Finnifix ® SH-1) | | | | | |
| Bentonite | | | | | |
| Miscellaneous (Dyes, perfumes, process aids, moisture and sodium sulphate) | Balance | Balance | Balance | Balance | Balance |

| | **36k** | **36l** | **36m** | **36n** | |
|---|---|---|---|---|---|
| **Surfactants** | | | | | |
| $C_{10}$ Nonionic | 0.1979 | 0.1979 | 0.1979 | 0.1979 | |
| $C_{16-17}$ Branched alkyl sulfate | | | | | |
| $C_{12-14}$ alkyl sulphate | | | | | |
| Sodium linear alkylbenzenesulfonate with aliphatic chain length $C_{11}$-$C_{12}$ | 8.92 | 8.92 | 11.5 | 11.5 | |
| Sodium $C_{14/15}$ alcohol ethoxy – 3 - sulfate | 1.62 | 1.62 | 1.125 | 1.125 | |
| Sodium $C_{14/15}$ alkyl sulphate | | | | | |
| $C_{14/15}$ alcohol ethoxylate with average 7 moles of ethoxylation | 1.0 | 1.0 | 1.5 | 1.5 | |
| mono-$C_{8-10}$ alkyl mono-hydroxyethyl di-methyl quaternary ammonium chloride | | | | | |
| Di methyl hydroxyl ethyl lauryl ammonium chloride | | | | | |
| Zeolite A | 1.63 | 1.63 | 2.0 | 2.0 | |
| Sodium Silicate 1.6.ratio | 4.75 | 4.75 | 4.75 | 4.75 | |
| Sodium Silicate 2.0.ratio | | | 0.06 | 0.06 | |
| Sodium Silicate 2.35.ratio | | | | | |
| Citric Acid | 1.10 | 1.10 | 1.1 | 1.1 | |
| Sodium tripolyphosphate | | | | | |
| Sodium Carbonate | 23.3 | 23.3 | 23.3 | 23.3 | |
| Nonanoyloxybenzenesuplhonate | | | | | |
| Oxaziridinium-based bleach booster | 0.021 | 0.021 | 0.015 | 0.015 | |
| Tetrasodium S,S,-ethylenediaminedisuccinate | 0.26 | 0.26 | 0.26 | 0.26 | |
| Diethylenetriamine penta (methylene phosphonic acid), heptasodium salt | | | | | |
| Hydroxyethane dimethylene phosphonic acid | 0.47 | 0.47 | 0.47 | 0.47 | |
| Ethylene diamine tetraacetate | | | | | |
| MgSO4 | 0.83 | 0.83 | 0.82 | 0.82 | |
| Sodium Percarbonate | 19.35 | 19.35 | 19.35 | 19.35 | |
| Tetra Acetyl Ethylene Diamine | 4.51 | 4.51 | 4.51 | 4.51 | |
| Sodium Perborate Monohydrate | | | | | |
| Carboxymethyl cellulose (e.g. Finnfix BDA ex CPKelco) | 1.01 | 1.01 | 1.01 | 1.01 | |

| | | | | | |
|---|---|---|---|---|---|
| Sodium Acrylic acid/maleic acid co-polymer (70/30) | 1.84 | 1.84 | 1.84 | 1.84 | |
| Sodium polyacrylate (Sokalan PA30 CL) | 0.007 | 0.007 | 0.005 | 0.005 | |
| Terephthalate polymer | 0.179 | 0.179 | 0.179 | 0.179 | |
| Polyethylene glycol/vinyl acetate random graft co polymer | 0.96 | 0.96 | 0.96 | 0.96 | |
| Photobleach- zinc phthalocyanine tetrasulfonate | | | | | |
| C.I.Fluorescent Brightener 260 | 0.153 | 0.153 | 0.171 | 0.171 | |
| C.I.Fluorescent Brightener 351 (Tinopal ® CBS) | | | | | |
| Suds suppressor granule | 0.042 | 0.042 | 0.042 | 0.042 | |
| Hyrdophobically modified carboxy methyl cellulose (Finnifix ® SH-1) | | | | | |
| Bentonite | | | | | |
| Miscellaneous (Dyes, perfumes, process aids, moisture and sodium sulphate) | Balance | Balance | Balance | Balance | Balance |

Notes for examples 36 a - n:

Surfactant ingredients can be obtained from BASF, Ludwigshafen, Germany (Lutensol®); Shell Chemicals, London, UK; Stepan, Northfield, Illinois, USA; Huntsman, Huntsman, Salt Lake City, Utah, USA; Clariant, Sulzbach, Germany (Praepagen®).

Zeolite can be obtained from Industrial Zeolite (UK) Ltd, Grays, Essex, UK.

Citric acid and sodium citrate can be obtained from Jungbunzlauer, Basel, Switzerland.

Sodium percarbonate, sodium carbonate, sodium bicarbonate and sodium sesquicarbonate can be obtained from Solvay, Brussels, Belgium.

Acrylate/maleate copolymers can be obtained from BASF, Ludwigshafen, Germany.

Carboxymethylcellulose and hydrophobically modified carboxymethyl cellulose can be obtained from CPKelco, Arnhem, The Netherlands.

C.I. Fluorescent Brightener 260 can be obtained from 3V Sigma, Bergamo, Italy as Optiblanc® Optiblanc® 2M/G, Optiblanc® 2MG/LT Extra, or Optiblanc® Ecobright.

Tetrasodium S,S-ethylenediamine disuccinate can be obtained from Innospec, Ellesmere Port, UK.

Terephthalate co-polymer can be obtained from Clariant under the tradename Repelotex SF 2.

1-Hydroxyethane -1,1-diphosphonic acid can be obtained from Thermphos, Vlissingen-Oost, The Netherlands.

Oxaziridinium-based bleach booster has the following structure, where R1 = 2-butyloctyl, and was produced

according to US 2006/0089284A1.

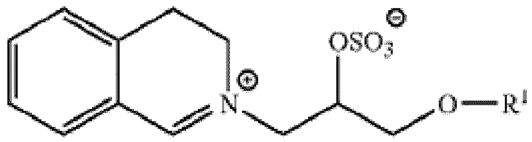

Enzymes Natalase ®, Termamyl ®, Stainzyme Plus®, Celluclean® and Mannaway ®, can be obtained from Novozymes, Bagsvaerd, Denmark.
Zinc phthalocyanine tetrasulfonate can be obtained from Ciba Specialty Chemicals, Basel, Switzerland, as Tinolux® BMC.
Suds suppressor granule can be obtained from Dow Corning, Barry, UK.
Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units.

## EXAMPLE 37

### Construction of NHJ2 Combinatorial Library

This Example describes the construction of a GG36 combinatorial library involving one or more of the following mutations: A016S, T022A, S101A, S103G, V104L, L111V, S128N, and L148I (BPN' numbering). The pHPLT-GG36 *B. subtilis* expression plasmid was provided to DNA 2.0 Inc.,for the generation of NHJ2 combinatorial library. A ligation reaction of the constructed NHJ2 library was provided by DNA 2.0, Inc. for transformation in the *B. subtilis* strain (genotype: ΔaprE, ΔnprE, amyE::xylRPxylAcomK-phleo). The variants generated containing one or several of the mutations described herein are tested for cold water cleaning applications using methods and detergent compositions described herein.

## EXAMPLE 38

### Construction of Additional Libraries and Variants of GG36

This Example describes the cold water cleaning of GG36 combinatorial variants containing one or more of the following mutations: A016S, T022A, S024R, N062E, N076D, E089P, S101A/G, S103G/A, V104L/I, L111V, S128N, P129E, A232V, L148I, A158E, G159D/E, S166D, R186H, S188D, Y209E, Q236H, N238R, Q245R, N248D/R, H249R, N252K/R, T253R, E271F (BPN' numbering). The combinatorial variants were constructed by DNA2.0, Inc., using a *B. subtilis* expression plasmid (*e.g.*, pHPLT-GG36; Fig. 2) and the *B. subtilis* strain (genotype: ΔaprE, ΔnprE, amyE::xylRPxylAcomK-phleo). In the case of DNA libraries, a ligation reaction of each of the constructed libraries using a *B. subtilis* expression plasmid (*e.g.*, pHPLT-GG36; Fig. 2) was provided by DNA 2.0, Inc. for transformation in the *B. subtilis* strain (genotype: ΔaprE, ΔnprE, amyE::xylRPxylAcomK-phleo). The variants generated containing one or more of the mutations listed above were tested for cold water cleaning in the BMI microswatch assay (TEST METHOD 6) using the methods and detergent compositions described in this application.

**TABLE 11-1. BMI cleaning performance of NHJ6 library variants.**
**PI = Performance Index**
**PI> or = 1.5 is +++; PI between 1.49 and 1.3 = ++; PI between 1.29 and 1.1 = + in detergent b, d or e**

| Sequence of GG36 Variants (BPN' numbering) | PI Relative to GG36; BMI assay; Detergent of Example 36b, 36d or 36e, 16C |
|---|---|
| T022A-S101G-S103A-V104I-P129E-A158E-S188D-A232V-Q245R-N248D-H249R | +++ |
| T022A-S024R-S101G-S103A-V104I-P129E-A158E-S188D-A232V-Q245R-N248D-H249R | +++ |
| T022A-S024R-S101G-S103A-V104I-P129E-G159E-S188D-A232V-Q245R-N248D-H249R | +++ |

(continued)

| TABLE 11-1. BMI cleaning performance of NHJ6 library variants.<br>PI = Performance Index<br>PI> or = 1.5 is +++; PI between 1.49 and 1.3 = ++; PI between 1.29 and 1.1 = + in detergent b, d or e | |
|---|---|
| **Sequence of GG36 Variants (BPN' numbering)** | **PI Relative to GG36; BMI assay; Detergent of Example 36b, 36d or 36e, 16C** |
| T022A-S024R-S 101G-S103A-V104I-A158E-G159E-S188D-A232V-Q245R-N248D-H249R | +++ |
| T022A-S024R-S101G-S103A-V104I-P129E-A158E-G159E-S188D-A232V-N238R-Q245R-N248D | +++ |
| T022A-S101G-S103A-V104I-P129E-A158E-G159E-A232V-N238R-Q245R-N248D | +++ |
| T022A-S024R-S101G-S103A-V104I-P129E-A158E-G159E-A232V-Q245R-N248D-H249R | +++ |
| T022A-S024R-S 101G-S103A-V104I-S128N-A158E-S188D-A232V-Q245R-N248D | +++ |
| T022A-S024R-S101G-S103A-V104I-P129E-A158E-A232V-Q245R-N248D | +++ |
| T022A-S024R-S101G-S103A-V104I-P129E-A232V-Q245R-N248D | +++ |
| T022A-S024R-S101G-S103A-V104I-P129E-S188D-A232V-Q245R-N248D | +++ |
| T022A-S101G-S103A-V104I-S128N-P129E-S188D-A232V-N238R-Q245R-N248D | +++ |
| T022A-S024R-S 101G-S103A-V104I-S128N-S188D-A232V-Q245R-N248D | +++ |
| | |
| T022A-S024R-S101G-S103A-V104I-S128N-P129E-A158E-A232V-Q245R-N248D | +++ |
| T022A-S101G-S103A-V104I-S128N-P129E-A232V-N238R-Q245R-N248D | +++ |
| T022A-S024R-S101G-S103A-V104I-P129E-A158E-S188D-A232V-N238R-Q245R-N248D | +++ |
| T022A-S024R-S 101G-S103A-V104I-A158E-G159E-S188D-A232V-Q245R-N248D | +++ |
| T022A-S101G-S103A-V104I-P129E-A158E-A232V-N238R-Q245R-N248D | +++ |
| T022A-S024R-S101G-S103A-V104I-P129E-A158E-A232V-Q245R-N248D-H249R | +++ |
| T022A-S 101G-S103A-V104I-A158E-G159E-S188D-A232V-Q245R-N248D-H249R | +++ |
| T022A-S 101G-S103A-V104I-A158E-S188D-A232V-Q245R-N248D | +++ |
| T022A-S101G-S103A-V104I-P129E-S188D-A232V-N238R-Q245R-N248D | +++ |
| T022A-S024R-S 101G-S103A-V104I-A158E-G159E-S188D-A232V-N238R-Q245R-N248D | +++ |

(continued)

| TABLE 11-1. BMI cleaning performance of NHJ6 library variants.<br>PI = Performance Index<br>PI> or = 1.5 is +++; PI between 1.49 and 1.3 = ++; PI between 1.29 and 1.1 = + in detergent b, d or e | |
|---|---|
| **Sequence of GG36 Variants (BPN' numbering)** | **PI Relative to GG36; BMI assay; Detergent of Example 36b, 36d or 36e, 16C** |
| T022A-S024R-S101G-S103A-V104I-S128N-P129E-S188D-A232V-Q245R-N248D | +++ |
| T022A-S101G-S103A-V104I-P129E-G159E-A232V-N238R-Q245R-N248D | +++ |
| T022A-S 101G-S103A-V104I-P129E-A158E-A232V-Q245R-N248D-H249R | ++ |
| T022A-S 101G-S103A-V104I-P129E-G159E-A232V-Q245R-N248D-H249R | ++ |
| T022A-S101G-S103A-V104I-G159E-S188D-A232V-Q245R-N248D-H249R | ++ |
| T022A-S 101G-S103A-V104I-P129E-G159E-S188D-A232V-Q245R-N248D-H249R | ++ |
| T022A-S 101G-S103A-V104I-A158E-G159E-A232V-N238R-Q245R-N248D | ++ |
| T022A-S101G-S103A-V104I-P129E-A158E-G159E-A232V-Q245R-N248D-H249R | ++ |
| T022A-S024R-S101G-S103A-V104I-P129E-A158E-G159E-A232V-Q245R-N248D | ++ |
| T022A-S024R-S 101G-S103A-V104I-A158E-A232V-Q245R-N248D-H249R | ++ |
| T022A-S024R-S 101G-S103A-V104I-L148I-A158E-A232V-Q245R-N248D | ++ |
| T022A-S101G-S103A-V104I-G159E-S188D-A232V-N238R-Q245R-N248D | ++ |
| T022A-S024R-S101G-S103A-V104I-P129E-A158E-G159E-S188D-A232V-Q245R-N248D-H249R | ++ |
| T022A-S101G-S103A-V104I-P129E-A232V-N238R-Q245R-N248D | ++ |
| T022A-S024R-S 101G-S103A-V104I-S188D-A232V-Q245R-N248D-H249R | ++ |
| T022A-S 101G-S103A-V104I-A158E-G159E-A232V-Q245R-N248D-H249R | ++ |
| T022A-S024R-S101G-S103A-V104I-P129E-A232V-Q245R-N248D-H249R | ++ |
| T022A-S024R-S101G-S103A-V104I-P129E-L148I-A232V-Q245R-N248D | ++ |
| T022A-S024R-S 101G-S103A-V104I-A158E-A232V-N238R-Q245R-N248D | ++ |
| T022A-S 101G-S103A-V104I-S128N-G159E-A232V-Q245R-N248D | + |

(continued)

| TABLE 11-1. BMI cleaning performance of NHJ6 library variants.<br>PI = Performance Index<br>PI> or = 1.5 is +++; PI between 1.49 and 1.3 = ++; PI between 1.29 and 1.1 = + in detergent b, d or e | |
|---|---|
| **Sequence of GG36 Variants (BPN' numbering)** | **PI Relative to GG36; BMI assay; Detergent of Example 36b, 36d or 36e, 16C** |
| T022A-S101G-S103A-V104I-S128N-P129E-A158E-A232V-N238R-Q245R-N248D | + |
| T022A-S024R-S101G-S103A-V104I-S128N-P129E-A158E-S188D-A232V-Q245R-N248D-H249R | + |
| T022A-S024K-S 101G-S103A-V104I-S128N-A158E-G159E-A232V-Q245R-N248D | + |
| T022A-S 101G-S103A-V104I-L148I-S188D-A232V-Q245R-N248D | + |

| TABLE 11-2. BMI cleaning performance of NHJ6 library variants.<br>PI = Performance Index<br>PI > or = 1.5 is +++; PI between 1.49 and 1.3 = ++; PI between 1.29 and 1.1 = + | |
|---|---|
| **Sequence of GG36 Variants (BPN' numbering)** | **PI Relative to GG36; BMI assay, Detergent of Example 36g, 16C** |
| T022A-S024R-S101G-S103A-V104I-A158E-A232V-Q245R-N248D-H249R | +++ |
| T022A-S024R-S101G-S103A-V104I-P129E-A232V-Q245R-N248D-H249R | +++ |
| T022A-S024R-S101G-S103A-V104I-A158E-A232V-N238R-Q245R-N248D | +++ |
| T022A-S024R-S101G-S103A-V104I-P129E-A232V-Q245R-N248D | +++ |
| T022A-S024R-S101G-S103A-V104I-P129E-A158E-A232V-Q245R-N248D-H249R | ++ |
| T022A-S101G-S103A-V104I-P129E-A232V-N238R-Q245R-N248D | ++ |
| T022A-S024R-S 101G-S103A-V104I-L148I-A158E-A232V-Q245R-N248D | + |
| T022A-S024R-S 101G-S103A-V104I-A158E-G159E-S188D-A232V-N238R-Q245R-N248D | + |
| T022A-S 101G-S103A-V104I-A158E-G159E-A232V-N238R-Q245R-N248D | + |
| T022A-S024R-S101G-S103A-V104I-P129E-A158E-A232V-Q245R-N248D | + |
| T022A-S 101G-S103A-V104I-A158E-G159E-A232V-Q245R-N248D-H249R | + |

| TABLE 11-3. BMI cleaning performance of NHJ7 combinatorial variants.<br>PI = Performance Index<br>PI> or = 1.5 is +++; PI between 1.49 and 1.3 = ++; PI between 1.29 and 1.1 = + in detergent of Example 36b, 36d, or 36e | |
|---|---|
| **Sequence of GG36 Variants (BPN' numbering)** | **PI Relative to GG36; BMI assay, Detergent of Example 36b, 36d or 36e, 16C** |
| T022A-S128N-A158E-H249R | +++ |
| T022A-N062E-A158E | +++ |
| T022A-S101A-A158E-R186H-H249R | ++ |
| A016S-T022A-A158E-R186H-E271F | + |
| T022A-S128N-A158E-R186H | + |
| T022A-S128N-R186H-S188D- | + |
| N062E-V104L-A158E-R186H-S188D-H249R | + |
| T022A-A158E-R186H-H249R-E271F | + |
| T022A-V104L-A158E-H249R | + |
| T022A-V104L-R186H-S188D-H249R | + |

## EXAMPLE 39

### Construction of Additional Libraries and Variants of GG36

This Example describes the construction of GG36 variants and libraries in *B. subtilis* using one or more of the following mutations (BPN' numbering): A001R, Q002S, Q002M, Q002A, Q002R, Q002W, S003R, V004R, V004S, V004C, I008A, S009A, S009F, S009W, R010S, R010A, R010H, R010M, Q012F, Q012R, P014K, P014F, P014Q, A015R, A015F, A016S, H017R, H017M, H017F, N018R, N018K, G020F, G020K, G020R, T022A, T022Y, T022V, T022Q, T022L, T022W, G023A, G023S, G023F, S024R, S024F, S024W, S024Q, S024H, S024L, G025V, G025F, G025R, V026F, K027L, K027F, K027R, K027V, V028A, V028N, V028E, A029T, V030E, L031F, T033S, T033G, T033D, G034P, I035M, S036T, S036F, S036R, T038L, T038F, T038R, P040N, P040L, P040T, P040W, P040H, P040R, L042I, N043A, N043F, N043I, N043S, N043R, N043M, N043W, N043D, R045T, G046R, A048R, F050C, V051W, V051F, V051H, P052F, P052E, P052N, P055Y, T057R, Q059A, Q059F, Q059R, D060P, D060Q, D060A, N062E, N062Q, G063V, G063M, G063T, G063I, G063A, G063S, G063H, G063Q, G063D, G063E, G063P, H064F, H064T, V068A, V068C, A069N, A069T, A069P, A069W, T071G, I072C, A074C, L075A, L075F, L075E, L075R, N076D, S078R, S078N, S078I, I079W, I079Q, V081R, L082F, L082T, L082V, L082R, L082M, A085M, P086W, P086L, P086I, E089P, E089T, E089G, E089H, E089L, E089V, E089W, E089F, E089I, Y091N, Y091F, A092F, K094N, S099F, S099T, S099P, S099G, S099M, G100S, G100N, G100Q, G100I, S101A, S101N, S101G, S101T, S101D, S101E, S101P, S101F, G102A, G102T, G102N, G102H, G102E, S103G, S103N, S103D, S103A, V104L, V104I, V104E, V104D, S105T, S105E, S105Q, S106G, S106T, S106E, S106D, S106A, S106V, S106F, I107M, I107F, A108I, A108G, Q109M, L111V, L111I, E112V, E112L, E112Q, A114G, G115K, G115R, N116K, N116A, N116L, N117F, G118R, G118I, M119C, H120A, H120F, H120R, V121F, V121E, N123G, N123E, L124S, S128D, S128F, S128L, S128N, S128H, S128M, S128I, S128Q, P129E, S132A, S132E, A138G, S144R, V147L, L148I, A158E, G159D, G159E, G159C, S160D, S166D, S166E, Y167W, M175V, V177C, D181A, Q182R, N183I, N183D, N183M, N183F, N183R, N185E, N185V, N185I, R186H, R186K, S188E, S188D, S188R, Y192H, Y192W, A194E, A194V, A194F, D197F, I198L, I198F, V203E, V203C, T208S, Y209S, Y209N, Y209F, Y209T, Y209E, Y209H, Y209G, Y209L, P210R, P210V, P210L, G211Q, G211R, S212I, S212M, S212F, T213A, Y214F, A215N, A215D, A215E, A215H, A215F, S216F, S216A, L217E, L217N, L217D, N218D, N218P, N218E, T224A, T224G, V227I, A230E, A231I, A231C, A232V, L233C, V234F, K235F, Q236F, Q236N, Q236H, N238R, N238K, N238L, P239K, P239G, P239R, P239H, P239T, P239N, P239S, P239F, S240R, W241R, S242L, S242R, N243F, N243R, V244R, Q245R, I246S, N248D, N248V, N248I, N248R, H249R, H249T, L250I, K251R, K251S, N252I, N252F, N252R, N252K, N252H, T253I, T253R, T253F, A254C, S256N, G258R, T260V, T260I, L262D, L262H, Y263F, S265F, L267V, L267N, L267M, N269I, N269R, A270C, E271I, E271V, E271H, E271M, E271L, E271P, E271A, E271F, E271T, A272F, A272F, A272R, A273F, A273I, and T274G. The variants and libraries were constructed by DNA2.0, Inc., as described in Example 41. The variants generated containing one or more of these mutations were tested for cold water cleaning in the BMI microswatch assay using the methods and detergent compositions described in this application in TEST METHOD 6.

| TABLE 12-6. BMI cleaning performance of NHJ8, NHJ9, NHJ14 library variants.<br>PI = Performance Index<br>PI> or = 1.5 is +++; PI between 1.49 and 1.3 = ++; PI between 1.29 and 1.1 = + in detergent of Example 36c | |
|---|---|
| **Sequence of GG36 Variants (BPN' numbering)** | **PI Relative to GG36; BMI assay, Detergent of Example 36c 16C** |
| T022A-S101G-S103A-V104I-G159D-L217E-A232V-Q245R-N248D-E271F | +++ |
| T022A-N043R-S 101G-S103A-V104I-G159D-S188D-L217E-A232V-Q245R-N248D-E271F | +++ |
| T022A-S 101G-S103A-V104I-G159D-S1 88D-A232V-Q245R-N248D-E271F | +++ |
| T022A-N076D-S 101G-S103A-V104I-G159D-S188D-A232V-Q245R-N248D-E271F | ++ |
| T022A-N043R-N076D-S 101G-S103A-V104I-G159D-S188D-A232V-Q245R-N248D-E271F | ++ |
| T022A-N076D-S101G-S103A-V104I-G159D-A232V-Q245R-N248D-E271F | ++ |
| T022A-S101G-S103A-V104I-G159D-A232V-Q245R-N248D-E271F | ++ |

| TABLE 12-7. BMI cleaning performance of NHJ9 library variants.<br>PI = Performance Index<br>PI > or = 1.3 is +++; PI between 1.29 and 1.2 = ++; PI between 1.19 and 1.1 = + for detergent of Example 36f | |
|---|---|
| **Sequence of GG36 Variants (BPN' numbering)** | **PI Relative to GG36; BMI assay; Detergent of Example 36f 16C** |
| H017R-T022A-N076D-S101G-S103A-V104I-G159D-S188D-A232V-Q245R-N248D-E271F | +++ |
| T022A-N043R-S101G-S103A-V104I-G159D-A232V-Q245R-N248D-E271F | ++ |
| T022A-S 101G-S103A-V104I-G159D-S188D-A232V-Q245R-N248D-H249RE271F | ++ |
| H017R-T022A-N076D-S101G-S103A-V104I-G159D-A232V-Q245R-N248D-E271F | ++ |
| T022A-N076D-S101G-S103A-V104I-G159D-A232V-Q245R-N248D-H249RE271F | + |
| T022A-S101G-G102A-S103A-V104I-G159D-S188D-A232V-Q245R-N248D-E271F | + |

| TABLE 12-10. BMI cleaning performance of NHJ15 library variants. PI = Performance Index PI> or = 1.3 is +++; PI between 1.29 and 1.2 = ++; PI between 1.19 and 1.1 = + in detergent of Example 36a | |
|---|---|
| **Sequence of GG36 Variants (BPN' numbering)** | **PI Relative to GG36; BMI assay, Detergent of Example 36a 16C** |
| T022A-S024R-S101D-S103A-V104I-G118R-G159D-S188D-A232V-N248D-E271F | +++ |
| T022A-S024R-S103A-V104I-P129E-G159D-S188D-A232V-N248D-E271F | +++ |
| T022A-S024R-S103A-V104I-G118R-G159D-S188D-L217D-A232V-N248D | +++ |
| T022A-S024R-S101D-S103A-V104I-G118R-P129E-G159D-S188D-A232V-Q245R-N248D | +++ |
| T022A-S024R-S 1 01D-S 103A-V104I-G159D-S188D-A232V-Q245R-N248D | +++ |
| T022A-N043R-S 103A-V104I-G118R-P129E-G159D-S188D-A232V-Q245R-N248D | +++ |
| T022A-N043R-S103A-V104I-G118R-S128I-P129E-G159D-S188D-A232V-N248D | ++ |
| T022A-N043R-S101D-S103A-V104I-G118R-P129E-G159D-S188D-A232V-N248D-E271F | ++ |
| T022A-S024R-N043R-S101D-S103A-V104I-G159D-S188D-A232V-Q245R-N248D | ++ |
| T022A-S 103A-V104I-G159D-S188D-A232V-N248D | ++ |
| T022A-S024R-S103A-V104I-G118R-P129E-G159D-S188D-A232V-N248D-E271F | ++ |
| T022A-S024R-S 103A-V104I-G159D-S188D-L217D-A232V-Q245R-N248D-E271F | ++ |
| T022A-N043R-N062E-S 103A-V104I-G159D-S188D-A232V-Q245R-N248D-E271F | ++ |
| T022A-N043R-S 103A-V104I-P129E-G159D-S188D-A232V-Q245R-N248D | ++ |
| T022A-S024R-S103A-V104I-G159D-S188D-L217D-A232V-N248D-E271F | ++ |
| T022A-S103A-V104I-G118R-G159D-S188D-L217D-A232V-Q245R-N248D | ++ |
| T022A-S024R-S101D-S103A-V104I-G118R-S128I-G159D-S188D-A232V-Q245R-N248D | ++ |
| T022A-S024R-N043R-S103A-V104I-G159D-S188D-L217D-A232V-N248D-E271F | ++ |
| T022A-N043R-S 103A-V104I-G118R-G159D-S188D-L217D-A232V-N248D-E271F | ++ |
| T022A-N043R-S103A-V104I-G118R-G159D-S188D-A232V-N248D-E271F | ++ |
| T022A-S103A-V104I-S128I-P129E-G159D-S188D-A232V-N248D-E271F | + |

(continued)

| TABLE 12-10. BMI cleaning performance of NHJ15 library variants.<br>PI = Performance Index<br>PI> or = 1.3 is +++; PI between 1.29 and 1.2 = ++; PI between 1.19 and 1.1 = + in detergent of Example 36a | |
|---|---|
| **Sequence of GG36 Variants (BPN' numbering)** | **PI Relative to GG36; BMI assay, Detergent of Example 36a 16C** |
| T022A-S 103A-V104I-G159D-S1 88D-L217D-A232V-Q245R-N248D-E271F | + |
| T022A-N043R-S103A-V104I-S128I-G159D-S188D-A232V-Q245R-N248D | + |
| T022A-S101D-S103A-V104I-G118R-G159D-S188D-L217D-A232V-Q245R-N248D-E271F | + |
| T022A-S 103A-V104I-G118R-P129E-G159D-S188D-A232V-Q245R-N248D-E271F | + |
| T022A-S024R-N043R-S 103A-V104I-G118R-G159D-S188D-L217D-A232V-N248D | + |

| TABLE 12-12. BMI cleaning performance of NHJ15 and NHJ16 library variants.<br>PI = Performance Index<br>PI > or = 1.3 is +++; PI between 1.29 and 1.2 = ++; PI between 1.19 and 1.1 = + in detergent of Example 36a, 36d or 36f | |
|---|---|
| **Sequence of GG36 Variants (BPN' numbering)** | **PI Relative to GG36; BMI Detergent of Example 36a, 36d or 36f; 16C** |
| T022A-N043R-S103A-V104I-S128I-P129E-G159D-S188D-A232V-Q245R-N248D | +++ |
| T022A-N043R-S103A-V104I-G118R-S128I-P129E-G159D-S188D-A232V-N248D-E271F | +++ |
| T022A-S024R-S103A-V104I-G118R-S128I-P129E-G159D-S188D-A232V-N248D | +++ |
| T022A-S024R-S 103A-V104I-S128I-G159D-S188D-A232V-N248D | + |

## Claims

**1.** A composition comprising an adjunct material and a cold water protease variant, said composition being a consumer product, and said protease variant being a variant of a parent protease, said parent protease's sequence being at least 95% identical to the amino acid sequence SEQ ID NO:1, said variant having one or more of the following characteristics:

a) a Test Method 2 performance index of at least 1.1, at least 1.2, at least 1.3, at least 1.4, at least 1.5, at least 1.6, at least 1.7, at least 1.8, at least 1.9, at least 2; from 1.1 to 10, from 1.1 to 8 or even from 1.1 to 5;
b) a Test Method 3 performance index of at least 1.1, at least 1.2, at least 1.3, at least 1.4, at least 1.5, at least 1.6, at least 1.7, at least 1.8, at least 1.9, at least 2; from 1.1 to 10, from 1.1 to 8 or even from 1.1 to 5;
c) a Test Method 4 performance index of at least 1.0, at least 1.1, at least 1.2, at least 1.3, at least 1.4, at least 1.5, at least 1.6, at least 1.7, at least 1.8, at least 1.9, at least 2, from 1.0 to 10, from 1.0 to 8 or even from 1.0 to 5;
d) a Test Method 6 performance index of at least 1.1, at least 1.2, at least 1.3, at least 1.4, at least 1.5, at least 1.6, at least 1.7, at least 1.8, at least 1.9, at least 2; from 1.1 to 10, from 1.1 to 8 or even from 1.1 to 5,

wherein Test Method 2, Test Method 3, Test Method 4 and Test Method 6 are explicitly described under "TEST METHODS" herein, said protease variant being mutated such that the mutations consist of one of the following sets of mutations, wherein each amino acid position is numbered according to the numbering of the corresponding amino acid position in the amino acid sequence of *Bacillus amyloliquefaciens* subtilisin BPN', shown in SEQ ID NO: 2:

| |
|---|
| T022A-N062E, T022A-P129E, T022A-L111V, T022A-S188D, A016S-T022A, |
| T022A-E089P, T022A-G159E, T022A-A158E, |
| T22A-L111V-G159E, |
| T22A-S103G-G159E |
| T22A-S 128N-E271F-Y209E |
| T22A-Y209E-E271F |
| T22A-S101A-Y209E |
| T22A-L111V-S128N |
| T22A-S101A-G159E |
| T22A-S101A-S103G-V104L |
| T22A-S101G-S103A-V104I-G159D-A232V-Q245R-N248D-H249R |
| T22A-S101G-S103A-V104I-G159D-A232V-Q245R-N248D-E271F |
| T022A-S101G-S103A-V104I-P129E-A158E-S188D-A232V-Q245R-N248D-H249R |
| T022A-S024R-S101G-S103A-V104I-P129E-A158E-S188D-A232V-Q245R-N248D-H249R |
| T022A-S024R-S101G-S103A-V104I-P129E-G159E-S188D-A232V-Q245R-N248D-H249R |
| T022A-S024R-S 101G-S103A-V104I-A158E-G159E-S188D-A232V-Q245R-N248D-H249R |
| T022A-S024R-S101G-S103A-V104I-P129E-A158E-G159E-S188D-A232V-N238R-Q245R-N248D |
| T022A-S 101G-S103A-V104I-P129E-A158E-G159E-A232V-N238R-Q245R-N248D |
| T022A-S024R-S101G-S103A-V104I-P129E-A158E-G159E-A232V-Q245R-N248D-H249R |
| T022A-S024R-S101G-S103A-V104I-S128N-A158E-S188D-A232V-Q245R-N248D |
| T022A-S024R-S101G-S103A-V104I-P129E-A158E-A232V-Q245R-N248D |
| T022A-S024R-S101G-S103A-V104I-P129E-A232V-Q245R-N248D |
| T022A-S024R-S101G-S103A-V104I-P129E-S188D-A232V-Q245R-N248D |
| T022A-S101G-S103A-V104I-S128N-P129E-S188D-A232V-N238R-Q245R-N248D |
| T022A-S024R-S 101G-S103A-V104I-S128N-S188D-A232V-Q245R-N248D |
| T022A-S024R-S101G-S103A-V104I-S128N-P129E-A158E-A232V-Q245R-N248D |
| T022A-S101G-S103A-V104I-S128N-P129E-A232V-N238R-Q245R-N248D |
| T022A-S024R-S101G-S103A-V104I-P129E-A158E-S188D-A232V-N238R-Q245R-N248D |
| T022A-S024R-S 101G-S103A-V104I-A158E-G159E-S188D-A232V-Q245R-N248D |
| T022A-S101G-S103A-V104I-P129E-A158E-A232V-N238R-Q245R-N248D |
| T022A-S024R-S101G-S103A-V104I-P129E-A158E-A232V-Q245R-N248D-H249R |
| T022A-S 101G-S103A-V104I-A158E-G159E-S188D-A232V-Q245R-N248D-H249R |
| T022A-S 101G-S103A-V104I-A158E-S188D-A232V-Q245R-N248D |
| T022A-S 101G-S103A-V104I-P129E-S188D-A232V-N238R-Q245R-N248Daaa |
| T022A-S024R-S 101G-S103A-V104I-A158E-G159E-S188D-A232V-N238R-Q245R-N248D |
| T022A-S024R-S101G-S103A-V104I-S128N-P129E-S188D-A232V-Q245R-N248D |
| T022A-S101G-S103A-V104I-P129E-G159E-A232V-N238R-Q245R-N248D |
| T022A-S 101G-S103A-V104I-P129E-A158E-A232V-Q245R-N248D-H249R |

(continued)

| |
|---|
| T022A-S 101G-S103A-V104I-P129E-G159E-A232V-Q245R-N248D-H249R |
| T022A-S 101G-S103A-V104I-G159E-S188D-A232V-Q245R-N248D-H249R |
| T022A-S 101G-S103A-V104I-P129E-G159E-S188D-A232V-Q245R-N248D-H249R |
| T022A-S 101G-S103A-V104I-A158E-G159E-A232V-N238R-Q245R-N248D |
| T022A-S 101G-S103A-V104I-P129E-A158E-G159E-A232V-Q245R-N248D-H249R |
| T022A-S024R-S101G-S103A-V104I-P129E-A158E-G159E-A232V-Q245R-N248D |
| T022A-S024R-S 101G-S103A-V104I-A158E-A232V-Q245R-N248D-H249R |
| T022A-S024R-S 101G-S103A-V104I-L148I-A158E-A232V-Q245R-N248D |
| T022A-S 101G-S103A-V104I-G159E-S188D-A232V-N238R-Q245R-N248D |
| T022A-S024R-S101G-S103A-V104I-P129E-A158E-G159E-S188D-A232V-Q245R-N248D-H249R |
| T022A-S101G-S103A-V104I-P129E-A232V-N238R-Q245R-N248D |
| T022A-S024R-S 101G-S103A-V104I-S188D-A232V-Q245R-N248D-H249R |
| T022A-S 101G-S103A-V104I-A158E-G159E-A232V-Q245R-N248D-H249R |
| T022A-S024R-S101G-S103A-V104I-P129E-A232V-Q245R-N248D-H249R |
| T022A-S024R-S101G-S103A-V104I-P129E-L148I-A232V-Q245R-N248D |
| T022A-S024R-S 101G-S103A-V104I-A158E-A232V-N238R-Q245R-N248D |
| T022A-S 101G-S103A-V104I-S128N-G159E-A232V-Q245R-N248D |
| T022A-S101G-S103A-V104I-S128N-P129E-A158E-A232V-N238R-Q245R-N248D |
| T022A-S024R-S101G-S103A-V104I-S128N-P129E-A158E-S188D-A232V-Q245R-N248D-H249R |
| T022A-S024K-S 101G-S103A-V104I-S128N-A158E-G159E-A232V-Q245R-N248D |
| T022A-S 101G-S103A-V104I-L148I-S188D-A232V-Q245R-N248D |
| T022A-S 128N-A158E-H249R |
| T022A-N062E-A158E |
| T022A-S1 01A-A158E-R186H-H249R |
| A016S-T022A-A158E-R186H-E271F |
| T022A-S 128N-A158E-R186H |
| T022A-S 128N-R186H-S188D- |
| T022A-A158E-R186H-H249R-E271F |
| T022A-V104L-A158E-H249R |
| T022A-V104L-R186H-S188D-H249R |
| T022A-S101G-S103A-V104I-G159D-L217E-A232V-Q245R-N248D-E271F |
| T022A-N043R-S 101G-S103A-V104I-G159D-S188D-L217E-A232V-Q245R-N248D-E271F |
| T022A-S 101G-S103A-V104I-G159D-S1 88D-A232V-Q245R-N248D-E271F |
| T022A-N076D-S 101G-S103A-V104I-G159D-S1 88D-A232V-Q245R-N248D-E271F |
| T022A-N043R-N076D-S 101G-S103A-V104I-G159D-S188D-A232V-Q245R-N248D-E271F |
| T022A-N076D-S 101G-S103A-V104I-G1 59D-A232V-Q245R-N248D-E271F |
| T022A-S 101G-S103A-V104I-G159D-A232V-Q245R-N248D-E27 1F |
| H017R-T022A-N076D-S 101G-S103A-V104I-G159D-S188D-A232V-Q245R-N248D-E271F |
| T022A-N043R-S 101G-S103A-V104I-G1 59D-A232V-Q245R-N248D-E271F |

(continued)

| |
|---|
| T022A-S 101G-S103A-V104I-G159D-S1 88D-A232V-Q245R-N248D-H249R-E271F |
| H017R-T022A-N076D-S 101G-S103A-V104I-G1 59D-A232V-Q245R-N248D-E271F |
| T022A-N076D-S101G-S103A-V104I-G159D-A232V-Q245R-N248D-H249R-E271F |
| T022A-S 101G-G102A-S103A-V104I-G159D-S1 88D-A232V-Q245R-N248D-E271F |
| T022A-S024R-S101D-S103A-V104I-G118R-G159D-S188D-A232V-N248D-E271F |
| T022A-S024R-S103A-V104I-P129E-G159D-S188D-A232V-N248D-E271F |
| T022A-S024R-S103A-V104I-G118R-G159D-S188D-L217D-A232V-N248D |
| T022A-S024R-S101D-S103A-V104I-G118R-P129E-G159D-S188D-A232V-Q245R-N248D |
| T022A-S024R-S 1 01D-S 103A-V104I-G 159D-S188D-A232V-Q245R-N248D |
| T022A-N043R-S 103A-V104I-G118R-P129E-G159D-S188D-A232V-Q245R-N248D |
| T022A-N043R-S 103A-V104I-G118R-S128I-P129E-G159D-S188D-A232V-N248D |
| T022A-N043R-S101D-S103A-V104I-G118R-P129E-G159D-S188D-A232V-N248D-E271F |
| T022A-S024R-N043R-S 1 01D-S 103A-V104I-G159D-S188D-A232V-Q245R-N248D |
| T022A-S 103A-V104I-G159D-S188D-A232V-N248D |
| T022A-S024R-S103A-V104I-G118R-P129E-G159D-S188D-A232V-N248D-E271F |
| T022A-S024R-S103A-V104I-G159D-S188D-L217D-A232V-Q245R-N248D-E271F |
| T022A-N043R-N062E-S 103A-V104I-G159D-S1 88D-A232V-Q245R-N248D-E271F |
| T022A-N043R-S 103A-V104I-P129E-G159D-S188D-A232V-Q245R-N248D |
| T022A-S024R-S103A-V104I-G159D-S188D-L217D-A232V-N248D-E271F |
| T022A-S 103A-V104I-G118R-G159D-S188D-L217D-A232V-Q245R-N248D |
| T022A-S024R-S101D-S103A-V104I-G118R-S128I-G159D-S188D-A232V-Q245R-N248D |
| T022A-S024R-N043R-S 103A-V104I-G159D-S188D-L217D-A232V-N248D-E271F |
| T022A-N043R-S 103A-V104I-G118R-G159D-S1 88D-L217D-A232V-N248D-E271F |
| T022A-N043R-S103A-V1 04I-G118R-G159D-S188D-A232V-N248D-E271F |
| T022A-S103A-V104I-S128I-P129E-G159D-S188D-A232V-N248D-E271F |
| T022A-S 103A-V104I-G159D-S1 88D-L217D-A232V-Q245R-N248D-E271F |
| T022A-N043R-S 103A-V104I-S128I-G159D-S188D-A232V-Q245R-N248D |
| T022A-S 1 01D-S 103A-V104I-G118R-G159D-S188D-L217D-A232V-Q245R-N248D-E271F |
| T022A-S103A-V104I-G118R-P129E-G159D-S188D-A232V-Q245R-N248D-E271F |
| T022A-S024R-N043R-S 103A-V104I-G118R-G159D-S188D-L217D-A232V-N248D |
| T022A-N043R-S 103A-V104I-S128I-P129E-G159D-S188D-A232V-Q245R-N248D |
| T022A-N043R-S 103A-V104I-G118R-S128I-P129E-G159D-S188D-A232V-N248D-E271F |
| T022A-S024R-S103A-V104I-G118R-S128I-P129E-G159D-S188D-A232V-N248D |
| T022A-S024R-S 103A-V104I-S128I-G159D-S188D-A232V-N248D |

**2.** A composition according to claim 1 wherein the cold water protease has a Test Method 6 performance index of at least 1.1, at least 1.2, at least 1.3, at least 1.4, at least 1.5, at least 1.6, at least 1.7, at least 1.8, at least 1.9, at least 2; from 1.1 to 10, from 1.1 to 8 or even from 1.1 to about 5.

**3.** A composition according to claim 1 or claim 2, wherein said adjunct material comprises one or more selected from:

a. an encapsulate comprising a perfume, said encapsulate preferably comprising a perfume micro capsule;
b. a hueing agent preferably comprising a material selected from the group consisting of basic, acid, hydrophobic, direct and polymeric dyes, and dye-conjugates having a peak absorption wavelength of from 550nm to 650nm and mixtures thereof;
c. detersive surfactant preferably comprising a material selected from the group consisting of anionic detersive surfactants, non-ionic detersive surfactant, cationic detersive surfactants, zwitterionic detersive surfactants and amphoteric detersive surfactants and mixtures thereof;
d. builder preferably comprising a material selected from the group consisting of zeolites, phosphates and mixtures thereof;
e. a silicate salt, preferably comprising a material selected from the group consisting of sodium silicate, potassium silicate and mixtures thereof;
f. brightener comprises a material selected from the group consisting of cold-water soluble brighteners and mixtures thereof;
g. a carboxylate polymer preferably comprising a material selected from the group consisting of maleate/acrylate random copolymer or polyacrylate homopolymer and mixtures thereof;
h. a soil release polymer preferably comprising a material selected from the group consisting of terephthalate co-polymers and mixtures thereof;
i. a cellulosic polymer preferably comprising a material selected from the group consisting of alkyl cellulose, alkyl alkoxyalkyl cellulose, carboxyalkyl cellulose, alkyl carboxyalkyl cellulose and mixtures thereof;
j. a bleach catalyst preferably comprising a material selected from the group consisting of iminium cations, iminium polyions; iminium zwitterions; modified amines; modified amine oxides; N-sulphonyl imines; N-phosphonyl imines; N-acyl imines; thiadiazole dioxides; perfluoroimines; cyclic sugar ketones and transition metal catalysts or ligands for the formation thereof, or mixtures thereof;
k. a bleach activator preferably comprising a material selected from the group consisting of dodecanoyl oxybenzene sulphonate, decanoyl oxybenzene sulphonate, decanoyl oxybenzoic acid or salts thereof, 3,5,5-trimethyl hexanoyloxybenzene sulphonate, tetraacetyl ethylene diamine (TAED), nonanoyloxybenzene sulphonate (NOBS) and mixtures thereof;
l. a source of hydrogen peroxide preferably comprising a material selected from the group consisting of inorganic perhydrate salts, including alkali metal salts such as sodium salts of perborate (usually mono- or tetra-hydrate), percarbonate, persulphate, perphosphate, persilicate salts and mixtures thereof;
m. a chelant preferably comprising a material selected from the group consisting of DTPA (Diethylene triamine pentaacetic acid), HEDP (Hydroxyethane diphosphonic acid), DTPMP (Diethylene triamine penta(methylene phosphonic acid)), ethylenediaminedisuccinic acid (EDDS), 1,2-Dihydroxybenzene-3,5-disulfonic acid disodium salt hydrate, derivatives of said chelants;
n. an additional enzyme selected from the group consisting of: (a) first wash lipases; (b) bacterial cleaning cellulases; (c) alpha-amylases; and (d) mixtures thereof; and
o. mixtures thereof.

**4.** A composition according to any preceding claim provided in the form of a single or multi-compartment unit dose, preferably a multi-compartment unit dose, wherein the protease variant is preferably in a different compartment to any source of hydrogen peroxide and/or chelant.

**5.** A method of treating and/or cleaning a surface, preferably a fabric surface comprising the steps of (i) contacting said surface with a composition according to any preceding claim in an aqueous wash liquor; and (ii) rinsing and/or drying the surface.

**6.** A method according to claim 5 wherein the aqueous wash liquor comprises from 0.1g/l to 3g/l of surfactant.

**Patentansprüche**

**1.** Zusammensetzung, umfassend ein Zusatzmaterial und eine Kaltwasserprotease-Variante, wobei die Zusammensetzung ein Endprodukt ist und die Proteasevariante eine Variante einer Ausgangsprotease ist, wobei die Sequenz der Ausgangsprotease zu mindestens 95 % mit der Aminosäuresequenz SEQ-ID Nr. 1 identisch ist, wobei die Variante eine oder mehrere der folgenden Eigenschaften aufweist:

a) einen Leistungsindex gemäß Prüfverfahren 2 von mindestens 1,1, mindestens 1,2, mindestens 1,3, mindestens 1,4, mindestens 1,5, mindestens 1,6, mindestens 1,7, mindestens 1,8, mindestens 1,9, mindestens 2; von

1,1 bis 10, von 1,1 bis 8 oder sogar von 1,1 bis 5;
b) einen Leistungsindex gemäß Prüfverfahren 3 von mindestens 1,1, mindestens 1,2, mindestens 1,3, mindestens 1,4, mindestens 1,5, mindestens 1,6, mindestens 1,7, mindestens 1,8, mindestens 1,9, mindestens 2; von 1,1 bis 10, von 1,1 bis 8 oder sogar von 1,1 bis 5;
c) einen Leistungsindex gemäß Prüfverfahren 4 von mindestens 1,0, mindestens 1,1, mindestens 1,2, mindestens 1,3, mindestens 1,4, mindestens 1,5, mindestens 1,6, mindestens 1,7, mindestens 1,8, mindestens 1,9, mindestens 2, von 1,0 bis 10, von 1,0 bis 8 oder sogar von 1,0 bis 5;
d) einen Leistungsindex gemäß Prüfverfahren 6 von mindestens 1,1, mindestens 1,2, mindestens 1,3, mindestens 1,4, mindestens 1,5, mindestens 1,6, mindestens 1,7, mindestens 1,8, mindestens 1,9, mindestens 2; von 1,1 bis 10, von 1,1 bis 8 oder sogar von 1,1 bis 5,

wobei Prüfverfahren 2, Prüfverfahren 3, Prüfverfahren 4 und Prüfverfahren 6 explizit unter "PRÜFVERFAHREN" hierin beschrieben sind, wobei die Proteasevariante derart mutiert ist, dass die Mutationen aus einem der folgenden Sätze von Mutationen bestehen, wobei jede Aminosäureposition gemäß der Nummerierung der entsprechenden Aminosäureposition in der Aminosäuresequenz von *Bacillus Amyloliquefaciens*-Subtilisin BPN', dargestellt in SEQ-ID Nr. 2, nummeriert ist:

| |
|---|
| T022A-N062E, T022A-P129E, T022A-L111V, T022A-S188D, A016S-T022A, |
| T022A-E089P, T022A-G159E, T022A-A158E, |
| T22A-L111V-G159E, |
| T22A-S103G-G159E |
| T22A-S128N-E271F-Y209E |
| T22A-Y209E-E271F |
| T22A-S101A-Y209E |
| T22A-L111V-S128N |
| T22A-S101A-G159E |
| T22A-S101A-S103G-V104L |
| T22A-S101G-S103A-V104I-G159D-A232V-Q245R-N248D-H249R |
| T22A-S101G-S103A-V104I-G159D-A232V-Q245R-N248D-E271F |
| T022A-S101G-S103A-V104I-P129E-A158E-S188D-A232V-Q245R-N248D-H249R |
| T022A-S024R-S101G-S103A-V104I-P129E-A158E-S188D-A232V-Q245R-N248D-H249R |
| T022A-S024R-S101G-S103A-V104I-P129E-G159E-S188D-A232V-Q245R-N248D-H249R |
| T022A-S024R-S101G-S103A-V104I-A158E-G159E-S188D-A232V-Q245R-N248D-H249R |
| T022A-S024R-S101G-S103A-V104I-P129E-A158E-G159E-S188D-A232V-N238R-Q245R-N248D |
| T022A-S101G-S103A-V104I-P129E-A158E-G159E-A232V-N238R-Q245R-N248D |
| T022A-S024R-S101G-S103A-V104I-P129E-A158E-G159E-A232V-Q245R-N248D-H249R |
| T022A-S024R-S101G-S103A-V104I-S128N-A158E-S188D-A232V-Q245R-N248D |
| T022A-S024R-S101G-S103A-V104I-P129E-A158E-A232V-Q245R-N248D |
| T022A-S024R-S101G-S103A-V104I-P129E-A232V-Q245R-N248D |
| T022A-S024R-S101G-S103A-V104I-P129E-S188D-A232V-Q245R-N248D |
| T022A-S101G-S103A-V104I-S128N-P129E-S188D-A232V-N238R-Q245R-N248D |
| T022A-S024R-S101G-S103A-V104I-S128N-S188D-A232V-Q245R-N248D |
| T022A-S024R-S101G-S103A-V104I-S128N-P129E-A158E-A232V-Q245R-N248D |
| T022A-S101G-S103A-V104I-S128N-P129E-A232V-N238R-Q245R-N248D |

(fortgesetzt)

| |
|---|
| T022A-S024R-S101G-S103A-V104I-P129E-A158E-S188D-A232V-N238R-Q245R-N248D |
| T022A-S024R-S101G-S103A-V104I-A158E-G159E-S188D-A232V-Q245R-N248D |
| T022A-S101G-S103A-V104I-P129E-A158E-A232V-N238R-Q245R-N248D |
| T022A-S024R-S101G-S103A-V104I-P129E-A158E-A232V-Q245R-N248D-H249R |
| T022A-S101G-S103A-V104I-AI58E-G159E-S188D-A232V-Q245R-N248D-H249R |
| T022A-S101G-S103A-V104I-A158E-S188D-A232V-Q245R-N248D |
| T022A-S101G-S103A-V104I-P129E-S188D-A232V-N238R-Q245R-N248Daaa |
| T022A-S024R-S101G-S103A-V104I-A158E-G159E-S188D-A232V-N238R-Q245R-N248D |
| T022A-S024R-S101G-S103A-V104I-S128N-P129E-S188D-A232V-Q245R-N248D |
| T022A-S101G-S103A-V104I-P129E-G159E-A232V-N238R-Q245R-N248D |
| T022A-S101G-S103A-V104I-P129E-A158E-A232V-Q245R-N248D-H249R |
| T022A-S101G-S103A-V104I-P129E-G159E-A232V-Q245R-N248D-H249R |
| T022A-S101G-S103A-V104I-G159E-S188D-A232V-Q245R-N248D-H249R |
| T022A-S101G-S103A-V104I-P129E-G159E-S188D-A232V-Q245R-N248D-H249R |
| T022A-S101G-S103A-V104I-A158E-G159E-A232V-N238R-Q245R-N248D |
| T022A-S101G-S103A-V104I-P129E-A158E-G159E-A232V-Q245R-N248D-H249R |
| T022A-S024R-S101G-S103A-V104I-P129E-A158E-G159E-A232V-Q245R-N248D |
| T022A-S024R-S101G-S103A-V104I-A158E-A232V-Q245R-N248D-H249R |
| T022A-S024R-S101G-S103A-V104I-L148I-A158E-A232V-Q245R-N248D |
| T022A-S101G-S103A-V104I-G159E-S188D-A232V-N238R-Q245R-N248D |
| T022A-S024R-S101G-S103A-V104I-P129E-A158E-G159E-S188D-A232V-Q245R-N248D-H249R |
| T022A-S101G-S103A-V104I-P129E-A232V-N238R-Q245R-N248D |
| T022A-S024R-S101G-S103A-V104I-S188D-A232V-Q245R-N248D-H249R |
| T022A-S101G-S103A-V104I-A158E-G159E-A232V-Q245R-N248D-H249R |
| T022A-S024R-S101G-S103A-V104I-P129E-A232V-Q245R-N248D-H249R |
| T022A-S024R-S101G-S103A-V104I-P129E-L148I-A232V-Q245R-N248D |
| T022A-S024R-S101G-S103A-V104I-A158E-A232V-N238R-Q245R-N248D |
| T022A-S101G-S103A-V104I-S128N-G159E-A232V-Q245R-N248D |
| T022A-S101G-S103A-V104I-S128N-P129E-A158E-A232V-N238R-Q245R-N248D |
| T022A-S024R-S101G-S103A-V104I-S128N-P129E-A158E-S188D-A232V-Q245R-N248D-H249R |
| T022A-S024K-S101G-S103A-V104I-S128N-A158E-G159E-A232V-Q245R-N248D |
| T022A-S101G-S103A-V104I-L148I-S188D-A232V-Q245R-N248D |
| T022A-S128N-A158E-H249R |
| T022A-N062E-A158E |
| T022A-S101A-A158E-R186H-H249R |
| A016S-T022A-A158E-R186H-E271F |
| T022A-S128N-A158E-R186H |
| T022A-S128N-R186H-S188D- |
| T022A-A158E-R186H-H249R-E271F |

(fortgesetzt)

| |
|---|
| T022A-V104L-A158E-H249R |
| T022A-V104L-R186H-S188D-H249R |
| T022A-S101G-S103A-V104I-G159D-L217E-A232V-Q245R-N248D-E271F |
| T022A-N043R-S101G-S103A-V104I-G159D-S188D-L217E-A232V-Q245R-N248D-E271F |
| T022A-S101G-S103A-V104I-G159D-S188D-A232V-Q245R-N248D-E271F |
| T022A-N076D-S101G-S103A-V104I-G159D-S188D-A232V-Q245R-N248D-E271F |
| T022A-N043R-N076D-S101G-S103A-V104I-G159D-S188D-A232V-Q245R-N248D-E271F |
| T022A-N076D-S101G-S103A-V104I-G159D-A232V-Q245R-N248D-E271F |
| T022A-S101G-S103A-V104I-G159D-A232V-Q245R-N248D-E271F |
| H017R-T022A-N076D-S101G-S103A-V104I-G159D-S188D-A232V-Q245R-N248D-E271F |
| T022A-N043R-S101G-S103A-V104I-G159D-A232V-Q245R-N248D-E271F |
| T022A-S101G-S103A-V104I-G159D-S188D-A232V-Q245R-N248D-H249R-E271F |
| H017R-T022A-N076D-S101G-S103A-V104I-G159D-A232V-Q245R-N248D-E271F |
| T022A-N076D-S101G-S103A-V104I-G159D-A232V-Q245R-N248D-H249R-E271F |
| T022A-S101G-G102A-S103A-V104I-G159D-S188D-A232V-Q245R-N248D-E271F |
| T022A-S024R-S101D-S103A-V104I-G118R-G159D-S188D-A232V-N248D-E271F |
| T022A-S024R-S103A-V104I-P129E-G159D-SI88D-A232V-N248D-E271F |
| T022A-S024R-S103A-V104I-G118R-G159D-S188D-L217D-A232V-N248D |
| T022A-S024R-S101D-S103A-V104I-G118R-P129E-G159D-S188D-A232V-Q245R-N248D |
| T022A-S024R-S101D-S103A-V104I-G159D-S188D-A232V-Q245R-N248D |
| T022A-N043R-S103A-V104I-G118R-P129E-G159D-S188D-A232V-Q245R-N248D |
| T022A-N043R-S103A-V104I-G118R-S128I-P129E-G159D-S188D-A232V-N248D |
| T022A-N043R-S101D-S103A-V104I-G118R-P129E-G159D-S188D-A232V-N248D-E271F |
| T022A-S024R-N043R-S101D-S103A-V104I-G159D-S188D-A232V-Q245R-N248D |
| T022A-S103A-V104I-G159D-S188D-A232V-N248D |
| T022A-S024R-S103A-V104I-G118R-P129E-G159D-S188D-A232V-N248D-E271F |
| T022A-S024R-S103A-V104I-G159D-S188D-L217D-A232V-Q245R-N248D-E271F |
| T022A-N043R-N062E-S103A-V104I-G159D-S188D-A232V-Q245R-N248D-E271F |
| T022A-N043R-S103A-V104I-P129E-G159D-S188D-A232V-Q245R-N248D |
| T022A-S024R-S103A-V104I-G159D-S188D-L217D-A232V-N248D-E271F |
| T022A-S103A-V104I-G118R-G159D-S188D-L217D-A232V-Q245R-N248D |
| T022A-S024R-S101D-S103A-V104I-G118R-S128I-G159D-S188D-A232V-Q245R-N248D |
| T022A-S024R-N043R-S103A-V104I-G159D-S188D-L217D-A232V-N248D-E271F |
| T022A-N043R-S103A-V104I-G118R-G159D-S188D-L217D-A232V-N248D-E271F |
| T022A-N043R-S103A-V104I-G118R-G159D-S188D-A232V-N248D-E271F |
| T022A-S103A-V104I-S128I-P129E-G159D-S188D-A232V-N248D-E271F |
| T022A-S103A-V104I-G159D-S188D-L217D-A232V-Q245R-N248D-E271F |
| T022A-N043R-S103A-V104I-S128I-G159D-S188D-A232V-Q245R-N248D |
| T022A-S101D-S103A-V104I-G118R-G159D-S188D-L217D-A232V-Q245R-N248D-E271F |

(fortgesetzt)

| |
|---|
| T022A-S103A-V104I-G118R-P129E-G159D-S188D-A232V-Q245R-N248D-E271F |
| T022A-S024R-N043R-S103A-V104I-G118R-G159D-S188D-L217D-A232V-N248D |
| T022A-N043R-S103A-V104I-S128I-P129E-GI59D-S188D-A232V-Q245R-N248D |
| T022A-N043R-S103A-V104I-G118R-S128I-P129E-G159D-S188D-A232V-N248D-E271F |
| T022A-S024R-S103A-V104I-G118R-S128I-P129E-G159D-S188D-A232V-N248D |
| T022A-S024R-S103A-V104I-S128I-G159D-S188D-A232V-N248D |

**2.** Zusammensetzung nach Anspruch 1, wobei die Kaltwasserprotease einen Leistungsindex gemäß Prüfverfahren 6 von mindestens 1,1, mindestens 1,2, mindestens 1,3, mindestens 1,4, mindestens 1,5, mindestens 1,6, mindestens 1,7, mindestens 1,8, mindestens 1,9, mindestens 2; von 1,1 bis 10, von 1,1 bis 8 oder sogar von 1,1 bis etwa 5 aufweist.

**3.** Zusammensetzung nach Anspruch 1 oder Anspruch 2, wobei das Zusatzmaterial eines oder mehrere umfasst, die ausgewählt sind aus:

a. einem eingekapselten Teilchen, das einen Duftstoff umfasst, wobei das eingekapselte Teilchen vorzugsweise eine Duftstoffmikrokapsel umfasst;
b. einem Abtönungsmittel, vorzugsweise umfassend ein Material, das ausgewählt ist aus der Gruppe bestehend aus basischen, sauren, hydrophoben, direkten und polymeren Farbstoffen und Farbstoffkonjugaten mit einer Peak-Absorptionswellenlänge von 550 nm bis 650 nm und Mischungen davon;
c. Reinigungstensid, vorzugsweise umfassend ein Material, das ausgewählt ist aus der Gruppe bestehend aus anionischen Reinigungstensiden, nichtionischem Reinigungstensid, kationischen Reinigungstensiden, zwitterionischen Reinigungstensiden und amphoteren Reinigungstensiden und Mischungen davon;
d. Builder, vorzugsweise umfassend ein Material, das ausgewählt ist aus der Gruppe bestehend aus Zeolithen, Phosphaten und Mischungen davon;
e. einem Silicatsalz, vorzugsweise umfassend ein Material, das ausgewählt ist aus der Gruppe bestehend aus Natriumsilicat, Kaliumsilicat und Mischungen davon;
f. Aufheller, umfassend ein Material, das ausgewählt ist aus der Gruppe bestehend aus kaltwasserlöslichen Aufhellern und Mischungen davon;
g. einem Carboxylatpolymer, vorzugsweise umfassend ein Material, das ausgewählt ist aus der Gruppe bestehend aus statistischem Maleat/Acrylat-Copolymer oder Polyacrylat-Homopolymer und Mischungen davon;
h. einem Schmutzabweisungspolymer, vorzugsweise umfassend ein Material, das ausgewählt ist aus der Gruppe bestehend aus Terephthalat-Copolymeren und Mischungen davon;
i. einem Cellulosepolymer, vorzugsweise umfassend ein Material, das ausgewählt ist aus der Gruppe bestehend aus Alkylcellulose, Alkylalkoxyalkylcellulose, Carboxyalkylcellulose, Alkylcarboxyalkylcellulose und Mischungen davon;
j. einen Bleichmittelkatalysator, vorzugsweise umfassend ein Material, das ausgewählt ist aus der Gruppe bestehend aus Iminkationen, Iminpolyionen; Iminzwitterionen; modifizierten Aminen; modifizierten Aminoxiden; N-Sulphonyliminen; N-Phosphonyliminen; N-Acetyliminen; Thiadiazoldioxiden; Perfluoriminen; cyclischen Zuckerketonen und Übergangsmetallkatalysatoren oder Liganden für die Bildung dieser, oder Mischungen davon;
k. einem Bleichaktivator, vorzugsweise umfassend ein Material, das ausgewählt ist aus der Gruppe bestehend aus Dodecanoyloxybenzolsulfonat, Decanoyloxybenzolsulfonat, Decanoyloxybenzoesäure oder Salzen davon, 3,5,5-Trimethylhexanoyloxybenzolsulfonat, Tetraacetylethylendiamin (TAED), Nonanoyloxybenzolsulfonat (NOBS) und Mischungen davon;
l. einer Wasserstoffperoxidquelle, vorzugsweise umfassend ein Material, das ausgewählt ist aus der Gruppe bestehend aus anorganischen Perhydratsalzen, einschließlich Alkalimetallsalzen wie Natriumsalzen von Perborat (gewöhnlich Mono- oder Tetrahydrat), Percarbonat-, Persulfat-, Perphosphat-, Persilicatsalzen und Mischungen davon;
m. einem Chelatbildner, vorzugsweise umfassend ein Material, das ausgewählt ist aus der Gruppe bestehend aus DTPA (Diethylentriaminpentaessigsäure), HEDP (Hydroxyethandiphosphonsäure), DTPMP (Diethylentriaminpenta(methylenphosphonsäure)), Ethylendiamindibernsteinsäure (EDDS), 1,2-Dihydroxybenzol-3,5-disulfonsäure-Dinatriumsalz-Hydrat, Derivaten der Chelatbildner;
n. einem zusätzlichen Enzym, das ausgewählt ist aus der Gruppe bestehend aus: (a) Lipasen für die erste Waschung; (b) bakteriellen Reinigungscellulasen; (c) Alpha-Amylasen; und (d) Mischungen davon; und

o. Mischungen davon.

4. Zusammensetzung nach einem der vorstehenden Ansprüche, die in der Form einer Ein- oder Mehrkammer-Einheitsdosis, vorzugsweise einer Mehrkammer-Einheitsdosis, bereitgestellt wird, wobei sich die Proteasevariante vorzugsweise in einer anderen Kammer als eine Wasserstoffperoxidquelle und/oder ein Chelatbildner befindet.

5. Verfahren zur Behandlung und/oder Reinigung einer Oberfläche, vorzugsweise einer Stoffoberfläche, umfassend die Schritte (i) Inkontaktbringen der Oberfläche mit einer Zusammensetzung nach einem der vorstehenden Ansprüche in einer wässrigen Waschflotte; und (ii) Spülen und/oder Trocknen der Oberfläche.

6. Verfahren nach Anspruch 5, wobei die wässrige Flotte von 0,1 g/l bis 3 g/l Tensid umfasst.

## Revendications

1. Composition comprenant un matériau additif et une variante de protéase dans de l'eau froide, ladite composition étant un produit de consommation, et ladite variante de protéase étant une variante d'une protéase mère, ladite séquence de la protéase mère étant au moins identique à 95 % à la séquence d'acides aminés SEQ ID NO: 1, ladite variante ayant une ou plusieurs des caractéristiques suivantes :

   a) un indice de performance de la méthode d'essai 2 d'au moins 1,1, d'au moins 1,2, d'au moins 1,3, d'au moins 1,4, d'au moins 1,5, d'au moins 1,6, d'au moins 1,7, d'au moins 1,8, d'au moins 1,9, d'au moins 2 ; de 1,1 à 10, de 1,1 à 8 ou même de 1,1 à 5 ;
   b) un indice de performance de la méthode d'essai 3 d'au moins 1,1, d'au moins 1,2, d'au moins 1,3, d'au moins 1,4, d'au moins 1,5, d'au moins 1,6, d'au moins 1,7, d'au moins 1,8, d'au moins 1,9, d'au moins 2 ; de 1,1 à 10, de 1,1 à 8 ou même de 1,1 à 5 ;
   c) un indice de performance de la méthode d'essai 4 d'au moins 1,0, d'au moins 1,1, d'au moins 1,2, d'au moins 1,3, d'au moins 1,4, d'au moins 1,5, d'au moins 1,6, d'au moins 1,7, d'au moins 1,8, d'au moins 1,9, d'au moins 2, de 1,0 à 10, de 1,0 à 8 ou même de 1,0 à 5 ;
   d) un indice de performance de la méthode d'essai 6 d'au moins 1,1, d'au moins 1,2, d'au moins 1,3, d'au moins 1,4, d'au moins 1,5, d'au moins 1,6, d'au moins 1,7, d'au moins 1,8, d'au moins 1,9, d'au moins 2 ; de 1,1 à 10, de 1,1 à 8 ou même de 1,1 à 5,

   dans laquelle la méthode d'essai 2, la méthode d'essai 3, la méthode d'essai 4 et la méthode d'essai 6 sont explicitement décrites dans les « MÉTHODES D'ESSAI » de la présente invention, ladite variante de protéase étant mutée de sorte que les mutations consistent en l'un des ensembles de mutations suivants, dans laquelle chaque position d'acide aminé est numérotée conformément à la numérotation de la position d'acide aminé correspondante dans la séquence d'acides aminés de la subtilisine BPN' de *Bacillus amyloliquefaciens*, représentée dans la SEQ ID NO: 2 :

| |
|---|
| T022A-N062E, T022A-P129E, T022A-L111V, T022A-S188D, A016S-T022A, |
| T022A-E089P, T022A-G159E, T022A-A158E, |
| T22A-L111V-G159E, |
| T22A-S103G-G159E |
| T22A-S128N-E271F-Y209E |
| T22A-Y209E-E271F |
| T22A-S101A-Y209E |
| T22A-L111V-S128N |
| T22A-S101A-G159E |
| T22A-S101A-S103G-V104L |
| T22A-S101G-S103A-V104I-G159D-A232V-Q245R-N248D-H249R |

(suite)

| |
|---|
| T22A-S101G-S103A-V104I-G159D-A232V-Q245R-N248D-E271F |
| T022A-S101G-S103A-V104I-P129E-A158E-S188D-A232V-Q245R-N248D-H249R |
| T022A-S024R-S101G-S103A-V104I-P129E-A158E-S188D-A232V-Q245R-N248D-H249R |
| T022A-S024R-S101G-S103A-V104I-P129E-G159E-S188D-A232V-Q245R-N248D-H249R |
| T022A-S024R-S101G-S103A-V104I-A158E-G159E-S188D-A232V-Q245R-N248D-H249R |
| T022A-S024R-S101G-S103A-V104I-P129E-A158E-G159E-S188D-A232V-N238R-Q245R-N248D |
| T022A-S101G-S103A-V104I-P129E-A158E-G159E-A232V-N238R-Q245R-N248D |
| T022A-S024R-S101G-S103A-V104I-P129E-A158E-G159E-A232V-Q245R-N248D-H249R |
| T022A-S024R-S101G-S103A-V104I-S128N-A158E-S188D-A232V-Q245R-N248D |
| T022A-S024R-S101G-S103A-V104I-P129E-A158E-A232V-Q245R-N248D |
| T022A-S024R-S101G-S103A-V104I-P129E-A232V-Q245R-N248D |
| T022A-S024R-S101G-S103A-V104I-P129E-S188D-A232V-Q245R-N248D |
| T022A-S101G-S103A-V104I-S128N-P129E-S188D-A232V-N238R-Q245R-N248D |
| T022A-S024R-S101G-S103A-V104I-S128N-S188D-A232V-Q245R-N248D |
| T022A-S024R-S101G-S103A-V104I-S128N-P129E-A158E-A232V-Q245R-N248D |
| T022A-S101G-S103A-V104I-S128N-P129E-A232V-N238R-Q245R-N248D |
| T022A-S024R-S101G-S103A-V104I-P129E-A158E-S188D-A232V-N238R-Q245R-N248D |
| T022A-S024R-S101G-S103A-V104I-A158E-G159E-S188D-A232V-Q245R-N248D |
| T022A-S101G-S103A-V104I-P129E-A158E-A232V-N238R-Q245R-N248D |
| T022A-S024R-S101G-S103A-V104I-P129E-A158E-A232V-Q245R-N248D-H249R |
| T022A-S101G-S103A-V104I-AI58E-G159E-S188D-A232V-Q245R-N248D-H249R |
| T022A-S101G-S103A-V104I-A158E-S188D-A232V-Q245R-N248D |
| T022A-S101G-S103A-V104I-P129E-S188D-A232V-N238R-Q245R-N248Daaa T022A-S024R-S101G-S103A-V104I-A158E-G159E-S188D-A232V-N238R-Q245R-N248D |
| T022A-S024R-S101G-S103A-V104I-S128N-P129E-S188D-A232V-Q245R-N248D |
| T022A-S101G-S103A-V104I-P129E-G159E-A232V-N238R-Q245R-N248D |
| T022A-S101G-S103A-V104I-P129E-A158E-A232V-Q245R-N248D-H249R |
| T022A-S101G-S103A-V104I-P129E-G159E-A232V-Q245R-N248D-H249R |
| T022A-S101G-S103A-V104I-G159E-S188D-A232V-Q245R-N248D-H249R |
| T022A-S101G-S103A-V104I-P129E-G159E-S188D-A232V-Q245R-N248D-H249R |
| T022A-S101G-S103A-V104I-A158E-G159E-A232V-N238R-Q245R-N248D |
| T022A-S101G-S103A-V104I-P129E-A158E-G159E-A232V-Q245R-N248D-H249R |
| T022A-S024R-S101G-S103A-V104I-P129E-A158E-G159E-A232V-Q245R-N248D |
| T022A-S024R-S101G-S103A-V104I-A158E-A232V-Q245R-N248D-H249R |
| T022A-S024R-S101G-S 103 A-VI 04I-L1481-AI 58E-A232V-Q245R-N248D |
| T022A-S101G-S103A-V104I-G159E-S188D-A232V-N238R-Q245R-N248D |
| T022A-S024R-S101G-S103A-V104I-P129E-A158E-G159E-S188D-A232V-Q245R-N248D-H249R |
| T022A-S101G-S103A-V104I-P129E-A232V-N238R-Q245R-N248D |
| T022A-S024R-S101G-S103A-V104I-S188D-A232V-Q245R-N248D-H249R |

(suite)

| |
|---|
| T022A-S101G-S103A-V104I-A158E-G159E-A232V-Q245R-N248D-H249R |
| T022A-S024R-S101G-S103A-V104I-P129E-A232V-Q245R-N248D-H249R |
| T022A-S024R-S101G-S103A-V104I-P129E-L148I-A232V-Q245R-N248D |
| T022A-S024R-S101G-S103A-V104I-A158E-A232V-N238R-Q245R-N248D |
| T022A-S101G-S103A-V104I-S128N-G159E-A232V-Q245R-N248D |
| T022A-S101G-S103A-V104I-S128N-P129E-A158E-A232V-N238R-Q245R-N248D |
| T022A-S024R-S101G-S103A-V104I-S128N-P129E-A158E-S188D-A232V-Q245R-N248D-H249R |
| T022A-S024K-S101G-S103A-V104I-S128N-A158E-G159E-A232V-Q245R-N248D |
| T022A-S101G-S103A-V104I-L148I-S188D-A232V-Q245R-N248D |
| T022A-S128N-A158E-H249R |
| T022A-N062E-A158E |
| T022A-S101A-A158E-R186H-H249R |
| A016S-T022A-A158E-R186H-E271F |
| T022A-S128N-A158E-R186H |
| T022A-S128N-R186H-S188D- |
| T022A-A158E-R186H-H249R-E271F |
| T022A-V104L-A158E-H249R |
| T022A-V104L-R186H-S188D-H249R |
| T022A-S101G-S103A-V104I-G159D-L217E-A232V-Q245R-N248D-E271F |
| T022A-N043R-S101G-S103A-V104I-G159D-S188D-L217E-A232V-Q245R-N248D-E271F |
| T022A-S101G-S103A-V104I-G159D-S188D-A232V-Q245R-N248D-E271F |
| T022A-N076D-S101G-S103A-V104I-G159D-S188D-A232V-Q245R-N248DE271F |
| T022A-N043R-N076D-S101G-S103A-V104I-G159D-S188D-A232V-Q245R-N248D-E271F |
| T022A-N076D-S101G-S103A-V104I-G159D-A232V-Q245R-N248D-E271F |
| T022A-S101G-S103A-V104I-G159D-A232V-Q245R-N248D-E271F |
| H017R-T022A-N076D-S101G-S103A-V104I-G159D-S188D-A232V-Q245R-N248D-E271F |
| T022A-N043R-S101G-S103A-V104I-G159D-A232V-Q245R-N248D-E271F |
| T022A-S101G-S103A-V104I-G159D-S188D-A232V-Q245R-N248D-H249R-E271F |
| H017R-T022A-N076D-S101G-S103A-V104I-G159D-A232V-Q245R-N248DE271F |
| T022A-N076D-S101G-S103A-V104I-G159D-A232V-Q245R-N248D-H249R-E271F |
| T022A-S101G-G102A-S103A-V104I-G159D-S188D-A232V-Q245R-N248DE271F |
| T022A-S024R-S101D-S103A-V104I-G118R-G159D-S188D-A232V-N248DE271F |
| T022A-S024R-S103A-V104I-P129E-G159D-SI88D-A232V-N248D-E271F |
| T022A-S024R-S103A-V104I-G118R-G159D-S188D-L217D-A232V-N248D |
| T022A-S024R-S101D-S103A-V104I-G118R-P129E-G159D-S188D-A232V-Q245R-N248D |
| T022A-S024R-S101D-S103A-V104I-G159D-S188D-A232V-Q245R-N248D |
| T022A-N043R-S103A-V104I-G118R-P129E-G159D-S188D-A232V-Q245R-N248D |
| T022A-N043R-S103A-V104I-G118R-S128I-P129E-G159D-S188D-A232V-N248D |
| T022A-N043R-S101D-S103A-V104I-G118R-P129E-G159D-S188D-A232V-N248D-E271F |

(suite)

| |
|---|
| T022A-S024R-N043R-S101D-S103A-V104I-G159D-S188D-A232V-Q245R-N248D |
| T022A-S103A-V104I-G159D-S188D-A232V-N248D |
| T022A-S024R-S103A-V104I-G118R-P129E-G159D-S188D-A232V-N248DE271F |
| T022A-S024R-S103A-V104I-G159D-S188D-L217D-A232V-Q245R-N248DE271F |
| T022A-N043R-N062E-S103A-V104I-G159D-S188D-A232V-Q245R-N248DE271F |
| T022A-N043R-S103A-V104I-P129E-G159D-S188D-A232V-Q245R-N248D |
| T022A-S024R-S103A-V104I-G159D-S188D-L217D-A232V-N248D-E271F |
| T022A-S103A-V104I-G118R-G159D-S188D-L217D-A232V-Q245R-N248D |
| T022A-S024R-S101D-S103A-V104I-G118R-S128I-G159D-S188D-A232V-Q245R-N248D |
| T022A-S024R-N043R-S103A-V104I-G159D-S188D-L217D-A232V-N248DE271F |
| T022A-N043R-S103A-V104I-G118R-G159D-S188D-L217D-A232V-N248DE271F |
| T022A-N043R-S103A-V104I-G118R-G159D-S188D-A232V-N248D-E271F |
| T022A-S103A-V104I-S128I-P129E-G159D-S188D-A232V-N248D-E271F |
| T022A-S103A-V104I-G159D-S188D-L217D-A232V-Q245R-N248D-E271F |
| T022A-N043R-S103A-V104I-S128I-G159D-S188D-A232V-Q245R-N248D |
| T022A-S101D-S103A-V104I-G118R-G159D-S188D-L217D-A232V-Q245R-N248D-E271F |
| T022A-S103A-V104I-G118R-P129E-G159D-S188D-A232V-Q245R-N248DE271F |
| T022A-S024R-N043R-S103A-V104I-G118R-G159D-S188D-L217D-A232V-N248D |
| T022A-N043R-S103A-V104I-S128I-P129E-GI59D-S188D-A232V-Q245R-N248D |
| T022A-N043R-S103A-V104I-G118R-S128I-P129E-G159D-S188D-A232V-N248D-E271F |
| T022A-S024R-S103A-V104I-G118R-S128I-P129E-G159D-S188D-A232V-N248D |
| T022A-S024R-S103A-V104I-S128I-G159D-S188D-A232V-N248D |

**2.** Composition selon la revendication 1, dans laquelle la protéase dans de l'eau froide a un indice de performance de la méthode d'essai 6 d'au moins 1,1, d'au moins 1,2, d'au moins 1,3, d'au moins 1,4, d'au moins 1,5, d'au moins 1,6, d'au moins 1,7, d'au moins 1,8, d'au moins 1,9, d'au moins 2 ; de 1,1 à 10, de 1,1 à 8 ou même de 1,1 à environ 5.

**3.** Composition selon la revendication 1 ou la revendication 2, dans lequel ledit matériau additif comprend un ou plusieurs éléments choisis parmi :

a. une capsule comprenant un parfum, ladite capsule comprenant de préférence une micro-capsule de parfum ;
b. un agent teintant comprenant de préférence un matériau choisi dans le groupe constitué de teintures basiques, acides, hydrophobes, directes et polymères et de conjugués de teintures ayant une longueur d'onde d'absorption maximale allant de 550 nm à 650 nm et des mélanges de ceux-ci ;
c. un agent tensioactif détersif comprenant de préférence un matériau choisi dans le groupe constitué d'agents tensioactifs détersifs anioniques, agent tensioactif détersif non ionique, agents tensioactifs détersifs cationiques, agents tensioactifs détergents zwittérioniques et agents tensioactifs détersifs amphotères et des mélanges de ceux-ci ;
d. un adjuvant comprenant de préférence un matériau choisi dans le groupe constitué de zéolites, phosphates et des mélanges de ceux-ci ;
e. un sel silicate, comprenant de préférence un matériau choisi dans le groupe constitué de silicate de sodium, silicate de potassium et des mélanges de ceux-ci ;
f. un azurant optique qui comprend un matériau choisi dans le groupe constitué d'azurants optiques solubles dans l'eau froide et des mélanges de ceux-ci ;
g. un polymère carboxylate comprenant de préférence un matériau choisi dans le groupe constitué de copolymère aléatoire maléate-acrylate ou homopolymère polyacrylate et des mélanges de ceux-ci ;

h. un polymère antisalissure comprenant de préférence un matériau choisi dans le groupe constitué de copolymères de téréphtalate et des mélanges de ceux-ci ;
i. un polymère cellulosique comprenant de préférence un matériau choisi dans le groupe constitué d'alkylcellulose, alkylalcoxyalkylcellulose, carboxyalkylcellulose, alkylcarboxyalkylcellulose et des mélanges de ceux-ci ;
j. un catalyseur de blanchiment comprenant de préférence un matériau choisi dans le groupe constitué de cations iminium, polyions iminium ; zwittérions iminium ; aminés modifiées ; oxydes d'amine modifiée ; N-sulfonylimines ; N-phosphonylimines ; N-acylimines ; dioxydes de thiadiazole ; perfluoro-imines ; cétones de sucre cycliques et catalyseurs à base de métal de transition ou ligands pour la formation de ceux-ci, ou mélanges de ceux-ci ;
k. un activateur de blanchiment comprenant de préférence un matériau choisi dans le groupe constitué de sulfonate de dodécanoyl-oxybenzène, sulfonate de décanoyl-oxybenzène, acide décanoyl-oxybenzoïque ou des sels de celui-ci, sulfonate de 3,5,5-triméthyl-hexanoyloxybenzène, tétra-acétyléthylène diamine (TAED), sulfonate de nonanoyloxybenzène (NOBS) et des mélanges de ceux-ci ;
l. une source de peroxyde d'hydrogène comprenant de préférence un matériau choisi dans le groupe constitué de sels perhydrate inorganiques, y compris des sels de métal alcalin tels que des sels sodiques de perborate (habituellement mono- ou tétra-hydraté), percarbonate, persulfate, perphosphate, sels persilicate et leurs mélanges ;
m. un agent chélatant comprenant de préférence un matériau choisi dans le groupe constitué de DTPA (acide diéthylène triamine penta-acétique), HEDP (acide hydroxyéthane diphosphonique), DTPMP (acide diéthylène triamine pentaméthylène phosphonique), acide éthylène diamine disuccinique (EDDS), sel disodique hydraté de l'acide 1,2-dihydroxybenzène-3,5-disulfonique, et dérivés desdits agents chélatants ;
n. une enzyme supplémentaire choisie dans le groupe constitué de : (a) premières lipases de lavage ; (b) cellulases de nettoyage bactériennes ; (c) alpha-amylases ; et (d) mélanges de celles-ci ; et
o. des mélanges de ceux-ci.

**4.** Composition selon l'une quelconque des revendications précédentes fournie sous la forme d'une dose unitaire à un seul compartiment ou à compartiments multiples, de préférence une dose unitaire à compartiments multiples, le variant de protéase étant de préférence dans un compartiment distinct d'une quelconque source de peroxyde d'hydrogène et/ou agent chélatant.

**5.** Procédé de traitement et/ou de nettoyage d'une surface, de préférence une surface textile, comprenant les étapes consistant à (i) mettre en contact ladite surface avec une composition selon l'une quelconque des revendications précédentes dans une liqueur de lavage aqueuse ; et (ii) rincer et/ou sécher la surface.

**6.** Procédé selon la revendication 5, dans laquelle la liqueur aqueuse comprend de 0,1 g/L à 3 g/L d'agent tensioactif.

**FIGURE 1 -** alignment of the mature reference proteases Bacillus amyloliquefaciens subtilisin BPN' (SEQ ID NO:2) and B. lentus subtilisin GG36 protease (SEQ ID NO:1).

```
BPN'   1      AQSVPYGVSQ IKAPALHSQG YTGSNVKVAV IDSGIDSSHP DLKVAGGASM VPSETNPFQD
GG36   1      AQSVPWGISR VQAPAAHNRG LTGSGVKVAV LDTGIS-THP DLNIRGGASF VPGEPST-QD

BPN'   61 NNSHGTHVAG TVAALNNSIG VLGVAPSASL YAVKVLGADG SGQYSWIING IEWAIANNMD
GG36   59 GNGHGTHVAG TIAALNNSIG VLGVAPSAEL YAVKVLGASG SGSVSSIAQG LEWAGNNGMH

BPN'   121 VINMSLGGPS GSAALKAAVD KAVASGVVVV AAAGNEGTSG SSSTVGYPGK YPSVIAVGAV
GG36   119 VANLSLGSPS PSATLEQAVN SATSRGVLVV AASGNSGAGS ----ISYPAR YANAMAVGAT

BPN'   181 DSSNQRASFS QYGPELDVMA PGVSIQSTLP GNKYGAYNGT SMASPHVAGA AALILSKHPN
GG36   175 DQNNNRASFS QYGAGLDIVA PGVNVQSTYP GSTYASLNGT SMATPHVAGA AALVKQKNPS

BPN'   241 WTNTQVRSSL ENTTTKLGDS FYYGKGLINV QAAAQ
GG36   235 WSNVQIRNHL KNTATSLGST NLYGSGLVNA EAATR
```

**FIGURE 2** - pHPLT-GG36 Expression Plasmid

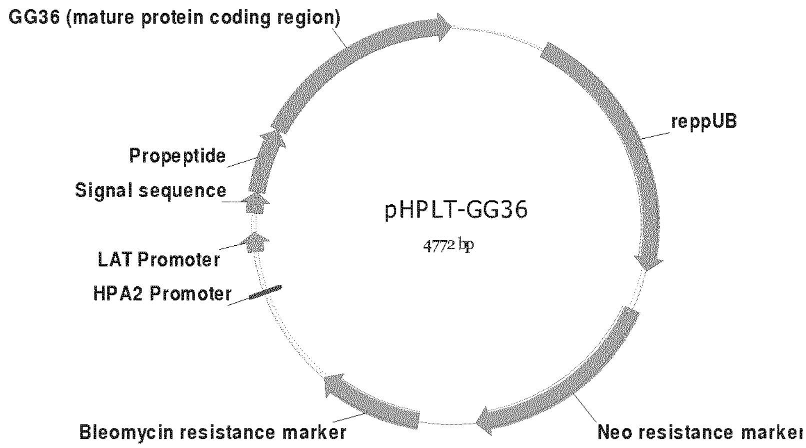

**FIGURE 3** - pRA68 Expression Plasmid.

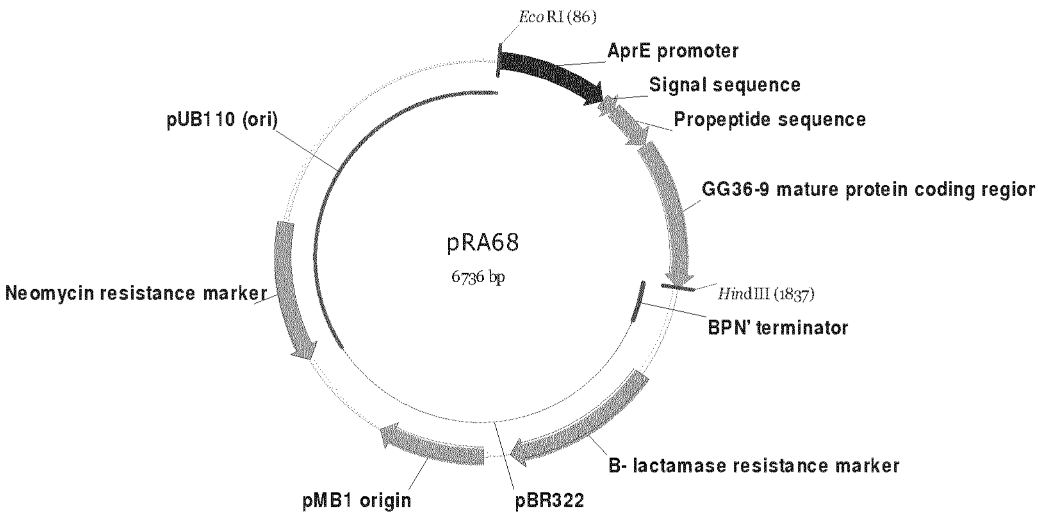

**FIGURE 4** - pRA96 Expression Plasmid

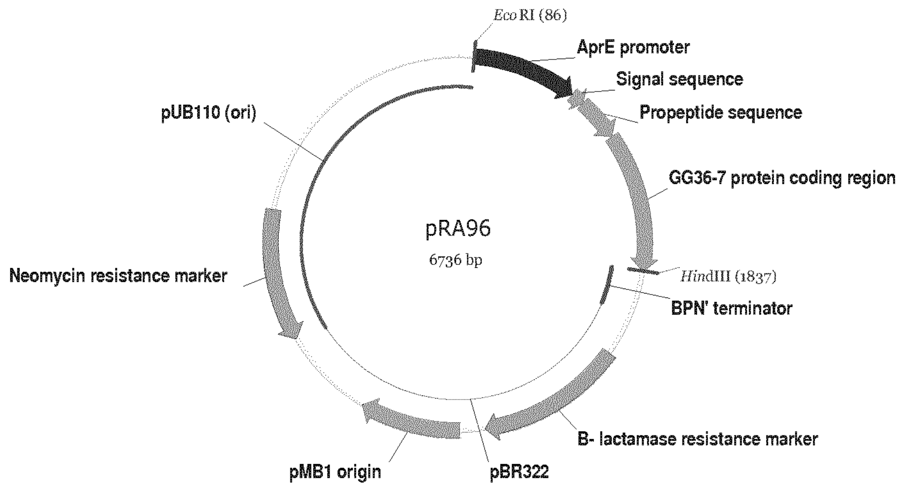

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9920771 A
- US 7208459 B2
- US 20080305982 A1
- US 20090247449 A1
- US 6312936 B1
- US 5679630 A
- US 4760025 A
- US 7262042 B
- WO 09021867 A
- US 5288627 A
- US 20080063774 A1
- US 20080293610 A1
- US 5352604 A
- US P7153818 B
- US 6979731 B
- US 6916645 B
- WO 9402597 A
- US 6297037 B
- US 7378264 B
- US 5763385 A
- US P5856164 A
- US 6673589 B
- US 6093562 A
- US 6187576 B
- US 20080193999 A1
- US 6939702 B1
- US 20090217464 A
- US 4435307 A
- US 5648263 A
- US 5691178 A
- US 5776757 A
- US 5520838 A
- US 5948672 A
- US 5919691 A
- US 6001639 A
- WO 9808940 A
- US 6114296 A
- US 5457046 A
- US 5686593 A
- US 5763254 A
- US 6117664 A
- US 20090170747 A1
- DK 9800299 W
- US 7141403 B
- US 7361736 B
- US 6268197 B
- US 6630340 B
- WO 2002077242 A
- US 7172891 B
- US 5576282 A
- US 6306812 B1
- US 6326348 B1
- US 6380144 B
- EP 1109965 A
- EP 1240378 A
- US 20070173430 A1
- US 4430243 A
- US 5597936 A
- US 5595967 A
- US 7501389 B
- US 6225464 B
- US 4990280 A
- US 20030087791 A1
- US 20030087790 A1
- US 20050003983 A1
- US 20040048764 A1
- US 4762636 A
- US 6291412 B
- US 20050227891 A1
- EP 1070115 A2
- US 5879584 A
- US 5691297 A
- US 5574005 A
- US 5569645 A
- US 5565422 A
- US 5516448 A
- US 5489392 A
- US 5486303 A
- US 20060089284 A1
- WO 2004059556 A3
- EP 0200362 A
- EP 0201184 A
- US 4683195 A
- US 4683202 A
- US 6472184 B
- US 6566112 B
- WO 2002014490 A

### Non-patent literature cited in the description

- **STEMMER.** *Proc Natl Acad Sci U S A.*, 1994, vol. 25, 10747-51

- **OSTERMEIER et al.** *Bioorg Med Chem.,* 1999, vol. 7, 2139-44
- **LUTZ et al.** *Proc Natl Acad Sci USA.,* 2001, vol. 98, 11248-53
- **SIEBER et al.** *Nat Biotechnol.,* 2001, vol. 19, 456-60
- **BITTKER et al.** *Nat Biotechnol.,* 2001, vol. 20, 1024-9
- **BITTKER et al.** *Proc Natl Acad Sci. USA.,* 2004, vol. 101, 7011-6
- **NESS et al.** *Nat Biotechnol.,* 2002, vol. 20, 1251-5
- **COCO et al.** *Nat Biotechnol.,* 2002, vol. 20, 1246-50
- **ZHA et al.** *Chembiochem.,* 2003, vol. 3, 34-9
- **GLASER et al.** *J Immunol.,* 1992, vol. 149, 3903-13
- **SONDEK ; SHORTLE.** *Proc Natl Acad Sci USA,* 1992, vol. 89, 3581-5
- **YÁÑEZ et al.** *Nucleic Acids Res.,* 2004, vol. 32, e158
- **OSUNA et al.** *Nucleic Acids Res.,* 2004, vol. 32, e136
- **GAYTÁN et al.** *Nucleic Acids Res.,* 2001, vol. 29, E9
- **GAYTÁN et al.** *Nucleic Acids Res.,* 2002, vol. 30, e84
- **PISARCHIK et al.** *Prot. Eng. Des. Select.,* 2007, vol. 20, 257-265
- Dyes are listed in the Color Index International. Industrial Dyes. Society of Dyers and Colourists, 2002
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. USA,* 1988, vol. 85, 2444
- **DEVEREUX et al.** *Nucl. Acid Res.,* 1984, vol. 12, 387-395
- **FENG ; DOOLITTLE.** *J. Mol. Evol.,* 1987, vol. 35, 351-360
- **HIGGINS ; SHARP.** *CABIOS,* 1989, vol. 5, 151-153
- **ALTSCHUL et al.** *J. Mol. Biol.,* 1990, vol. 215, 403-410
- **KARLIN ; ALTSCHUL.** *Proc. Natl. Acad. Sci. USA,* 1993, vol. 90, 5873-5787
- **ALTSCHUL et al.** *Meth. Enzymol.,* 1996, vol. 266, 460-480
- **HENIKOFF ; HENIKOFF.** *Proc. Natl. Acad. Sci. USA,* 1992, vol. 89, 10915
- **ALTSCHUL et al.** *Nucleic Acids Res.,* 1997, vol. 25 (17), 3389-3402
- **A. B. BORASTON et al.** *Biochemical Journal,* 2004, vol. 382, 769-781
- *Biochem J.,* 1991, vol. 280, 309-316
- **BROOKE, D. N. ; DOBBS, A. J. ; WILLIAMS, N.** *Ecotoxicology and Environmental Safety,* 1986, vol. 11 (3), 251-260
- **ADAM, W. ; HAAS, W. ; LOHRAY, B. B.** *Journal of the American Chemical Society,* 1991, vol. 113 (16), 6202-6208
- **HEINZ et al.** *Biochemistry,* 1992, vol. 31, 8755-66
- **MCKENZIE et al.** *Plasmid,* 1986, vol. 15, 93-103
- **NEIDHARDT et al.** *J. Bacteriol.,* 1974, vol. 119, 736-747
- **BABÉ et al.** *Biotech. Appl. Biochem.,* 1998, vol. 27, 117-124
- **BABE et al.** *Biotech. Appl. Biochem.,* 1998, vol. 27, 117-124